(19) Europäisches Patentamt
European Patent Office
Office européen des brevets

(11) EP 1 479 676 A1

(12) **EUROPEAN PATENT APPLICATION**

(43) Date of publication:
**24.11.2004 Bulletin 2004/48**

(21) Application number: **03011305.4**

(22) Date of filing: **19.05.2003**

(51) Int Cl.[7]: **C07D 413/14**, C07D 401/04, C07D 401/14, C07D 417/14, C07D 471/04, A61K 31/4184, A61P 7/02

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Aventis Pharma Deutschland GmbH
65929 Frankfurt am Main (DE)**

(72) Inventors:
- **Nazaré, Marc, Dr.
65510 Idstein (DE)**
- **Wagner, Michael, Dr.
64665 Alsbach (DE)**
- **Wehner, Volkmar, Dr.
97657 Sandberg (DE)**
- **Matter, Hans, Dr.
63505 Langenselbold (DE)**
- **Urmann, Matthias, Dr.
65760 Eschborn (DE)**
- **Ritter, Kurt, Dr.
60594 Frankfurt am Main (DE)**

(54) **Benzimidazole-derivatives as factor xa inhibitors**

(57) Benzimidazole-derivatives as factor Xa inhibitors

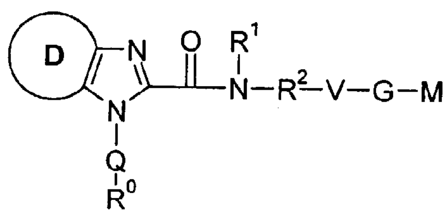

**(I)**

The present invention relates to compounds of the formula I,
wherein $R^0$ ; $R^1$ ; $R^2$ ; $R^3$ ; $R^4$; Q; V, G and M have the meanings indicated in the claims. The compounds of the formula I are valuable pharmacologically active compounds. They exhibit a strong antithrombotic effect and are suitable, for example, for the therapy and prophylaxis of cardiovascular disorders like thromboembolic diseases or restenoses. They are reversible inhibitors of the blood clotting enzymes factor Xa (FXa) and/or factor VIIa (FVIIa), and can in general be applied in conditions in which an undesired activity of factor Xa and/or factor VIIa is present or for the cure or prevention of which an inhibition of factor Xa and/or factor VIIa is intended. The invention furthermore relates to processes for the preparation of compounds of the formula I, their use, in particular as active ingredients in pharmaceuticals, and pharmaceutical preparations comprising them.

EP 1 479 676 A1

## Description

The present invention relates to compounds of the formula I,

(I) 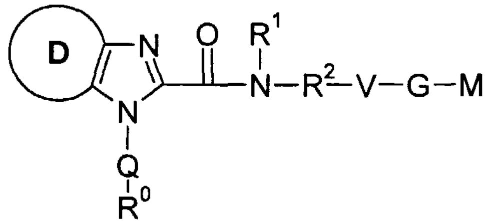

in which $R^0$; $R^1$ ; $R^2$ ; $R^3$ ; $R^4$; Q; V, G and M have the meanings indicated below. The compounds of the formula I are valuable pharmacologically active compounds. They exhibit a strong antithrombotic effect and are suitable, for example, for the therapy and prophylaxis of cardiovascular disorders like thromboembolic diseases or restenoses. They are reversible inhibitors of the blood clotting enzymes factor Xa (FXa) and/or factor VIIa (FVIIa), and can in general be applied in conditions in which an undesired activity of factor Xa and/or factor VIIa is present or for the cure or prevention of which an inhibition of factor Xa and/or factor VIIa is intended. The invention furthermore relates to processes for the preparation of compounds of the formula I, their use, in particular as active ingredients in pharmaceuticals, and pharmaceutical preparations comprising them.

Normal haemeostasis is the result of a complex balance between the processes of clot initiation, formation and clot dissolution. The complex interactions between blood cells, specific plasma proteins and the vascular surface, maintain the fluidity of blood unless injury and blood loss occurs (EP-A-987274). Many significant disease states are related to abnormal haemeostasis. For example, local thrombus formation due to rupture of atheroslerotic plaque is a major cause of acute myocardial infarction and unstable angina. Treatment of an occlusive coronary thrombus by either thrombolytic therapy or percutaneous angioplasty may be accompanied by acute thrombolytic reclosure of the affected vessel.

There continues to be a need for safe and effective therapeutic anticoagulants to limit or prevent thrombus formation. It is most desirable to develop agents that inhibit coagulation without directly inhibiting thrombin but by inhibiting other steps in the coagulation cascade like factor Xa and/or factor VIIa activity. It is now believed that inhibitors of factor Xa carry a lower bleeding risk than thrombin inhibitors (A. E. P. Adang & J. B. M. Rewinkel, Drugs of the Future 2000, 25, 369-383).

Low molecular weight, factor Xa-specific blood clotting inhibitors that are effective but do not cause unwanted side effects have been described, for example, in WO-A-95/29189.
However, besides being an effective factor Xa-specific blood clotting inhibitor, it is desirable that such inhibitors also have further advantageous properties, for instance stability in plasma and liver and selectivity versus other serine proteases whose inhibition is not intended, such as thrombin. There is an ongoing need for further low molecular weight factor Xa specific blood clotting inhibitors, which are effective and have the above advantages as well.

Specific inhibition of the factor VIIa/tissue factor catalytic complex using monoclonal antibodies (WO-A-92/06711) or a protein such as chloromethyl ketone inactivated factor VIIa (WO-A-96/12800, WO-A-97/47651) is an extremely effective means of controlling thrombus formation caused by acute arterial injury or the thrombotic complications related to bacterial septicemia. There is also experimental evidence suggesting that inhibition of factor VIIa/ tissue factor activity inhibits restenosis following balloon angioplasty. Bleeding studies have been conducted in baboons and indicate that inhibition of the factor VIIa/tissue factor complex has the widest safety window with respect to therapeutic effectiveness and bleeding risk of any anticoagulant approach tested including thrombin, platelet and factor Xa inhibition. Certain inhibitors of factor VIIa have already been described. EP-A-987274, for example discloses compounds containing a tripeptide unit, which inhibit factor VIIa. However, the property profile of these compounds is still not ideal, and there is an ongoing need for further low molecular weight factor Vlla inhibitory blood clotting inhibitors

The present invention satisfies the above needs by providing novel compounds of the formula I, which exhibit better factor Xa and/or factor Vlla inhibitory activity and are favorable agents with high bioavailability.

Thus, the present invention relates to compounds of the formula I,

(I) 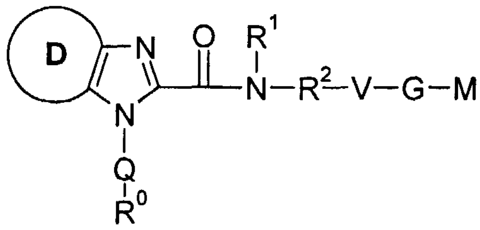

wherein

$R^0$ is 1) a monocyclic or bicyclic 6- to 14-membered aryl, wherein aryl is mono-, di- or trisubstituted independently of one another by R8,
2) a monocyclic or bicyclic 4- to 15-membered heterocyclyl out of the group pyridinyl, pyrimidinyl, indolyl, isoindolyl, indazolyl, phthalazinyl, quinolyl, isoquinolyl, benzothiophen, quinazolinyl and phenylpyridyl, wherein said heterocyclyl is unsubstituted or mono-, di- or trisubstituted independently of one another by R8, or
3) a monocyclic or bicyclic 4- to 15-membered heterocyclyl, containing one, two, three or four heteroatoms chosen from nitrogen, sulfur or oxygen,

wherein said heterocyclyl is unsubstituted or mono-, di- or trisubstituted independently of one another by R8, and which is additionally substituted by a monocyclic or bicyclic 4- to 15-membered heterocyclyl, containing one, two, three or four heteroatoms chosen from nitrogen, sulfur or oxygen,
wherein heterocyclyl is unsubstituted or mono-, di- or trisubstituted independently of one another by R8,

R8 is 1) halogen,
2) $-NO_2$,
3) -CN,
4) $-C(O)-NH_2$,
5) -OH,
6) $-NH_2$,
7) $-O-CF_3$
8) a monocyclic or bicyclic 6- to 14-membered aryl, wherein aryl is mono-, di- or trisubstituted independently of one another by halogen or-O-($C_1$-$C_8$)-alkyl,
9) -($C_1$-$C_8$)-alkyl, wherein alkyl is unsubstituted or mono-, di- or trisubstituted independently of one another by halogen, $NH_2$, -OH or a methoxy residue,
10) -O-($C_1$-$C_8$)-alkyl, wherein alkyl is unsubstituted or mono-, di- or trisubstituted independently of one another by halogen, $NH_2$, -OH or a methoxy residue,
11) $-SO_2-CH_3$ or
12) $-SO_2-CF_3$,

provided that R8 is at least one halogen, $-C(O)-NH_2$ or-O-($C_1$-$C_8$)-alkyl residue, if $R^0$ is a monocyclic or bicyclic 6- to 14-membered aryl,
the substructure

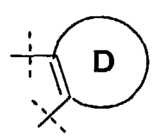

in formulae I is
a 4-to 8 membered saturated, partially unsaturated or aromatic cyclic group containing zero, 1, 2, 3 or 4 heteroatoms chosen from nitrogen, sulfur or oxygen and is unsubstituted or substituted 1, 2, 3, 4, 5 or 6 times by R3,

Q is a direct bond, -($C_0$ -$C_2$)-alkylene-C(O)-$NR^{10}$-, -$NR^{10}$-C(O)-$NR^{10}$-, -$NR^{10}$-C(O)-, $-SO_2-$, -($C_1$-$C_6$)-alkylene, $-(CH_2)_m-NR^{10}-C(O)-NR^{10}-(CH_2)_n-$, $-(CH_2)_m-NR^{10}-C(O)-(CH_2)_n-$, $-(CH_2)_m-S-(CH_2)_n-$, $-(CH_2)_m-C(O)-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10}-SO_2-NR^{10}-(CH_2)_n-$, $-(CH_2)_m-CH(OH)-(CH_2)_n-$, $-(CH_2)_m-O-C(O)-NR^{10}-(CH_2)_n-$, -($C_2$-$C_3$)-alkylene-

$O-(C_0-C_3)$-alkylene-, $-(C_2-C_3)$-alkylene-S(O)-, $-(C_2-C_3)$-alkylene-$S(O)_2$-, $-(CH_2)_m-NR^{10}$-C(O)-O-$(CH_2)_n$-, $-(C_2-C_3)$-alkylene-$S(O)_2$-NH-($R^{10}$)-, $-(C_2-C_3)$-alkylene-N($R^{10}$)- or $-(C_0-C_3)$-alkylene-C(O)-O-,
wherein $R^{10}$ is as defined below, and wherein n and m are independently of one another identical or different and are the integers zero, 1,2, 3, 4, 5 or 6, wherein the alkylene residues which are formed by $-(CH_2)_m$- or $-(CH_2)_n$- are unsubstituted or mono-, di- or trisubstituted independently of one another by halogen, $-NH_2$ or -OH; or $-(C_3-C_6)$-cycloalkylen, wherein cycloalkylen is unsubstituted or mono-, di- or trisubstituted independently of one another by halogen, $-NH_2$ or -OH;

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-$R^{10}$, a monocyclic or bicyclic 6- to 14-membered aryl, wherein aryl is mono-, di- or trisubstituted independently of one another by R8, wherein R8 is as defined above; a monocyclic or bicyclic 4- to 15-membered heterocyclyl, containing one, two, three or four heteroatoms chosen from nitrogen, sulfur or oxygen; $-(C_1-C_3)$-perfluoroalkylene, $-(C_1-C_3)$-alkylene-S(O)-$(C_1-C_4)$-alkyl, $-(C_1-C_3)$-alkylene-$S(O)_2$-$(C_1-C_3)$-alkyl, $-(C_1-C_3)$-alkylene-$S(O)_2$-N($R^{4'}$)-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a 3- to 7- membered cyclic residue, containing up to 1, 2, 3 or 4 heteroatoms chosen from nitrogen, sulfur or oxygen, wherein said cyclic residue is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$ and $R^3$ together with the atoms to which they are bonded can form a 6- to 8-membered cyclic group, containing 1, 2, 3 or 4 heteroatoms chosen from nitrogen, sulfur or oxygen, wherein said cyclic group is unsubstituted or mono-, di- or trisubstituted independently of one another by R14, or

$R^1$-N-$R^2$-V can form a 4- to 8-membered cyclic group, containing 1, 2, 3 or 4 heteroatoms chosen from nitrogen, sulfur or oxygen, wherein said cyclic group is unsubstituted or mono-, di- or trisubstituted independently of one another by R14,

R14 is halogen, -OH, =O, $-(C_1-C_8)$-alkyl, $-(C_1-C_4)$-alkoxy, $-NO_2$, -C(O)-OH, -CN, $-NH_2$, - C(O)-O-$(C_1-C_4)$-alkyl, $-(C_1-C_8)$-alkylsulfonyl, $-SO_2$-N($R^{18}$)-$R^{21}$, -C(O)-NH-$(C_1-C_8)$-alkyl, -C(O)-N-[$(C_1-C_8)$-alkyl]$_2$, $-NR^{18}$-C(O)-NH-$(C_1-C_8)$-alkyl, -C(O)-$NH_2$, -S-$R^{18}$, or $-NR^{18}$-C(O)-NH-[$(C_1-C_8)$-alkyl]$_2$,
wherein $R^{18}$ and $R^{21}$ are independently from each other hydrogen atom, $-(C_1-C_3)$-perfluoroalkyl or $-(C_1-C_6)$-alkyl,

V is 1) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14,
2) a 6- to 14-membered aryl, wherein aryl is unsubstituted or mono-, di- or trisubstituted independently of one another by R14, or
3) a monocyclic or bicyclic 4- to 15-membered heterocyclyl, wherein said heterocyclyl is unsubstituted or mono-, di- or trisubstituted independently of one another by R14,

G is a direct bond, $-(CH_2)_m-NR^{10}-SO_2-NR^{10}-(CH_2)_n$-, $-(CH_2)_m$-CH(OH)-$(CH_2)_n$-, $-(CH_2)_m$-, $-(CH_2)_m$-O-$(CH_2)_n$-, $-(CH_2)_m$-C(O)-$NR^{10}$-$(CH_2)_{n-}$, $-(CH_2)$-$SO_2$-$(CH_2)_n$-, $-(CH_2)_m-NR^{10}$-C(O)-$NR^{10}$-$(CH_2)_n$-, $-(CH_2)_m-NR^{10}$-C(O)-$(CH_2)_n$-, $-(CH_2)_m$-C(O)-$(CH_2)_n$-, $-(CH_2)$-S-$(CH_2)_n$-, $-(CH_2)_m-SO_2-NR^{10}$-$(CH_2)_n$-, $-(CH_2)_m-NR^{10}-SO_2-(CH_2)_n$-, $-(CH_2)_m-NR^{10}$-, $-(CH_2)_m$-O-C(O)-$NR^{10}$-$(CH_2)_n$- or $-(CH_2)_m-NR^{10}$-C(O)-O-$(CH_2)_n$-,

n and m are independently of one another identical or different and are the integers zero, 1, 2, 3, 4, 5 or 6,

M is 1) a hydrogen atom,

2) -($C_1$-$C_8$)-alkyl, wherein alkyl is unsubstituted or mono-, di- or trisubstituted independently of one another by R14,
3) -C(0)-N(R11)-R12,
4) -($CH_2$)$_m$-$NR^{10}$,
5) a 6- to14-membered aryl, wherein aryl is unsubstituted or mono-, di- or trisubstituted independently of one another by R14,
6) a monocyclic or bicyclic 4- to 15-membered heterocyclyl, wherein heterocyclyl is unsubstituted or mono-, di- or trisubstituted independently of one another by R14,
7) -($C_3$-$C_8$)-cycloalkyl, wherein said cycloalkyl is unsubstituted or mono-, di- or trisubstituted independently of one another by R14, or
8) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14, wherein R14 is defined above,

R3 is

1) hydrogen atom,
2) halogen,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently. of one another by R13,
6) -($C_0$-$C_4$)-alkylene-O-R19, wherein R19 is

a) hydrogen atom,
b) -($C_1$-$C_4$)-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) -$CF_3$, or
e) -$CHF_2$,

7) -$NO_2$,
8) -CN,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O)-($C_1$-$C_4$)-alkyl,
18) -C(O)-O-C(R15, R16)-0-C(0)-R17,
19) -($C_0$-$C_2$)alkylene-C(O)-O-($C_2$-$C_4$)-alkylene-O-C(O)-O-($C_1$-$C_6$)-alkyl,
20) -C(0)-0- C(R15, R16)-O-C(O)-O-R17,
21) -($C_0$-$C_4$)-alkylene-($C_6$-$C_{14}$)-aryl, wherein aryl is mono-, di- or trisubstituted independently of one another by R13,
22) -($C_0$-$C_4$)-alkylene-($C_4$-$C_{15}$)-heterocyclyl, wherein heterocyclyl is unsubstituted or mono-, di- or trisubstituted independently of one another by R13
23) -($C_0$-$C_4$)-alkylene-($C_3$-$C_8$)-cycloalkyl, wherein cycloalkyl is unsubstituted or mono-, di- or trisubstituted independently of one another by R13,
24) -($C_0$-$C_4$)-alkylene-het, wherein het is unsubstituted or mono-, di- or trisubstituted independently of one another by R13, or
25) a residue from the following list

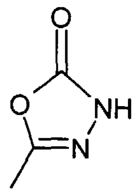 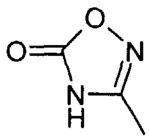 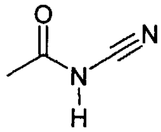 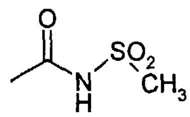 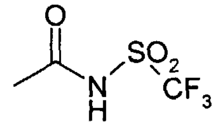

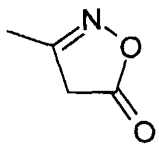 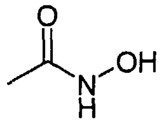 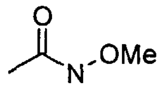 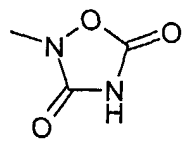 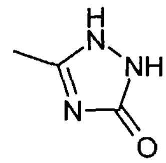

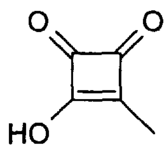 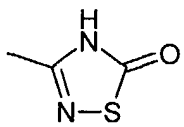 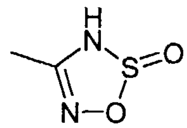 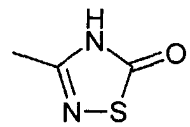 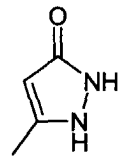 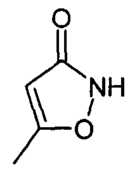

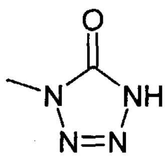 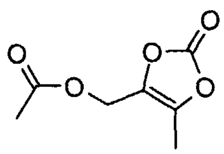 and 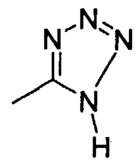 .

wherein Me is methyl, or

if two -OR19 residues are attached to adjacent atoms they can form together with the atoms which they are attached to a 5- or 6- membered ring, which is unsubstituted or substituted one, two, three or four times by R13,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_3$-$C_8$)-cycloalkyl,
4) -$SO_t$-$R^{10}$, wherein t is 1 or 2,
5) -($C_0$-$C_6$)-alkyl-($C_6$-$C_{14}$)-aryl, wherein alkyl and aryl independently from one another are unsubstituted or mono-, di- or trisubstituted by R13,
6) -($C_1$-$C_3$)-perfluoroalkyl,
7) -O-$R^{17}$, or
8) -($C_0$-$C_6$)-alkyl-($C_4$-$C_{15}$)-heterocyclyl, wherein alkyl and heterocyclyl independently from one another are unsubstituted or mono-, di- or trisubstituted by R13, or

R11 and R12 together with the nitrogen atom to which they are bonded can form a 4- to 8- membered monocyclic heterocyclic ring which in addition to the nitrogen atom can contain one or two identical or different ring heteroatoms chosen from oxygen, sulfur and nitrogen; wherein said heterocyclic ring is unsubstituted or mono-, di- or trisubstituted independently of one another by R13,

R13 is halogen, -$NO_2$, -CN, =O, -OH, -$CF_3$, -C(O)-O-$R^{10}$, -C(O)-N($R^{10}$)-$R^{20}$, -N($R^{10}$)-$R^{20}$, - ($C_3$-$C_8$)-cycloalkyl, -($C_0$-$C_3$)-alkylene-O-$R^{10}$, -Si-$(CH_3)_3$, -N($R^{10}$)-$S(O)_u$-$R^{10}$, wherein u is 1 or 2, -S-$R^{10}$, -$SO_r$-$R^{10}$, wherein r is 1 or 2, -$S(O)_v$-N($R^{10}$)-$R^{20}$, wherein v is 1 or 2, -C(O)-$R^{10}$, -($C_1$-$C_8$)-alkyl, -($C_1$-$C_8$)-alkoxy, phenyl, phenyloxy-, - 0-$CF_3$, -($C_0$-$C_4$)-alkyl-C(O)-O-C(R15, R16)-0-C(0)-R17, -($C_1$-$C_4$)-alkoxy-phenyl, - ($C_0$-$C_4$)-alkyl-C(O)-O-C(R15,R16)-O-C(O)-0-R17,-($C_1$-$C_3$)-perfluoroalkyl, -0-R15, -NH-C(O)-NH-$R^{10}$, -NH-C(O)-O-$R^{10}$, or a residue from the following list

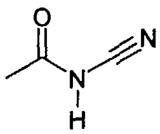 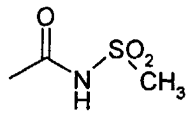 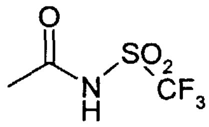 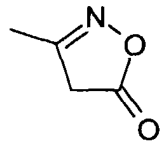 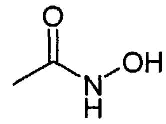

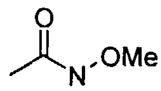 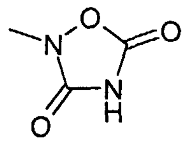 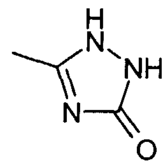 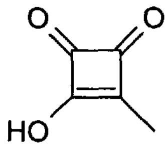 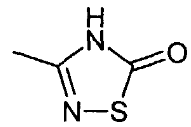

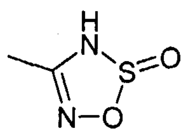 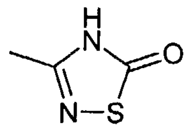 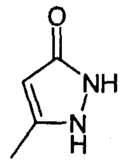 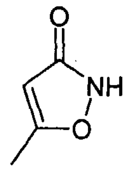 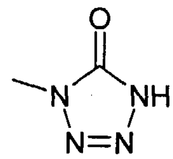

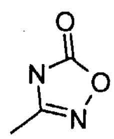 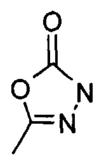 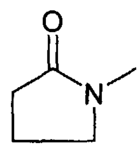 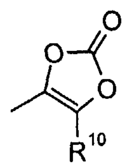 and 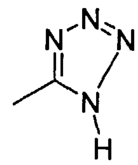 ,

$R^{10}$ and $R^{20}$ are independently of one another hydrogen, $-(C_1-C_6)$-alkyl, $-(C_O-C_4)$-alkyl-OH, - $(C_0-C_4)$-alkyl-O-$(C_1-C_4)$-akyl or $-(C_1-C_3)$-perfluoroalkyl,

R15 and R16 are independently of one another hydrogen, $-(C_1-C_6)$-alkyl, or together with the carbon atom to which they are bonded they can form a 3- to 6 membered carbocyclic ring which is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$,

in all its stereoisomeric forms and mixtures thereof in any ratio, and its physiologically tolerable salts.

2) The present invention also relates to compounds of the formula I,
wherein

$R^0$ is 1) a monocyclic or bicyclic 6- to 14-membered aryl, wherein aryl is mono-, di- or trisubstituted independently of one another by R8,
2) a monocyclic or bicyclic 4- to 15-membered heterocyclyl out of the group benzothiophen, indazolyl, indolyl, isoindolyl, isoquinolyl, phenylpyridyl, phthalazinyl, pyridyl, pyridinyl, pyrimidinyl, quinazolinyl and quinolyl, wherein said heterocyclyl is unsubstituted or mono-, di- or trisubstituted independently of one another by R8, or
3) a monocyclic or bicyclic 4- to 15-membered heterocyclyl, containing one, two, three or four heteroatoms chosen from nitrogen, sulfur or oxygen,

wherein said heterocyclyl is unsubstituted or mono-, di- or trisubstituted independently of one another by R8, and which is additionally substituted by a monocyclic or bicyclic 4- to 15-membered heterocyclyl, containing one, two, three or four heteroatoms chosen from nitrogen, sulfur or oxygen,
wherein heterocyclyl is unsubstituted or mono-, di- or trisubstituted independently of one another by R8,

R8 is 1) halogen,
2) $-NO_2$,
3) -CN,

4) $-C(O)-NH_2$,
5) -OH,
6) $-NH_2$,
7) $-O-CF_3$
8) a monocyclic or bicyclic 6- to 14-membered aryl, wherein aryl is mono-, di- or trisubstituted independently of one another by halogen or $-O-(C_1-C_8)$-alkyl,
9) $-(C_1-C_8)$-alkyl, wherein alkyl is unsubstituted or mono-, di- or trisubstituted independently of one another by halogen, $NH_2$, -OH or a methoxy residue, or
10) $-O-(C_1-C_8)$-alkyl, wherein alkyl is unsubstituted or mono-, di- or trisubstituted independently of one another by halogen, $NH_2$, -OH or a methoxy residue,
11) $-SO_2-CH_3$ or
12) $-SO_2-CF_3$,

provided that R8 is at least one halogen, $-C(O)-NH_2$ or $-O-(C_1-C_8)$-alkyl residue, if $R^0$ is a monocyclic or bicyclic 6- to 14-membered aryl,
substructure D is a 5-to 6 membered saturated, partially unsaturated or aromatic cyclic group containing zero, 1,2, 3 or 4 heteroatoms chosen from nitrogen, sulfur or oxygen and is substituted 1, 2, 3, 4, 5 or 6 times by R3,

Q is a direct bond, $-(C_0-C_2)$-alkylene-C(O)-$NR^{10}$-, $-NR^{10}-C(O)-NR^{10}-$, $-NR^{10}-C(O)-$, $-SO_2-$, $-(C_1-C_6)$-alkylene, $-(CH_2)_m-NR^{10}-C(O)-NR^{10}-(CH_2)_n-$, $-(CH_2)_m-NR^{10}-C(O)-(CH_2)_n-$, - $(CH_2)_m-S-(CH_2)_n-$, $-(CH_2)_m-C(O)-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10}-SO_2-NR^{10}-(CH_2)_n-$,$(CH_2)_m-CH(OH)-(CH_2)_n-$, $-(CH_2)_m-O-C(O)-NR^{10}-(CH_2)_n-$, $-(C_2-C_3)$-alkylene-O-$(C_O-C_3)$-alkylene-, $-(C_2-C_3)$-alkylene-S(O)-, $-(C_2-C_3)$-alkylene-$S(O)_2$-, - $(CH_2)_m-NR^{10}-C(O)-O-(CH_2)_n-$, $-(C_2-C_3)$-alkylene-$S(O)_2$-NH-$(R^{10})$-, $-(C_2-C_3)$-alkylene-N$(R^{10})$- or - $(C_0-C_3)$-alkylene-C(O)-O- ,
wherein $R^{10}$ is as defined below, and wherein n and m are independently of one another identical or different and are the integers zero, 1, 2, 3, 4, 5 or 6, wherein the alkylene residues which are formed by $-(CH_2)_m-$ or $-(CH_2)_n-$ are unsubstituted or mono-, di- or trisubstituted independently of one another by halogen, $-NH_2$ or -OH; or $-(C_3-C_6)$-cycloalkylen, wherein cycloalkylen is unsubstituted or mono-, di- or trisubstituted independently of one another by halogen, $-NH_2$ or -OH;

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-R15, a monocyclic or bicyclic 6- to 14-membered aryl, wherein aryl is mono-, di- or trisubstituted independently of one another by R8, wherein R8 is as defined above; a monocyclic or bicyclic 4- to 15-membered heterocyclyl, containing one, two, three or four 12 heteroatoms chosen from nitrogen, sulfur or oxygen; $-(C_1-C_3)$-perfluoroalkylene, $-(C_1-C_3)$-alkylene-S(O)-$(C_1-C_4)$-alkyl, $-(C_1-C_3)$-alkylene-$S(O)_2$-$(C_1-C_3)$-alkyl, $-(C_1-C_3)$-alkylene-$S(O)_2$-N(R4')-$R^{5'}$, $-(C_1-C_3)$-alkylene-O-$(C_1-C_4)$-alkyl, - $(C_0-C_3)$-alkylene-$(C_3-C_8)$-cycloalkyl, or-$(C_0-C_3)$-alkylene-het, wherein het is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$ and R3 together with the atoms to which they are bonded can form a 6- to 8-membered cyclic group, containing up to 1, 2, 3 or 4 heteroatoms chosen from nitrogen, sulfur or oxygen, wherein said cyclic group is unsubstituted or mono-, di- or trisubstituted independently of one another by R14, or

$R^1$-N-$R^2$-V can form a 4- to 8-membered cyclic group, containing 1, 2, 3 or 4 heteroatoms chosen from nitrogen, sulfur or oxygen, wherein said cyclic group is unsubstituted or mono-, di- or trisubstituted independently of one another by R14,

R14 is halogen, -OH, =O, $-(C_1-C_8)$-alkyl, $-(C_1-C_4)$-alkoxy, $-NO_2$, -C(O)-OH, -CN, $-NH_2$, - C(O) -O-$(C_1-C_4)$-alkyl, $-(C_1-C_8)$-alkylsulfonyl, $-SO_2-N(R^{18})-R^{21}$, -C(O)-NH-$(C_1-C_8)$-alkyl, -C (O)-N-$[(C_1-C_8)\text{-alkyl}]_2$, $-NR^{18}$-C(O)-NH-$(C_1-C_8)$-alkyl, $-C(O)-NH_2$, $-S-R^{18}$, or $-NR^{18}$-C

(O)-NH-[$(C_1-C_8)$-alkyl]$_2$,

wherein $R^{18}$ and $R^{21}$ are independently from each other hydrogen atom, -$(C_1-C_3)$-perfluoroalkyl or -$(C_1-C_6)$-alkyl,

V is

1) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14,
2) a 6- to 14-membered aryl, wherein aryl is unsubstituted or mono-, di- or trisubstituted independently of one another by R14, or
3) a monocyclic or bicyclic 4- to 15-membered heterocyclyl, wherein said heterocyclyl is unsubstituted or mono-, di- or trisubstituted independently of one another by R14,

G is

a direct bond, -$(CH_2)$m-$NR^{10}$-$SO_2$-$NR^{10}$-$(CH_2)_n$-, -$(CH_2)_m$-CH(OH)-$(CH_2)_n$-, -$(CH_2)_m$- -$(CH_2)_m$-O-$(CH_2)_n$-, -$(CH_2)$m-C(O)-$NR^{10}$-$(CH_2)_{n-}$ -$(CH_2)$-$SO_2$-$(CH_2)_n$-, - $(CH_2)_m$-$NR^{10}$-C(O)-$NR^{10}$-$(CH_2)_n$-, -$(CH_2)_m$-$NR^{10}$-C(O)-$(CH_2)_n$-, $(CH_2)_m$-C(O)-$(CH_2)_n$-, -$(CH_2)$-S-$(CH_2)_n$-, -$(CH_2)_m$-$SO_2$-$NR^{10}$-$(CH_2)_n$-, -$(CH_2)_m$-$NR^{10}$-$SO_2$-$(CH_2)_n$-, -$(CH_2)_m$-$NR^{10}$-, -$(CH_2)_m$-O-C(O)-$NR^{10}$-$(CH_2)_n$- or-$(CH_2)_m$-$NR^{10}$-C(O)-O$(CH_2)_n$-,

n and m are independently of one another identical or different and are the integers zero, 1, 2, 3, 4, 5 or 6,

M is

1) a hydrogen atom,
2) -$(C_1-C_8)$-alkyl, wherein alkyl is unsubstituted or mono-, di- or trisubstituted independently of one another by R14,
3) -C(O)-N(R11)-R12,
4) -$(CH_2)_m$-$NR^{10}$,
5) -$(C_6-C_{14})$-aryl, wherein aryl is unsubstituted or mono-, di- or trisubstituted independently of one another by R14,
6) -$(C_4-C_{15})$-heterocyclyl, wherein heterocyclyl is unsubstituted or mono-, di- or trisubstituted independently of one another by R14,
7) -$(C_3-C_8)$-cycloalkyl, wherein said cycloalkyl is unsubstituted or mono-, di- or trisubstituted independently of one another by R14, or
8) a 3- to 7-membered cyclic residue, containing up to 1, 2, 3 or 4 heteroatoms chosen from nitrogen, sulfur or oxygen, wherein said cyclic residue is unsubstituted or mono-, di- or trisubstituted independently of one another by R14, wherein R14 is defined above,

R3 is

1) hydrogen atom,
2) halogen,
3) -$(C_1-C_4)$-alkyl, wherein alkyl is unsubstituted or mono-, di- or trisubstituted independently of one another by R13,
4) -$(C_1-C_3)$-perfluoroalkyl,
5) phenyl, wherein phenyl is unsubstituted or mono-, di- or trisubstituted independently of one another by R13,
6) -$(C_0-C_4)$-alkylene-O-R19, wherein R19 is

a) hydrogen atom,
b) -$(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) -$CF_3$,
e) -$CHF_2$,

7) -$NO_2$,
8) -CN,
9) -$SO_s$-$R^{11}$, wherein s is 1 or 2,
10) -$SO_t$-N($R^{11}$)-$R^{12}$, wherein t is 1 or 2,

11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -$NR^{10}$-$SO_2$-$R^{10}$,
16) -S-$R^{10}$,
17) -($C_0$-$C_2$)alkylene-C(O)-O-($C_2$-$C_4$)-alkylene-O-C(O)-($C_1$-$C_4$)-alkyl,
18) -C(O)-O-C(R15, R16)-O-C(O)-R17,
19) -($C_0$-$C_2$)alkylene-C(O)-O-($C_2$-$C_4$)-alkylene-O-C(O)-O-($C_1$-$C_6$)-alkyl,
20) -C(O)-O- C(R15, R16)-O-C(O)-O-R17,
21) -($C_0$-$C_4$)-alkylene-($C_6$-$C_{14}$)-aryl, wherein aryl is mono-, di- or trisubstituted independently of one another by R13,
22) -($C_0$-$C_4$)-alkylene-($C_4$-$C_{15}$)-heterocyclyl, wherein heterocyclyl is unsubstituted or mono-, di- or trisubstituted independently of one another by R13
23) -($C_0$-$C_4$)-alkylene-($C_3$-$C_8$)-cycloalkyl, wherein cycloalkyl is unsubstituted or mono-, di- or trisubstituted independently of one another by R13,
24) -($C_0$-$C_4$)-alkylene-het, wherein het is unsubstituted or mono-, di- or trisubstituted independently of one another by R13, or
25) a residue from the following list

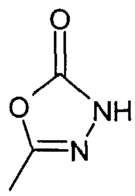
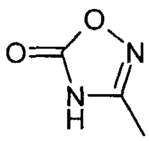
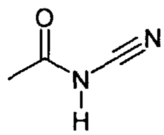
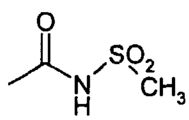
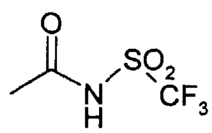
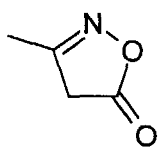
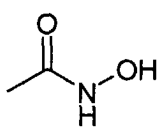
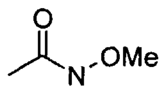
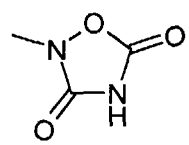
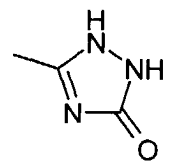
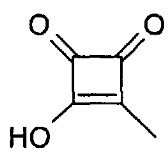
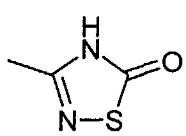
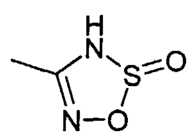
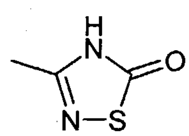
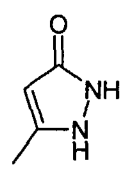
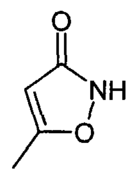
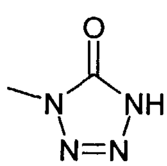
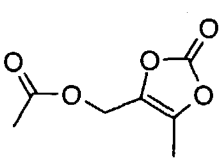
and
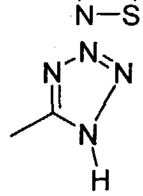

wherein Me is methyl, or

if two -OR19 residues are attached to adjacent atoms they can form together with the atoms which they are attached to a 5- or 6- membered ring, which is unsubstituted or substituted one, two, three or four times by R13,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_3$-$C_8$)-cycloalkyl,
4) -$SO_t$-$R^{10}$, wherein t is 1 or 2,
5) -($C_0$-$C_6$)-alkyl-($C_6$-$C_{14}$)-aryl, wherein alkyl and aryl independently from one another are unsubstituted or mono-, di- or trisubstituted by R13,
6) -($C_1$-$C_3$)-perfluoroalkyl,
7) -O-$R^{17}$, or
8) -($C_0$-$C_6$)-alkyl-($C_4$-$C_{15}$)-heterocyclyl, wherein alkyl and heterocyclyl independently from one another are unsubstituted or mono-, di- or trisubstituted by R13, or

R11 and R12 together with the nitrogen atom to which they are bonded can form a 4- to 8- membered monocyclic heterocyclic ring which in addition to the nitrogen atom can contain one or two identical or different ring heteroatoms chosen from oxygen, sulfur and nitrogen; wherein said heterocyclic ring is unsubstituted or mono-, di- or trisubstituted independently of one another by R13,

R13 is halogen, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_3$-$C_8$)-cycloalkyl, -($C_0$-$C_3$)-alkylene-O-$R^{10}$, -Si-$(CH_3)_3$, -N($R^{10}$)-$S(O)_u$-$R^{10}$, wherein u is 1 or 2, -S-$R^{10}$, -$SO_r$-$R^{10}$, wherein r is 1 or 2, -$S(O)_v$-N($R^{10}$)-$R^{20}$, wherein v is 1 or 2, -C(O)-$R^{10}$, -($C_1$-$C_8$)-alkyl, -($C_1$-$C_8$)-alkoxy, phenyl, phenyloxy-, -O-$CF_3$, -($C_0$-$C_4$)-alkyl-C(O)-O-C(R15, R16)-O-C(O)-R17, -($C_1$-$C_4$)-alkoxy-phenyl, -($C_0$-$C_4$)-alkyl-C(O)-O-C(R15, R16)-O-C(O)-O-R17, -($C_1$-$C_3$)-perfluoroalkyl, -O-R15, -NH-C(O)-NH-$R^{10}$, -NH-C(O)-O-$R^{10}$, or a residue from the following list

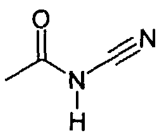 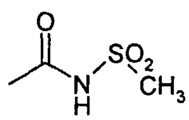 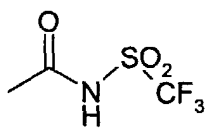 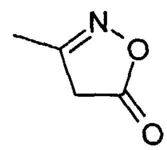 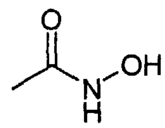

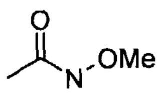 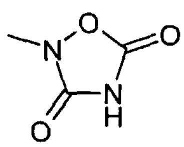 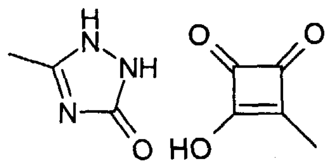 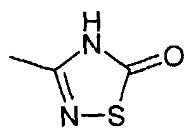

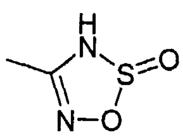 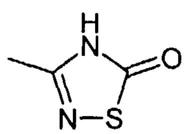 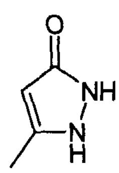 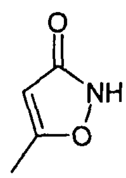 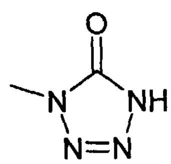

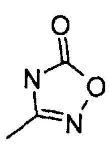 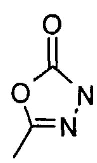 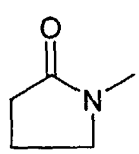 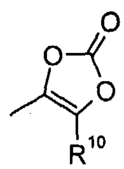 and 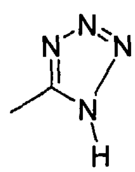 ,

$R^{10}$ and $R^{20}$ are independently of one another hydrogen, -($C_1$-$C_6$)-alkyl or -($C_1$-$C_3$)-perfluoroalkyl,

R15 and R16 are independently of one another hydrogen, -($C_1$-$C_6$)-alkyl, or together with the carbon atom to which they are bonded they can form a 3- to 6 membered carbocyclic ring which is unsubstituted or substituted one to three times by $R^{10}$, and

R17 is -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$,

in all its stereoisomeric forms and mixtures thereof in any ratio, and its physiologically tolerable salts

3) Thus, the present invention relates to compounds of the formula I, wherein

$R^0$ is 1) a monocyclic or bicyclic 6- to 14-membered aryl out of the group phenyl, naphthyl, biphenylyl, anthryl or fluorenyl, wherein aryl is mono-, di- or trisubstituted independently of one another by R8,

2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, phenylpyridyl, phthalazinyl, pteridinyl, purinyl, pyridinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, wherein said heterocyclyl is unsubstituted or mono-, di- or trisubstituted independently of one another by R8, or
3) a heterocyclyl, wherein heterocyclyl is selected out of the group acridinyl, azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1 ,3,4-oxadiazolyl, 1 ,2-oxa-thiepanyl, 1,2-oxathiolanyl, 1 ,4-oxazepanyl, 1,2-oxazinyl, 1 ,3-oxazinyl, 1 ,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, 1,4,5,6-tetrahydro-pyridazinyl, tetrahyd ropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,4-triazolyl, 1,2,5-triazolyl, 1,3,4-triazolyl and xanthenyl,

wherein said heterocyclyl is unsubstituted or mono-, di- or trisubstituted independently of one another by R8, and
which is additionally substituted by a heterocyclyl selected out of the group acridinyl, azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1 ,3-dioxolanyl, 1 ,3-dioxolenyl, 6H-1,5,2-dithiaziny), dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1 ,2-oxa-thiepanyl, 1,2-oxathiolanyl, 1,4-oxazepanyl, 1,2-oxazinyl, 1,3-oxazinyl, 1 ,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, 1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,4-triazolyl, 1,2,5-triazolyl, 1,3,4-triazolyl and xanthenyl,
wherein heterocyclyl is unsubstituted or mono-, di- or trisubstituted independently of one another by R8,

R8 is
1) halogen,
2) $-NO_2$,
3) -CN,
4) $-C(O)-NH_2$,
5) -OH,
6) $-NH_2$,
7) $-O-CF_3$
8) a monocyclic or bicyclic 6- to 14-membered aryl, wherein aryl is as defined above and wherein aryl is mono-, di- or trisubstituted independently of one another by halogen or $-O-(C_1-C_8)$-alkyl,
9) $-(C_1-C_8)$-alkyl, wherein alkyl is unsubstituted or mono-, di- or trisubstituted independently of one another by halogen, $NH_2$, -OH or a methoxy residue, or
10) $-O-(C_1-C_8)$-alkyl, wherein alkyl is unsubstituted or mono-, di- or trisubstituted independently of one another by halogen, $NH_2$, -OH or a methoxy residue,
11) $-SO_2-CH_3$ or
12) $-SO_2-CF_3$,

provided that R8 is at least one halogen, $-C(O)-NH_2$ or $-O-(C_1-C_8)$-alkyl residue, if $R^0$ is a monocyclic or bicyclic 6- to 14-membered aryl, wherein aryl is as defined above,
substructure D is a residue selected out of the group azetidine, azetine, azocane, azocane-2-one, cyclobutyl, cyclooctane, cyclooctene, cyclopentyl, cyclohexyl, cycloheptyl, cyclooctyl, 1,4-diazepane, 1,2-diazepine, 1,3-diazepine, 1,4-diazepine, [1,4]diazocane, [1,2]diazocan-3-one, [1,3]diazocan-2-one, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxa-thiepane, 1,2-oxathiolan, 1,2-oxazine, 1,3-oxazine, 1,4-oxazine, oxazole, [1,4]oxazocane, [1,3]oxazocan-2-one, oxetan, oxocane, oxocan-2-one, piperazine, piperidine, phenyl, pyran, pyrazine, pyrazole, pyrazoline, pyrazolidine, pyridazine, pyridine, pyrimidine, pyrrole, pyrrolidine, pyrrolidinone, pyrroline, 5,6,7,8-tetrahydro-1H-azocin-2-one, tetrahydrofuran, tetrahydropyran, tetrahydropyridine, tetrazine, thiadiazine, thiadiazole, 1,2-thiazine, 1,3-thiazine, 1,4-thiazine, 1,3-thiazole, thiazole, thiazolidine, thiazoline, thietan, thiocane, thiocane-1,1-dioxide, thiocane-1-oxide, thiocan-2-one, thiomorpholine, thiophene, thiopyran, 1,2,3-triazine, 1,2,4-triazine, 1,3,5-triazine, 1,2,3-triazole or 1,2,4-triazole and is unsubstituted or substituted 1, 2, 3, 4, 5 or 6 times by R3,

Q
is a direct bond, $-(C_0-C_2)$-alkylene-C(O)-$NR^{10}$-, $-NR^{10}-C(O)-NR^{10}-$, $-NR^{10}-C(O)-$, $-SO_2-$, $-(C_1-C_6)$-alkylene, $-(CH_2)_m-NR^{10}-C(O)-NR^{10}-(CH_2)_n-$, $-(CH_2)_m-NR^{10}-C(O)-(CH_2)_n-$, $-(CH_2)_m-S-(CH_2)_n-$, $-(CH_2)_m-C(O)-(CH_2)_n-$, $(CH_2)_m-SO_2-NR^{10}-(CH_2)_n-$, $-(CH_2)_m-NR^{10}-SO_2-(CH_2)_n-$, $-(CH_2)_m-NR^{10}-SO_2-NR^{10}-(CH_2)_n-$, $-(CH_2)_m-CH(OH)-(CH_2)_n-$, $-(CH_2)_m-O-C(O)-NR^{10}-(CH_2)_n-$, $-(C_2-C_3)$-alkylene-O-$(C_0-C_3)$-alkylene-, $-(C_2-C_3)$-alkylene-S(O)-, $-(C_2-C_3)$-alkylene-$S(O)_2$-, $-(CH_2)_m-NR^{10}-C(O)-O-(CH_2)_n-$, $-(C_2-C_3)$-alkylene-$S(O)_2-NH-(R^{10})$-, $-(C_2-C_3)$-alkylene-$N(R^{10})$- or $-(C_0-C_3)$-alkylene-C(O)-O-,
wherein $R^{10}$ is as defined below, and wherein n and m are independently of one another identical or different and are the integers zero, 1, 2, 3, 4, 5 or 6, wherein the alkylene residues which are formed by $-(CH_2)_m-$ or $-(CH_2)_n-$ are unsubstituted or mono-, di- or trisubstituted independently of one another by halogen, $-NH_2$ or -OH; or $-(C_3-C_6)$-cycloalkylen, wherein cycloalkylen is unsubstituted or mono-, di- or trisubstituted independently of one another by halogen, $-NH_2$ or -OH;

$R^1$ is
a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-R15, an aryl out of the group phenyl, naphthyl, biphenylyl, anthryl or fluorenyl, wherein aryl is mono-, di- or trisubstituted independently of one another by R8, wherein R8 is as defined above;
a monocyclic or bicyclic 4- to 15-membered heterocyclyl,which is as defined above; $-(C_1-C_3)$-perfluoroalkylene, $-(C_1-C_3)$-alkylene-S(O)-$(C_1-C_4)$-alkyl, $-(C_1-C_3)$-alkylene-$S(O)_2$-$(C_1-C_3)$-alkyl, $-(C_1-C_3)$-alkylene-$S(O)_2$-$N(R^{4'})$-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a residue selected out of the group azepine, azetidine, aziridine, azirine, 1,4-diazapane, 1,2-diazepine, 1,3-diazepine, 1,4-diazepine, diaziridine, diazirine, dioxazole, dioxazine, dioxole, 1,3-dioxolene, 1 ,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,4-oxazepane, 1,2-oxa-thiepane, 1,2-oxathiolane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$ and R3 together with the atoms to which they are bonded can form a 6- to 8-membered cyclic residue selected out of the group azocane, azocane-2-one, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxazine, 1,3-oxazine, 1,4-oxazine, [oxocane, oxocan-2-one, piperazine, piperidine, pyran, pyrazine, pyridazine, pyrimidine or 5,6,7,8-tetrahydro-1H-azocin-2-one, wherein said cyclic group is unsubstituted or mono-, di- or trisubstituted independently of one another by R14, or

$R^1$-N-$R^2$-V can form a 4- to 8-membered cyclic group selected out of the group azepine, azetidine, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole, wherein said cyclic group is unsubstituted or mono-, di- or trisubstituted independently of one another by R14,

R14 is fluorine, chlorine, bromine, iodine, -OH, =O, $-(C_1-C_8)$-alkyl, $-(C_1-C_4)$-alkoxy, $-NO_2$, -C(O)-OH, -CN, $-NH_2$, -C(O)-O-$(C_1-C_4)$-alkyl, $-(C_1-C_8)$-alkylsulfonyl, $-SO_2-(R^{18})-R^{21}$, -C(O)-NH-$(C_1-C_8)$-alkyl, -C(O)-N-[$(C_1-C_8)$-alkyl]$_2$, $-NR^{18}$-C(O)-NH-$(C_1-C_8)$-alkyl, -C(O)-$NH_2$,-S-$R^{18}$, or-$NR^{18}$-C(O)-NH-[$(C_1-C_8)$-alkyl]$_2$,
wherein $R^{18}$ and $R^{21}$ are independently from each other hydrogen atom, $-(C_1-C_3)$-perfluoroalkyl or $-(C_1-C_6)$-alkyl,

V is 1) a monocyclic or bicyclic 6- to 14-membered aryl out of the group phenyl, naphthyl, biphenylyl, anthryl or fluorenyl, wherein aryl is mono-, di- or trisubstituted independently of one another by R14,
2) a heterocyclyl out of the group acridinyl, azaindole ( 1H-pyrrolopyridine), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 1,4-diazepane, 4,5-dihydrooxa-zolinyl, dioxazolyl, dioxazinyl, 1,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,2-oxazinyl, 1,3-oxazinyl, 1,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisochinolinyl, tetrahydrochinolinyl, 1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenyl, thiopyranyl, 1,2,3-triazinyl, 1,2,3-triazolyl, 1,2,3-triazolyl, 1,2,4-triazolyl, 1,2,5-triazolyl, 1,3,4-triazolyl and xanthenyl, wherein said heterocyclyl is unsubstituted or mono-, di- or trisubstituted independently of one another by R14,

G is a direct bond, $-(CH_2)_m-NR^{10}-SO_2-NR^{10}-(CH_2)_n-$, $-(CH_2)_m-CH(OH)-(CH_2)_n-$, $-(CH_2)_m-$, $-(CH_2)_m-O-(CH_2)_n-$, $-(CH_2)_m-C(O)-NR^{10}-(CH_2)_n-$, $-(CH_2)-SO_2-(CH_2)_n-$, $-(CH_2)_m-NR^{10}-C(O)-NR^{10}-(CH_2)_n-$, $-(CH_2)_m-NR^{10}-C(O)-(CH_2)_n-$, $-(CH_2)_m-C(O)-(CH_2)_n-$, $-(CH_2)-S-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10}-$, $-(CH_2)_m-O-C(O)-NR^{10}-(CH_2)_n-$ or $-(CH_2)_m-NR^{10}-C(O)-O-(CH_2)_n-$,

n and m are independently of one another identical or different and are the integers zero, 1, 2, 3, 4, 5 or 6,

M is
1) a hydrogen atom,
2) $-(C_1-C_8)$-alkyl, wherein alkyl is unsubstituted or mono-, di- or trisubstituted independently of one another by R14,
3) -C(O)-N(R11)-R12,
4) $-(CH_2)_m-NR^{10}$,
5) $-(C_6-C_{14})$-aryl, wherein aryl is as defined above and wherein aryl is unsubstituted or mono-, di- or trisubstituted independently of one another by R14,
6) $-(C_4-C_{15})$-heterocyclyl, wherein heterocyclyl is as defined above and is unsubstituted or mono-, di- or trisubstituted independently of one another by R14, or
7) $-(C_3-C_8)$-cycloalkyl, wherein said cycloalkyl is unsubstituted or mono-, di- or trisubstituted independently of one another by R14,

R3 is
1) hydrogen atom,
2) halogen,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_4)$-alkylene-O-R19, wherein R19 is

a) hydrogen atom,
b) $-(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) $-CF_3$, or
e) $-CHF_2$,

7) $-NO_2$,
8) -CN,
9) $-SO_S-R^{11}$, wherein s is 1 or 2,
10) $-SO_t-N(R^{11})-R^{12}$, wherein t is 1 or 2,
11) $-(C_0-C_4)$-alkylene-$C(O)-R^{11}$,
12) $-(C_0-C_4)$-alkylene-$C(O)-O-R^{11}$,
13) $-(C_0-C_4)$-alkylene-$C(O)-N(R^{11})-R^{12}$,
14) $-(C_0-C_4)$-alkylene-$N(R^{11})-R^{12}$,
15) $-NR^{10}-SO_2-R^{10}$,
16) $-S-R^{10}$,
17) $-(C_0-C_2)$alkylene-C(O)-O-$(C_2-C_4)$-alkylene-O-C(O)-$(C_1-C_4)$-alkyl,
18) -C(O)-O-C(R15, R16)-O-C(O)-R17,
19) $-(C_0-C_2)$alkylene-C(O)-O-$(C_2-C_4)$-alkylene-O-C(O)-O-$(C_1-C_6)$-alkyl,

20) -C(O)-O- C(R15, R16)-O-C(O)-O-R17,
21) -$(C_0-C_4)$-alkylene-$(C_6-C_{14})$-aryl, wherein aryl is mono-, di- or trisubstituted independently of one another by R13,
22) -$(C_0-C_4)$-alkylene-$(C_4-C_{15})$-heterocyclyl, wherein heterocyclyl is unsubstituted or mono-, di- or trisubstituted independently of one another by R13
23) -$(C_0-C_4)$-alkylene-$(C_3-C_8)$-cycloalkyl, wherein cycloalkyl is unsubstituted or mono-, di- or trisubstituted independently of one another by R13,
24) -$(C_0-C_4)$-alkylene-het, wherein het is unsubstituted or mono-, di- or trisubstituted independently of one another by R13, or
25) a residue from the following list

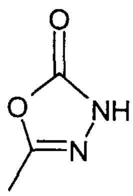
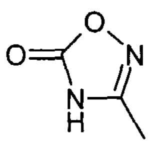
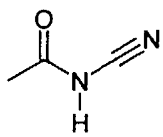
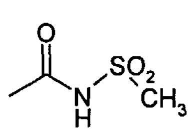
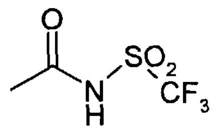
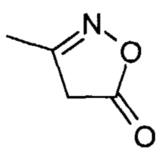
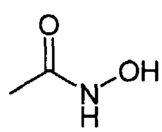
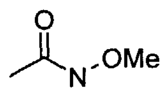
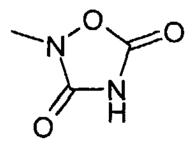
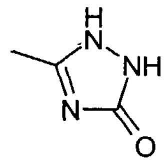
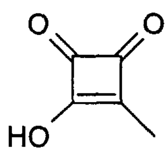
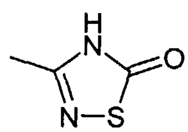
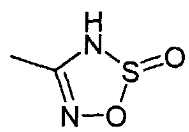
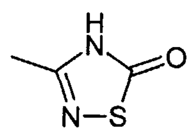
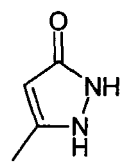
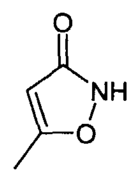
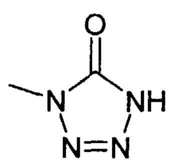
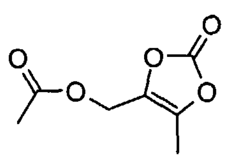
and
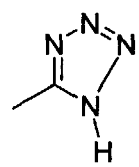

wherein Me is methyl, or

if two -OR19 residues are attached to adjacent atoms they can form together with the atoms which they are attached to a 1,3-dioxole ring or a 2,3-dihydro-[1,4]dioxine ring, which is substituted one, two, three or four times by R13,

R11 and R12 are independently of one another identical or different and are

1) hydrogen atom,
2) -$(C_1-C_6)$-alkyl, wherein alkyl is unsubstituted or mono-, di- or trisubstituted independently of one another by R13,
3) -$(C_0-C_6)$-alkyl-$(C_3-C_8)$-cycloalkyl,
4) -$SO_t$-$R^{10}$, wherein t is 1 or 2,
5) -$(C_0-C_6)$-alkyl-$(C_6-C_{14})$-aryl, wherein alkyl and aryl independently from one another are unsubstituted or mono-, di- or trisubstituted by R13,
6) -$(C_1-C_3)$-perfluoroalkyl,
7) -O-$R^{17}$, or
8) -$(C_0-C_6)$-alkyl-$(C_4-C_{15})$-heterocyclyl, wherein alkyl and heterocyclyl are as defined above and are independently from one another unsubstituted or mono-, di- or trisubstituted by R13, or

R11 and R12 together with the nitrogen atom to which they are bonded form a heterocyclic ring out of the group azepine, azetidine, dioxazole, dioxazine, 1,4-diazepane, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said heterocyclic ring is unsubstituted or mono-, di- or trisubstituted independently of one another by R13,

R13 is halogen, $-NO_2$, -CN, =O, -OH, $-CF_3$, $-C(O)-O-R^{10}$, $-C(O)-N(R^{10})-R^{20}$, $N(R^{10})-R^{20}$, $-(C_3-C_8)$-cycloalkyl, $-(C_0-C_3)$-alkylene-O-$R^{10}$, $-Si-(CH_3)_3$, $-N(R^{10})-S(O)_u-R^{10}$, wherein u is 1 or 2, $-S-R^{10}$, $-SO_r-R^{10}$, wherein r is 1 or 2, $-S(O)_v-N(R^{10})-R^{20}$, wherein v is 1 or 2, $-C(O)-R^{10}$, $-(C_1-C_8)$-alkyl, $-(C_1-C_8)$-alkoxy, phenyl, phenyloxy-, $-O-CF_3$, $-(C_0-C_4)$-alkyl-C(O)-O-C(R15, R16)-O-C(O)-R17, $-(C_1-C_4)$-alkoxy-phenyl, $-(C_0-C_4)$-alkyl-C(O)-O-C(R15, R16)-O-C(O)-O-R17, $-(C_1-C_3)$-perfluoroalkyl, -O-R15, -NH-C(O)-NH-$R^{10}$, -NH-C(O)-O-$R^{10}$, or a residue from the following list

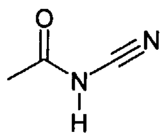 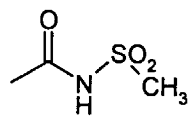 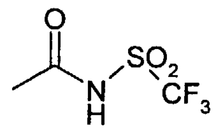 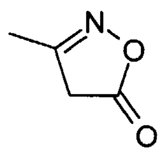 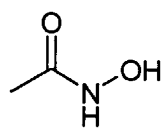

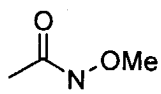 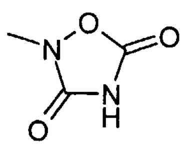 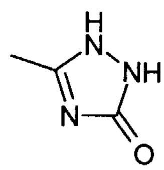 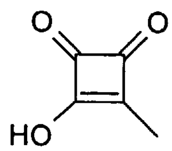 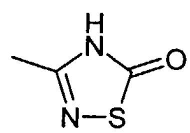

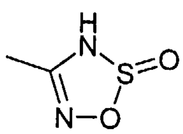 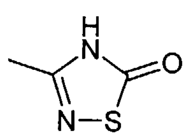 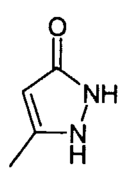 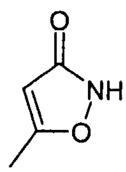 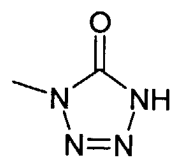

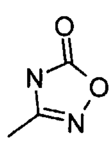 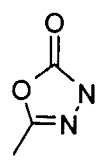 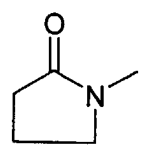 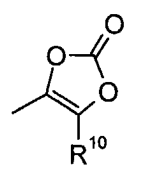 and 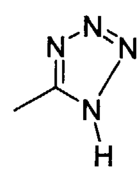 ,

$R^{10}$ and $R^{20}$ are independently of one another hydrogen, $-(C_1-C_6)$-alkyl, $-(C_0-C_4)$-alkyl-OH, - $(C_0-C_4)$-alkyl-O-$(C_1-C_4)$-akyl or $-(C_1-C_3)$-perfluoroalkyl,

R15 and R16 are independently of one another hydrogen, $-(C_1-C_6)$-alkyl, cyclopropyl, cyclobutyl, cyclopentyl or cyclohexyl, wherein each ring is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$,

in all its stereoisomeric forms and mixtures thereof in any ratio, and its physiologically tolerable salts

4) The present invention also relates to the compounds of the formula I, wherein

$R^0$ is 1) a monocyclic or bicyclic 6- to 14-membered aryl out of the group phenyl, naphthyl, biphenyl, anthryl or fluorenyl, wherein aryl is mono-, di- or trisubstituted independently of one another by R8,
2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, phenylpyridyl, phthalazinyl, pteridinyl, purinyl, pyridinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, wherein said heterocyclyl is unsubstituted or mono-, di- or trisubstituted independently of one another by R8, or
3) a heterocyclyl out of the group azabenzimidazolyl, benzimidazolyl, 1,3-benzodioxolyl, benzofuranyl, benzothiazolyl, benzothiophenyl, benzoxazolyl, chromanyl, cinnolinyl, 2-furyl, 3-furyl; imidazolyl, indolyl, indazolyl, isochromanyl, isoindolyl, isoquinolinyl, isothiazolyl, isoxazolyl, oxazolyl, phthalazinyl, pteridinyl, purinyl, pyrazinyl, pyrazolyl, pyridazinyl, pyridoimidazolyl, pyridopyridinyl, pyridopyrimidinyl, 2-pyridyl, 3-pyridyl, 4-pyridyl, pyrimidinyl, pyrrolyl; 2-pyrrolyl, 3-pyrrolyl, quinolinyl, quinazolinyl, quinoxalinyl, tetrazolyl, thiazolyl, 2-thienyl or 3-thienyl,
which is additionally substituted by a heterocyclyl selected out of the group acridinyl, azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1 ,2,5-oxadiazolyl, 1,3,4-oxadiazolyl, 1,2-oxa-thiepanyl, 1 ,2-oxathiolanyl, 1 ,4-oxazepanyl, 1,2-oxazinyl, 1 ,3-oxazinyl, 1,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisochinolinyl, tetrahydrochinolinyl, 1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenyl, thiopyranyl, 1,2,3-triazinyl, 1,2,3-triazolyl, 1,2,3-triazolyl, 1,2,4-triazolyl, 1,2,5-triazolyl, 1,3,4-triazolyl and xanthenyl,
wherein heterocyclyl is unsubstituted or mono-, di- or trisubstituted independently of one another by R8,

R8 is
1. fluor, chlor or brom,
2. $-NO_2$,
3. -CN,
4. $-C(O)-NH_2$,
5. -OH,
6. $-NH_2$,
7. $-OCF_3$
8. a monocyclic or bicyclic 6- to 14-membered aryl, wherein aryl is as defined above and is mono-, di- or trisubstituted independently of one another by halogen or $-O-(C_1-C_8)$-alkyl,
9. $-(C_1-C_8)$-alkyl, wherein alkyl is unsubstituted or mono-, di- or trisubstituted independently of one another by halogen, $NH_2$, -OH or a methoxy residue, or
10. $-O-(C_1-C_8)$-alkyl, wherein alkyl is unsubstituted or mono-, di- or trisubstituted independently of one another by halogen, $NH_2$, -OH or a methoxy residue,
11. $-SO_2CH_3$ or
12. $-SO_2CF_3$,

provided that R8 is at least one halogen, $-C(O)-NH_2$ or $-O-(C_1-C_8)$-alkyl residue, if R0 is a aryl or a heterocyclyl, which are as defined above,

substructure D is a residue selected out of the group phenyl, pyridyl, pyridyl-N-oxide pyridyl, pyrrolyl, furyl, thienyl, imidazolyl, pyrazolyl, oxazolyl, isoxazolyl, thiazolyl, triazolyl, isothiazolyl, thiadiazolyl, pyrimidinyl, pyridazinyl, pyrazinyl and is unsubstituted or substituted 1, 2, 3 or 4 times by R3,

Q is a direct bond, $-(C_0-C_2)$-alkylene-C(O)-$NR^{10}$-, $-NR^{10}$-C(O)-$NR^{10}$-, $-NR^{10}$-C(O)-, $-SO_2$-, $-(C_1-C_6)$-alkylene,

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-R15, $-(C_1-C_3)$-perfluoroalkylene, $-(C_1-C_3)$-alkylene-S(O)-$(C_1-C_4)$-alkyl, $-(C_1-C_3)$-alkylene-$S(O)_2$-$(C_1-C_3)$-alkyl, $-(C_1-C_3)$-alkylene-$S(O)_2$-$N(R^{4'})$-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a residue selected out of the group azepine, azetidine, aziridine, azirine, 1,4-diazepane, 1,2-diazepine, 1,3-diazepine, 1,4-diazepine, diaziridine, diazirine, dioxazole, dioxazine, dioxole, 1,3-dioxolene, 1 ,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxathiepane, 1,2-oxathiolane, 1,4-oxazepane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$-N-$R^2$-V form a 4- to 8-membered cyclic group selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, 1,4-oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole, wherein said cyclic group is unsubstituted or mono-, di- or trisubstituted independently of one another by R14,

R14 is fluorine, chlorine, bromine, iodine, -OH, =O, $-(C_1-C_8)$-alkyl, $-(C_1-C_4)$-alkoxy, $-NO_2$, -C(O)-OH, -CN, $-NH_2$, -C(O)-O-$(C_1-C_4)$-alkyl, $-(C_1-C_8)$-alkylsulfonyl, $-SO_2-(R^{18})-R^{21}$, -C(O)-NH-$(C_1-C_8)$-alkyl, -C(O)-N-$[(C_1-C_8)$-alkyl$]_2$, $-NR^{18}$-C(O)-NH-$(C_1-C_8)$-alkyl, -C(O)-$NH_2$, -S-$R^{18}$, or $-NR^{18}$-C(O)-NH-$[(C_1-C_8)$-alkyl$]_2$,
wherein $R^{18}$ and $R^{21}$ are independently from each other hydrogen atom, $-(C_1-C_3)$-perfluoroalkyl or-$(C_1-C_6)$-alkyl,

V is 1) a het residue out of the group azaindole ( 1H-pyrrolopyridine), azepine, azetidine, aziridine, azirine, 1,4-diazepane, 1,2-diazepine, 1,3-diazepine, 1,4-diazepine, diaziridine, diazirine, dioxazole, dioxazine, dioxole, 1,3-dioxolene, 1 ,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxathiepane, 1,2-oxathiolane, 1,4-oxazepane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine, thiadiazole, 1,2-thiazine, 1,3-thiazine, 1,4-thiazine, 1 ,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, which is as defined above and wherein het is unsubstituted or mono-, di- or trisubstituted independently of one another by R14, or
2) phenyl, wherein phenyl is unsubstituted or mono-, di- or trisubstituted independently of one another by R14,

G is a direct bond, $-(CH_2)_m-NR^{10}-SO_2-NR^{10}-(CH_2)_n-$, $-(CH_2)_m$-CH(OH)-$(CH_2)_n$-, $-CH_2)_m$-, $-(CH_2)_m$-

$O-(CH_2)_n-$, $-(CH_2)_m-C(O)-NR^{10}-(CH_2)_n-$, $-(CH_2)-SO_2-(CH_2)_n-$, $-(CH_2)_m-NR^{10}-C(O)-NR^{10}-(CH_2)_n-$, $-(CH_2)m-NR^{10}-C(O)-(CH_2)_n-$, $(CH_2)_m-C(O)-(CH_2)_n-$, $-(CH_2)-S-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10-}$,$(CH_2)m-O-C(O)-NR^{10}-(CH_2)_n-$ or-$(CH_2)_m-NR^{10}-C(O)-O-(CH_2)_n-$,

n and m are independently of one another identical or different and are the integers zero, 1, 2, 3, 4, 5 or 6,

M is
1) a hydrogen atom,
2) -$(C_1-C_8)$-alkyl, wherein alkyl is unsubstituted or mono-, di- or trisubstituted independently of one another by R14,
3) -C(O)-N(R11)-R12,
4) $-(CH_2)_m-NR^{10}$,
5) phenyl or naphthyl, wherein phenyl or naphthyl are unsubstituted or mono-, di- or trisubstituted independently of one another by R14,
6) heterocyclyl, wherein heterocyclyl is a residue out of the group which can be derived from azepane, azepine, 1,4-diazepane, 1,2-diazepine, 1,3-diazepine, 1,4-diazepine, imidazole, isothiazole, isoxazole, isoxazolidine, 2-isoxazoline, ketomorpholine, ketopiperazine, morpholine, oxazole, [1,4]-oxazepane, piperazine, piperazinone, piperidine, piperidinone, pyrazine, pyridazine, pyridazinone, pyridine, pyridone, pyrimidine, pyrrolidine, pyrrolidinone, tetrahydropyran, 1,4,5,6-tetrahydro-pyridazinyl, tetrazine, tetrazole, thiadiazole, thiazole, thiophene, thiomorpholine, 1,2,3-triazine, 1,2,4-triazine, 1,3,5-triazine, 1,2,3-triazole or 1,2,4-triazole, wherein said heterocyclyl is unsubstituted or mono-, di- or trisubstituted independently of one another by R14, or
7) -$(C_3-C_8)$-cycloalkyl, wherein said cycloalkyl is unsubstituted or mono-, di- or trisubstituted independently of one another by R14,

R3 is
1) hydrogen atom,
2) halogen,
3) -$(C_1-C_4)$-alkyl, wherein alkyl is unsubstituted or mono-, di- or trisubstituted independently of one another by R13,
4) -$(C_1-C_3)$-perfluoroalkyl,
5) phenyl, wherein phenyl is unsubstituted or mono-, di- or trisubstituted independently of one another by R13,
6) -$(C_0-C_4)$-alkylene-O-R19, wherein R19 is

a) hydrogen atom,
b) -$(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) $-CF_3$, or
e) $CHF_2$,

7) -CN,
8) -$(C_0-C_4)$-alkylene-$(C_4-C_{15})$-heterocyclyl, wherein heterocyclyl is as defined above and is unsubstituted or mono-, di- or trisubstituted independently of one another by R13,
9) $-SO_s-R^{11}$, wherein s is 1 or 2,
10) $-SO_t-N(R^{11})-R^{12}$, wherein t is 1 or 2,
11) -$(C_0-C_4)$-alkylene-$C(O)-R^{11}$,
12) -$(C_0-C_4)$-alkylene-$C(O)-O-R^{11}$,
13) -$(C_0-C_4)$-alkylene-$C(O)-N(R^{11})-R^{12}$,
14) -$(C_0-C_4)$-alkylene-$N(R^{11})-R^{12}$,
15) $-NR^{10}-SO_2-R^{10}$,
16) -$(C_0-C_4)$-alkylene-het, wherein het is as defined above and is unsubstituted or mono-, di- or trisubstituted independently of one another by R13,
17) -$(C_0-C_2)$alkylene-C(O)-O-$(C_2-C_4)$-alkylene-O-C(O)-$(C_1-C_4)$-alkyl,
18) -C(O)-O-C(R15, R16)-O-C(O)-R17,
19) -$(C_0-C_2)$alkylene-C(O)-O-$(C_2-C_4)$-alkylene-O-C(O)-O-$(C_1-C_6)$-alkyl,

20) -C(O)-O- C(R15, R16)-O-C(O)-O-R17,

21) -($C_0$-$C_4$)-alkylene-($C_6$-$C_{14}$)-aryl, wherein aryl is as defined above and is mono-, di- or trisubstituted independently of one another by R13,

22) -($C_0$-$C_4$)-alkylene-($C_3$-$C_8$)-cycloalkyl, wherein cycloalkyl is unsubstituted or mono-, di- or trisubstituted independently of one another by R13, or

23) a residue from the following list

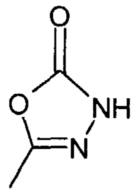
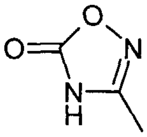
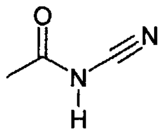
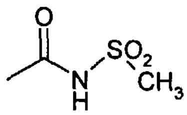
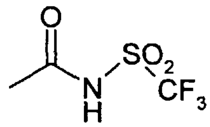
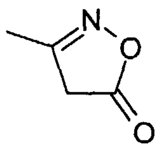
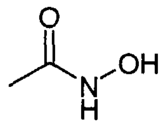
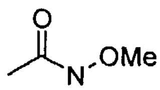
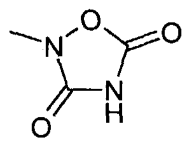
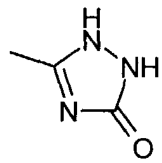
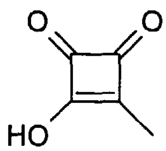
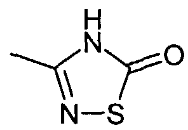
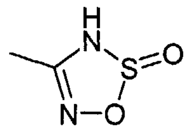
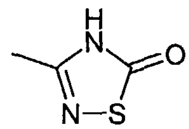
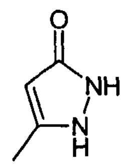
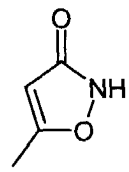
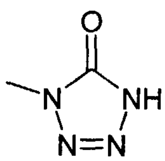
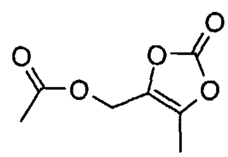
and
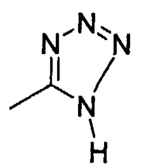

wherein Me is methyl,

if two -OR19 residues are attached to adjacent atoms they can form together with the atoms which they are attached to a 1,3-dioxole ring or a 2,3-dihydro-[1,4]dioxine ring, which is substituted one, two, three or four times by R13,

R11 and R12 are independently of one another identical or different and are

1) hydrogen atom,

2) -($C_1$-$C_6$)-alkyl, wherein alkyl is unsubstituted or mono-, di- or trisubstituted independently of one another by R13,

3) -($C_0$-$C_6$)-alkyl-($C_6$-$C_{14}$)-aryl, wherein aryl is as defined above and wherein alkyl and aryl are independently from one another unsubstituted or mono-, di- or trisubstituted by R13,

4) -O-$R^{17}$, or

5) -($C_0$-$C_6$)-alkyl-($C_4$-$C_{15}$)-heterocyclyl, wherein alkyl and heterocyclyl is as defined above and independently from one another are unsubstituted or mono-, di- or trisubstituted by R13, or

R11 and R12 together with the nitrogen atom to which they are bonded can form a ring selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole, which is unsubstituted or mono-, di- or trisubstituted independently of one another by R13,

R13 is fluorine, chlorine, bromine, iodine, $-NO_2$, -CN, =O, -OH, $-CF_3$, $-C(O)-O-R^{10}$, $-C(O)-N(R^{10})-R^{20}$, $-N(R^{10})-R^{20}$, $-(C_0-C_3)$-alkylene-$O-R^{10}$, $-Si-(CH_3)_3$, $-N(R^{10})-S(O)_2-R^{10}$, $-S-R^{10}$, $-SO_2-R^{10}$, $-S(O)_2-N(R^{10})-R^{20}$, $-C(O)-R^{10}$, $-(C_1-C_8)$-alkyl, $-(C_1-C_8)$-alkoxy, phenyl, phenyloxy-, $-O-CF_3$, $-(C_1-C_3)$-perfluoroalkyl, $-(C_0-C_4)$-alkyl-C(O)-O-C(R15, R16)-O-C(0)-R17, $-(C_1-C_4)$-alkoxy-phenyl, $-(C_0-C_4)$-alkyl-C(O)-O-C(R15, R16)-O-C(O)-O-R17, -O-R15, $-NH-C(O)-NH-R^{10}$, $-NH-C(O)-O-R^{10}$, or a residue from the following list

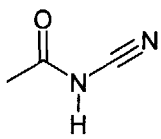 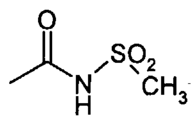 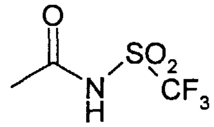 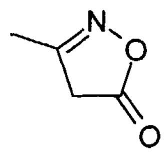 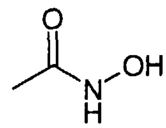

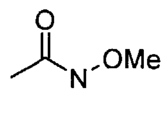 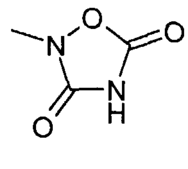 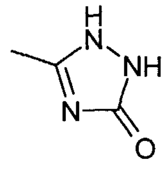 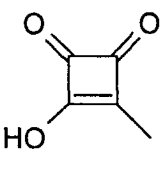 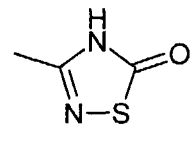

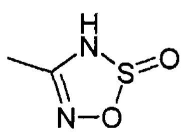 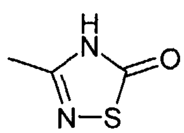 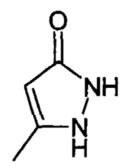 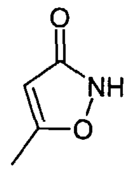 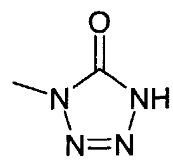

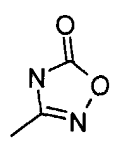 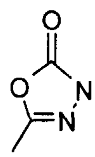 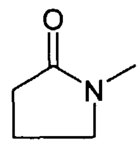 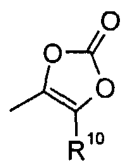 and 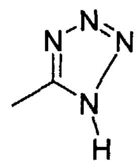 ,

$R^{10}$ and $R^{20}$ are independently of one another hydrogen, $-(C_1-C_6)$-alkyl, $-(C_0-C_4)$-alkyl-OH, $-(C_0-C_4)$-alkyl-O-$(C_1-C_4)$-akyl or $-(C_1-C_3)$-perfluoroalkyl,

R15 and R16 are independently of one another hydrogen, $-(C_1-C_6)$-alkyl, or together form a ring out of the droup cyclopropyl, cyclobutyl, cyclopentyl or cyclohexyl, wherein each ring is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$,

in all its stereoisomeric forms and mixtures thereof in any ratio, and its physiologically tolerable salts.

5) The present invention also relates to the compounds of the formula I, wherein

R0 is 1) phenyl, wherein phenyl is unsubstituted or mono-, di- or trisubstituted independently of one another by R8,
2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, phenylpyridyl, phthalazinyl, pteridinyl, purinyl, pyridinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, wherein said heterocyclyl is unsubstituted or mono-, di- or trisubstituted independently of one another by R8, or
3) a heterocyclyl out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said heterocyclyl is unsubstituted or mono-, di- or trisubstituted independently of one another by R8, and in addition is substituted by a residue selected

out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said residue is unsubstituted or mono-, di- or trisubstituted independently of one another by R8

R8 is 1. F, Cl, Br or J,
2. $-C(O)-NH_2$,
3. $-(C_1-C_4)$-alkyl, wherein alkyl is unsubstituted or mono-, di- or trisubstituted independently of one another by halogen, -OH or a methoxy residue, or
4. $-O-(C_1-C_4)$-alkyl, wherein alkyl is unsubstituted or mono-, di- or trisubstituted independently of one another by halogen or a methoxy residue,

provided that R8 is at least one halogen, $-C(O)-NH_2$ or-$O-(C_1-C_8)$-alkyl residue, if R0 is a aryl or a heterocyclyl, which are as defined above,

substructure D is a residue selected out of the group phenyl, pyridyl, pyridyl-N-oxide, pyrrolyl, furyl, thienyl, imidazolyl, pyrazolyl, oxazolyl, isoxazolyl, thiazolyl, triazolyl, isothiazolyl, thiadiazolyl, pyrimidinyl, pyridazinyl, pyrazinyl and is unsubstituted or substituted 1, 2, 3 or 4 times by R3,

Q is a direct bond, -C(O)-; $-SO_2-$ or $-(C_1-C_6)$-alkylene, $-(C_0-C_2)$-alkylene-$C(O)-NR^{10}$-,

$R^1$ is hydrogen atom, $-(C_1-C_2)$-alkyl, $-(C_1-C_3)$-alkylene-C(O)-NH-R0, $-(C_1-C_3)$-perfluoroalkylene, $-(C_1-C_3)$-alkylene-$C(O)-O-R^{15}$, $-(C_1-C_3)$-alkylene-$S(O)_2-(C_1-C_3)$-alkyl or $-(C_1-C_3)$-alkylene-$S(O)_2-N(R^{4'})-R^{5'}$, wherein $R^{4'}$ and $R^{5'}$ are independent of one another are identical or different and are hydrogen atom or $-(C_1-C_4)$-alkyl,

$R^2$ is a direct bond or $-(C_1-C_2)$-alkylene,

$R^1-N-R^2-V$ can form a 4- to 7- membered cyclic group out of the group azetidine, azetidinone, piperidine, piperazine, pyridine, pyrimidine, pyrrolidine, pyrrolidinone, 1,2,3-triazine, 1,2,4-triazine, 1,3,5-triazine, 1,2,3-triazole, 1,2,4-triazole, tetrazine, tetrazole, 1,4-diazepane, 1,2-diazepine, 1,3-diazepine, 1,4-diazepine, azepine, ketopiperazine, 1,4-oxazepane, oxazole, isoxazole, isoxazolidine, 2-isoxazoline, morpholine, thiazole, isothiazole, thiadiazole or thiomorpholine, wherein said cyclic group is unsubstituted or mono-, di- or trisubstituted independently of one another by R14,

R14 is fluorine, chlorine, -OH, =O, $-(C_1-C_8)$-alkyl, -C(O)-OH, -CN, $-NH_2$, $-C(O)-O-(C_1-C_4)$-alkyl, $-C(O)-NH-(C_1-C_8)$-alkyl, $-C(O)-N-[(C_1-C_8)\text{-alkyl}]_2$, $-C(O)-NH_2$ or $-N(R^{18})-R^{21}$, wherein $R^{18}$ and $R^{21}$ are independently from each other hydrogen atom, $-(C_1-C_3)$-perfluoroalkyl or $-(C_1-C_4)$-alkyl,

V is 1. a cyclic residue out of the group containing compounds which are derived from azaindole ( 1H-pyrrolopyridine), aziridine, azirine, azetidine, azetidinone, 1,4-diazepane, pyrrole, pyrrolidine, pyridonyl, imidazole, pyrazole, 1,2,3-triazole, 1,2,4-triazole, tetrazole, pyridine, pyrimidine, pyrazine, 1,2,3-triazine, 1,2,4-triazine, 1,3,5-triazine, tetrazine, tetrazole, azepine, diazirine, 1,2-diazepine, 1,3-diazepine, 1,4-diazepine, pyridazine, piperidine, piperazine, pyrrolidinone, ketopiperazine, furan, pyran, dioxole, 1,4-oxazepane, oxazole, isoxazole, 2-isoxazoline, isoxazolidine, morpholine, oxirane, oxaziridine, 1,3-dioxolene, 1,3-dioxolane, 1,2-oxazine, 1,3-oxazine, 1,4-oxazine, oxaziridine, thiophene, thiopyran, thietan, thiazole, isothiazole, isothiazoline, isothiazolidine, 1,2-oxathiolan, thiodiazole, thiopyran, 1,2-thiazine, 1,3-thiazole, 1,3-thiazine, 1,4-thiazine, thiadiazine or thiomorpholine,
wherein said cyclic residue is unsubstituted or mono-, di- or trisubstituted independently of one another by R14, or
2. phenyl, wherein phenyl is unsubstituted or mono-, di- or trisubstituted independently of one another by R14, or

G is a direct bond, $-(CH_2)_m$-, or $-(CH_2)_m-NR^{10}$-,

m is the integers zero, 1, 2, 3 or 4,

M is 1. a hydrogen atom,
2. heterocyclyl, wherein heterocyclyl is a residue out of the group which can be derived from azepane, azepine, 1,4-diazepane, 1,2-diazepine, 1,3-diazepine, 1,4-diazepine, imidazole, isothiazole, isoxazole, isoxazolidine, 2-isoxazoline, ketomorpholine, ketopiperazine, morpholine, oxazole, [1,4]-oxazepane, piperazine, piperazinone, piperidine, piperidinone, pyrazine, pyridazine, pyridazinone, pyridine, pyridone, pyrimidine, pyrrolidine, pyrrolidinone, tetrahydropyran, 1,4,5,6-tetrahydropyridazinyl, tetrazine, tetrazole, thiadiazole, thiazole, thiomorpholine, thiophene, 1,2,3-triazine, 1,2,4-triazine, 1,3,5-triazine, 1,2,3-triazole or 1,2,4-triazole, wherein said heterocyclyl is unsubstituted or mono-, di- or trisubstituted independently of one another by R14,
3. -$(C_1$-$C_6)$-alkyl, wherein alkyl is unsubstituted or mono-, di- or trisubstituted independently of one another by R14, or
4. $(C_3$-$C_6)$-cycloalkyl,
5. -C(O)-N($R^{11}$)-$R^{12}$,

R3 is
1) hydrogen atom,
2) halogen,
3) -$(C_1$-$C_4)$-alkyl, wherein alkyl is unsubstituted or mono-, di- or trisubstituted independently of one another by R13,
4) -$(C_1$-$C_3)$-perfluoroalkyl,
5) phenyl, wherein phenyl is unsubstituted or mono-, di- or trisubstituted independently of one another by R13,
6) -$(C_0$-$C_4)$-alkylene-O-R19, wherein R19 is

a) hydrogen atom,
b) -$(C_1$-$C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) -$CF_3$, or
e) $CHF_2$,

7) -CN,
8) -$NR^{10}$-$SO_2$-$R^{10}$,
9) -$SO_s$-$R^{11}$, wherein s is 1 or 2,
10) -$SO_t$-N($R^{11}$)-$R^{12}$, wherein t is 1 or 2,
11) -$(C_0$-$C_4)$-alkylene-C(O)-$R^{11}$,
12) -$(C_0$-$C_4)$-alkylene-C(O)-O-$R^{11}$,
13) -$(C_0$-$C_4)$-alkylene-C(O)-N($R^{11}$)-$R^{12}$,
14) -$(C_0$-$C_4)$-alkylene-N($R^{11}$)-$R^{12}$,
15) -$(C_0$-$C_2)$alkylene-C(O)-O-$(C_2$-$C_4)$-alkylene-O-C(O)-$(C_1$-$C_4)$-alkyl,
16) -C(O)-O-C(R15, R16)-O-C(O)-R17,
17) -$(C_0$-$C_2)$alkylene-C(O)-O-$(C_2$-$C_4)$-alkylene-O-C(O)-O-$(C_1$-$C_6)$-alkyl,
18) -C(O)-O- C(R15, R16)-O-C(O)-O-R17, or
19) a residue from the following list

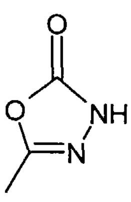
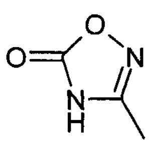
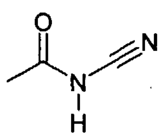
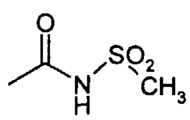
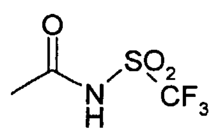
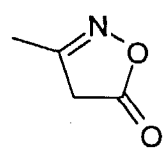

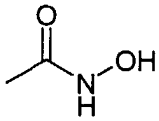
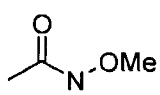
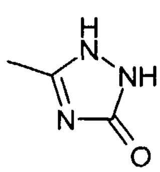
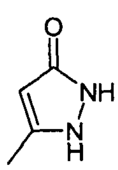
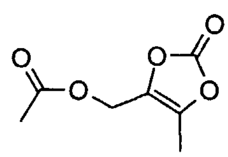
and
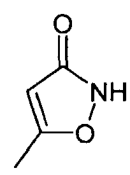

wherein Me is methyl,
if two -OR19 residues are attached to adjacent atoms they can form together with the atoms which they are attached to a 1,3-dioxole ring or a 2,3-dihydro-[1,4]dioxine ring, which is substituted one,

two, three or four times by R13,

$R^{11}$ and $R^{12}$ together with the nitrogen atom to which they are bonded can form a ring selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]-oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said ring is unsubstituted or mono-, di- or trisubstituted independently of one another by R13,

R13 is fluorine, chlorine, $-NO_2$, -CN, =O, -OH, $-CF_3$, $-C(O)-O-R^{10}$, $-C(O)-N(R^{10})-R^{20}$, $-N(R^{10})-R^{20}$, $-(C_0-C_3)$-alkylene-$O-R^{10}$, $-Si-(CH_3)_3$, $-N(R^{10})-S(O)_2-R^{10}$, $-S-R^{10}$, $-SO_2-R^{10}$, $-S(O)_2-N(R^{10})-R^{20}$, $-C(O)-R^{10}$, $-(C_1-C_8)$-alkyl, $-(C_1-C_8)$-alkoxy, phenyl, phenyloxy-, $-O-CF_3$, $-(C_1-C_3)$-perfluoroalkyl, $-NH-C(O)-NH-R^{10}$, $-(C_0-C_4)$-alkyl-C(O)-O-C(R15, R16)-O-C(O)-R17, $-(C_1-C_4)$-alkoxy-phenyl, $-(C_0-C_4)$-alkyl-C(O)-O-C(R15, R16)-O-C(O)-O-R17, -O-R15, $-NH-C(O)-O-R^{10}$, or a residue from the following list

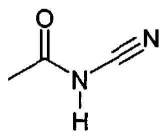 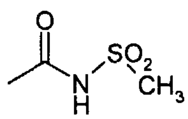 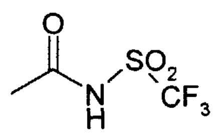 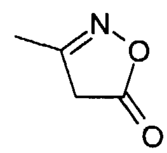 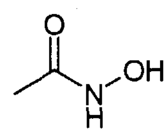

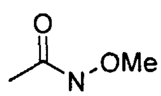 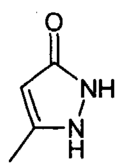 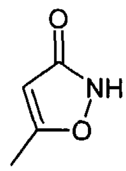 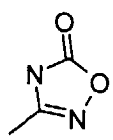 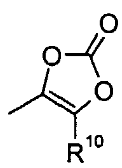 and, 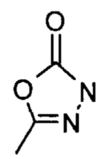

wherein Me is methyl,

$R^{10}$ and $R^{20}$ are independently of one another hydrogen, $-(C_1-C_6)$-alkyl, $-(C_0-C_4)$-alkyl-OH, $-(C_0-C_4)$-alkyl-$O-(C_1-C_4)$-akyl or $-(C_1-C_3)$-perfluoroalkyl,

R15 and R16 are independently of one another hydrogen, $-(C_1-C_6)$-alkyl, or together form a ring out of the droup cyclopropyl, cyclobutyl, cyclopentyl or cyclohexyl, wherein each ring is unsubstituted or substituted one to three times by $R^{10}$, and

R17 is $-(C_1-C_6)$-alkyl, $-(C_1-C_6)$-alkyl-OH, $-(C_1-C_6)$-alkyl-$O-(C_1-C_6)$-alkyl, $-(C_3-C_8)$-cycloalkyl, $-(C_1-C_6)$-alkyl-$O-(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, $-O-(C_1-C_4)$-alkyl or $R^{10}$,

in all its stereoisomeric forms and mixtures thereof in any ratio, and its physiologically tolerable salts.

6) The present invention also relates to the compounds of the formula I, wherein

R0 is 1. phenyl, wherein phenyl is unsubstituted or mono-, di- or trisubstituted independently of one another by R8,
2. a heterocyclyl selected out of the group indolyl, isoindolyl, benzofuranyl, benzothiophenyl, 1 ,3-benzodioxolyl, indazolyl, benzimidazolyl, benzoxazolyl, benzothiazolyl, quinolinyl, isoquinolinyl, chromanyl, isochromanyl, cinnolinyl, quinazolinyl, quinoxalinyl, phthalazinyl, pyridoimidazolyl, pyridopyridinyl, pyridopyrimidinyl, pyridinyl, purinyl and pteridinyl,

wherein said heterocyclyl is unsubstituted or mono-, di- or trisubstituted independently of one another by R8,

3. a heterocyclyl out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said heterocyclyl is unsubstituted or mono-, di- or trisubstituted independently of one another by R8,

and in addition is substituted by a residue selected out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said residue is unsubstituted or mono-, di- or trisubstituted independently of one another by R8

R8 is
1. is F, Cl, Br, J,
2. $-C(O)-NH_2$,
3. $-(C_1-C_4)$-alkyl, wherein alkyl is unsubstituted or mono-, di- or trisubstituted independently of one another by halogen, -OH or a methoxy residue, or
4. $-O-(C_1-C_4)$-alkyl, wherein alkyl is unsubstituted or mono-, di- or trisubstituted independently of one another by halogen or a methoxy residue,

provided that R8 is at least one halogen, $-C(O)-NH_2$ or $-O-(C_1-C_8)$-alkyl residue, if R0 is a aryl or a heterocyclyl, which are as defined above,

substructure D is a residue selected out of the group phenyl, pyridyl, pyridyl-N-oxide, pyrrolyl, furyl, thienyl, imidazolyl, pyrazolyl, oxazolyl, isoxazolyl, thiazolyl, triazolyl, isothiazolyl, thiadiazolyl, pyrimidinyl, pyridazinyl, pyrazinyl and is unsubstituted or substituted 1, 2, 3 or 4 times by $R^3$,

Q is a direct bond, -C(O)-; $-SO_2-$ or $-(C_1-C_6)$-alkylen, $-(C_0-C_2)$-alkylen-C(O)-$NR^{10}$-,
$R^1$ is hydrogen atom or $-(C_1-C_2)$-alkyl,
$R^2$ is a direct bond or $-(C_1-C_2)$-alkylen, or
$R^1$-N-$R^2$-V can form a 4- to 7- membered cyclic group out of the group piperidine, piperazine, pyridine, pyrimidine, pyrrolidine, pyrrolidinone, 1,2,3-triazine, 1,2,4-triazine, 1,3,5-triazine, 1,2,3-triazole, 1,2,4-triazole, tetrazine, tetrazole, 1,2-diazepine, 1,3-diazepine, 1,4-diazepine, azepine, ketopiperazine, oxazole, isoxazole, isoxazolidine, 2-isoxazoline, morpholine, thiazole, isothiazole, thiadiazole or thiomorpholine, wherein said cyclic group is unsubstituted or mono-, di- or trisubstituted independently of one another by R14,

R14 is fluoro, chlorine, $-(C_1-C_4)$-alkyl or $-NH_2$,

V is
1. a cyclic residue out of the group containing compounds, which are derived from azaindolyl (1H-pyrrolopyridyl), azetidine, azepine, aziridine, azirine, 1,4-diazepane, 1,2-diazepine, 1,3-diazepine, 1,4-diazepine, diazirine, 1 ,3-dioxolane, dioxazole, furan, imidazole, isoquinoline, isothiazole, isothiazolidine, isothiazoline, isoxazole, 2-isoxazoline, isoxazolidine, ketopiperazine, morpholine, 1,2-oxazine, 1,3-oxazine, 1,4-oxazine, oxazole, 1,2-oxathiolan, piperidine, pyran, pyrazine, pyrazole, pyridazine, piperazine, pyridine, pyridone, pyrimidine, pyrrole, pyrrolidine, pyrrolidinone, quinazoline, quinoline, tetrazine, tetrazole, thiadiazine, 1,2-thiazine, 1,3-thiazine, 1,4-thiazine, 1,3-thiazole, thietan, thiomorpholine, thiophene, thiopyran, 1,2,3-triazine, 1,2,4-triazine, 1,3,5-triazine, 1,2,3-triazole or 1,2,4-triazole,
wherein said cyclic residue is unsubstituted or mono-, di- or trisubstituted independently of one another by R14, or
2. phenyl, wherein phenyl is unsubstituted or mono-, di- or trisubstituted independently of one another by R14,

G is a direct bond, $-(CH_2)_m-$, or $-(CH_2)_m-NR^{10}-$,

m is the integers zero, 1, 2, 3 or 4,

M is
1. a hydrogen atom,
2. heterocyclyl, wherein heterocyclyl is a residue out of the group which can be derived from 1,4-diazepane, ketomorpholine, thiophene, pyridazone, piperidine, piperazine, pyridine, pyrimidine, pyrrolidine, pyrrolidinone, pyridonyl, imidazole, pyridazine, pyrazine, 1,2,3-triazine, 1,2,4-triazine, 1,3,5-triazine, 1,2,3-triazole, 1,2,4-triazole, tetrazine, tetrazole, 1,2-diazepine, 1,3-diazepine, 1,4-diazepine, azepine, ketopiperazine, oxazole, isoxazole, isoxazolidine, 2-isoxazoline, morpholine, thiazole, isothiazole, tetrahydropyran, 1,4,5,6-tetrahydro-pyridazinyl, thiadiazole or thiomorpholine, wherein said heterocyclyl is unsubstituted or mono-, di- or trisubstituted independently of one another by R14,
3. $-(C_1-C_6)$-alkyl, wherein alkyl is unsubstituted or mono-, di- or trisubstituted independently of one another by R14, or
4. $(C_3-C_6)$-cycloalkyl,

R3 is
1) hydrogen atom,
2) halogen,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_4)$-alkylene-O-R19, wherein R19 is

a) hydrogen atom,
b) $-(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) $-CF_3$, or
e) $-CHF_2$,

7) -CN,
8) $-NR^{10}-SO_2-R^{10}$,
9) $-SO_s-R^{11}$, wherein s is 1 or 2,
10) $-SO_t-N(R^{11})-R^{12}$, wherein t is 1 or 2,
11) $-(C_0-C_4)$-alkylene-C(O)-$R^{11}$,
12) $-(C_0-C_4)$-alkylene-C(O)-O-$R^{11}$,
13) $-(C_0-C_4)$-alkylene-C(O)-N($R^{11}$)-$R^{12}$,
14) $-(C_0-C_4)$-alkylene-N($R^{11}$)-$R^{12}$,
15) $-(C_0-C_2)$alkylene-C(O)-O-$(C_2-C_4)$-alkylene-O-C(O)-$(C_1-C_4)$-alkyl,
16) -C(O)-O-C(R15, R16)-O-C(O)-R17,
17) $-(C_0-C_2)$alkylene-C(O)-O-$(C_2-C_4)$-alkylene-O-C(O)-O-$(C_1-C_6)$-alkyl,
18) -C(O)-O- C(R15, R16)-O-C(O)-O-R17, or
19) a residue from the following list

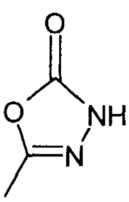
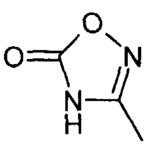
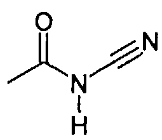
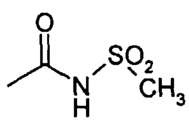
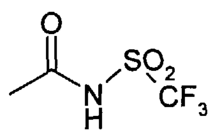
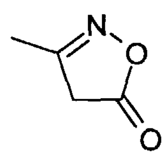
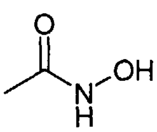
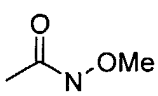
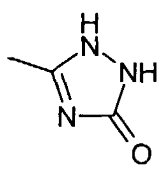
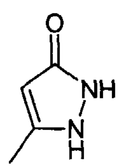
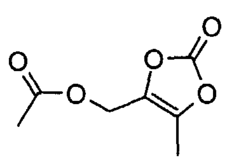
and
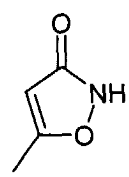

wherein Me is methyl,

R11 and R12 are independently of one another identical or different and are

1) hydrogen atom,
2) $-(C_1-C_4)$-alkyl, wherein alkyl is unsubstituted or mono-, di- or trisubstituted independently of one another by R13,
3) $-(C_0-C_6)$-alkyl-$(C_3-C_6)$-cycloalkyl,
4) -O-$R^{17}$, or
5) $-(C_0-C_6)$-alkyl-$(C_4-C_{15})$-heterocyclyl, wherein alkyl and heterocyclyl independently from one another are unsubstituted or mono-, di- or trisubstituted by R13 and wherein heterocyclyl is selected out of the group azetidine, cyclopropyl, cyclobutyl, 4,5-dihydro-oxazole, imidazolidine, morpholine, (1,4)-oxazepane, oxazolidine, piperidine, piperazine, pyrrolidine, tetrahydrothiophene, thiazolidine or thiomorpholine, or

R11 and R12 together with the nitrogen atom to which they are bonded form a heterocyclic ring, which is selected out of the group azetidine, cyclopropyl, cyclobutyl, 4,5-dihydro-oxazole, imidazolidine, morpholine, (1,4)-oxazepane, oxazolidine, piperidine, piperazine, pyrrolidine, tetrahydrothiophene, thiazolidine or thiomorpholine, wherein said ring is unsubstituted or mono-, di- or trisubstituted by R13,

R13 is fluorine, -CN, =O, -OH, $-CF_3$, -C(O)-O-$R^{10}$, -C(O)-N($R^{10}$)-$R^{20}$, -N($R^{10}$)-$R^{20}$, -($C_3$-$C_6$ )-cycloalkyl, -($C_0$-$C_3$)-alkylene-O-$R^{10}$, -Si-$(CH_3)_3$, -S-$R^{10}$, -$SO_2$-$R^{10}$, -($C_1$-$C_3$)-perfluoroalkyl, or a residue from the following list

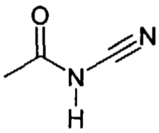 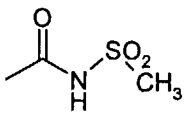 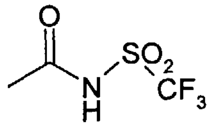 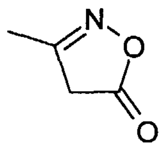 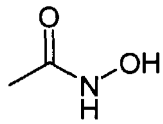 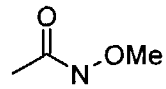

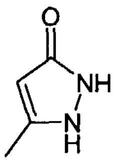 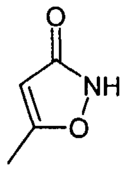 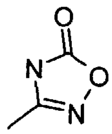 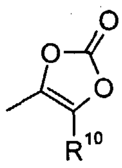 and, 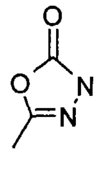

wherein Me is methyl,

$R^{10}$ and $R^{20}$ are independently of one another hydrogen, -($C_1$-$C_4$)-alkyl or -($C_1$-$C_3$)-perfluoroalkyl,

$R^{15}$ and $R^{16}$ are independently of one another hydrogen, -($C_1$-$C_4$)-alkyl, or together form a ring out of the droup cyclopropyl, cyclobutyl, cyclopentyl or cyclohexyl, wherein each ring is unsubstituted or substituted one to three times by $R^{10}$, and

R17 is -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$,

in all its stereoisomeric forms and mixtures thereof in any ratio, and its physiologically tolerable salts.

7) The present invention also relates to the compounds of the formula I, wherein

R0 is
1. phenyl, wherein phenyl is unsubstituted or mono- or disubstituted independently of one another by R8,
2. pyridyl, wherein pyridyl is unsubstituted or mono- or disubstituted independently of one another by R8, or
3. a heterocyclyl out of the group thienyl, thiadiazolyl, isoxazolyl and thiazolyl, wherein said heterocyclyl is substituted by a residue selected out of the group thienyl, 2-thienyl and 3-thienyl, wherein said residue is unsubstituted or mono- or disubstituted independently of one another by R8,

R8 is F, Cl, Br, $-OCH_3$, -C(O)-$NH_2$ or-O-$CF_3$,

substructure D is a residue selected out of the group phenyl, pyridyl, pyridyl-N-oxide, pyrrolyl, thienyl, imidazolyl, pyrazolyl, oxazolyl, isoxazolyl, thiazolyl, isothiazolyl, thiadiazolyl, pyrimidinyl, pyridazinyl or pyrazinyl and is unsubstituted or substituted 1, 2, 3 or 4 times by R3,

Q is a direct bond, -C(O)-; -$SO_2$-, -$CH_2$-C(O)-NH-, methylene or ethylene,

$R^1$ is hydrogen atom,

$R^2$ is a direct bond or methylene,

$R^1$-N-$R^2$-V can form a 4- to 8-membered cyclic group out of the group azetidine, pyrrolidine, piperidine and piperazine,

R14 is fluorine, chlorine, methyl, ethyl or -$NH_2$,

V is 1. a residue out of the group containing compounds which is derived from azaindolyl (1H-pyrrolopyridyl), azetidine, 1,4-diazepane, isoxazole, isoquinoline, piperazine, piperidine, pyrazine, pyridazine, pyrimidine, pyrrolidine, quinazoline, quinoline or tetrahydropyrane,
wherein said cyclic residue is unsubstituted or mono- or disubstituted independently of one another by R14, or

2. phenyl, wherein phenyl is unsubstituted or mono- or disubstituted independently of one another by R14,

G is a direct bond, $-(CH_2)_m-$, or $-(CH_2)_m-NR^{10}-$,

m is the integers zero, 1 or 2,

M is a hydrogen atom, $(C_2-C_4)$-alkyl, azepanyl, cyclopropyl, cyclobutyl, cyclopentyl, cyclohexyl, imidazolyl, ketomorpholinyl, morpholinyl, [1,4]Oxazepanyl, piperidinyl, piperidonyl, pyrazinyl, pyrazolyl, pyridazinyl, pyridinyl, pyrimidyl, pyrrolidinyl, 1,4,5,6-tetrahydropyridazinyl, or tetrahydropyranyl, wherein the residues are unsubstituted or mono- or disubstituted independently of one another by R14

R3 is
1) hydrogen atom,
2) fluorine, chlorine,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_2)$-alkylene-O-R19, wherein R19 is

a) hydrogen atom,
b) $-(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) $-CF_3$, or
e) $-CHF_2$,

7) -CN,
8) $-NR^{10}-SO_2-R^{10}$,
9) $-SO_s-R^{11}$, wherein s is 1 or 2,
10) $-SO_t-N(R^{11})-R^{12}$, wherein t is 1 or 2,
11) $-(C_0-C_4)$-alkylene-$C(O)-R^{11}$,
12) $-(C_0-C_4)$-alkylene-$C(O)-O-R^{11}$,
13) $-(C_0-C_4)$-alkylene-$C(O)-N(R^{11})-R^{12}$,
14) $-(C_0-C_4)$-alkylene-$N(R^{11})-R^{12}$,
15) $-(C_0-C_2)$alkylene-C(O)-O-$(C_2-C_4)$-alkylene-O-C(O)-$(C_1-C_4)$-alkyl,
16) -C(O)-O-C(R15, R16)-O-C(O)-R17,
17) $-(C_0-C_2)$alkylene-C(O)-O-$(C_2-C_4)$-alkylene-O-C(O)-O-$(C_1-C_6)$-alkyl,
18) -C(O)-O- C(R15, R16)-O-C(O)-O-R17, or
19) a residue from the following list

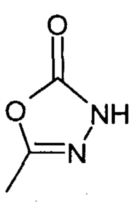 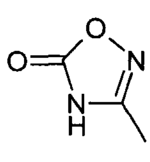 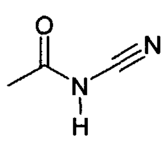 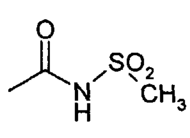 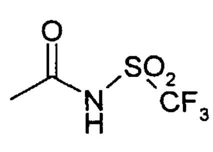 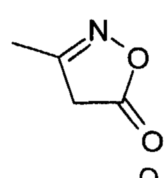

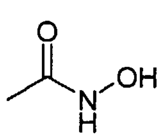 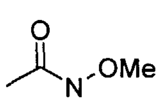 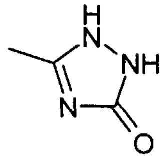 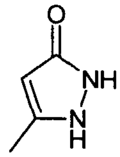 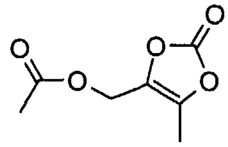 and 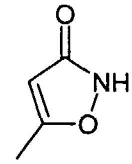

wherein Me is methyl,

R11 and R12 are independently of one another identical or different and are

1) hydrogen atom,
2) $-(C_1-C_4)$-alkyl, wherein alkyl is unsubstituted or mono-, di- or trisubstituted independently of one another by R13,
3) $-(C_0-C_6)$-alkyl-$(C_3-C_8)$-cycloalkyl,
4) -O-$R^{17}$, or
5) $-(C_0-C_6)$-alkyl-heterocyclyl, wherein alkyl and heterocyclyl independently from one another are unsubstituted or mono-, di- or trisubstituted by R13 and wherein heterocyclyl is selected out of the group azetidine, imidazolidine, morpholine, (1,4)-oxazepane or pyrrolidine or

R11 and R12 together with the nitrogen atom to which they are bonded can form a ring, which is selected out of the group azetidine, imidazolidine, morpholine, (1,4)-oxazepane piperazine, piperidine, pyrrolidine or thiomorpholine, wherein said ring is unsubstituted or mono-, di- or trisubstituted by R13,

R13 is fluorine, -CN, =O, -OH, $-CF_3$, -C(O)-O-$R^{10}$, -C(O)-N($R^{10}$)-$R^{20}$, -N($R^{10}$)-$R^{20}$, $-(C_3-C_6)$-cycloalkyl, $-(C_1-C_3)$-alkylene-O-$R^{10}$, $-Si-(CH_3)_3$, -S-$R^{10}$, $-SO_2$-$R^{10}$, $-(C_1-C_3)$-perfluoroalkyl, or a residue from the following list

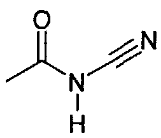
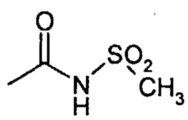
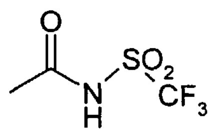
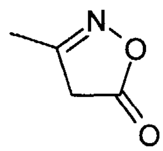
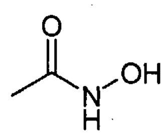
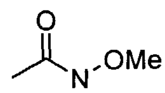
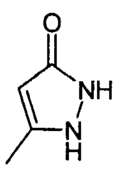
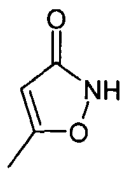
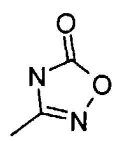
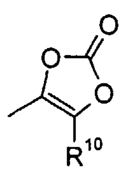
and,
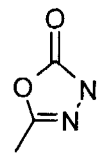

wherein Me is methyl,

$R^{10}$ and $R^{20}$ are independently of one another hydrogen, $-(C_1-C_4)$-alkyl or $-(C_1-C_3)$-perfluoroalkyl,

$R^{15}$ and $R^{16}$ are independently of one another hydrogen, $-(C_1-C_4)$-alkyl, or together form a ring out of the droup cyclopropyl, cyclobutyl, cyclopentyl or cyclohexyl, wherein each ring is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$,

in all its stereoisomeric forms and mixtures thereof in any ratio, and its physiologically tolerable salts.

8) The present invention also relates to the compounds of the formula I, which are

1-[5-(5-Chloro-thiophen-2-yl)-isoxazol-3-ylmethyl]-1H-benzoimidazole-2-carboxylic acid (1-isopropyl-piperidin-4-yl)-amide

1-[(4-Chloro-phenylcarbamoyl)-methyl]-1H-benzoimidazole-2-carboxylic acid (1-isopropyl-piperidin-4-yl)-amide

1-[(5-Chloro-pyridin-2-ylcarbamoyl)-methyl]-1H-benzoimidazole-2-carboxylic acid (1- isopropyl-piperidin-4-yl)-amide

1-[5-(5-Chloro-thiophen-2-yl)-isoxazol-3-ylmethyl]-1H-benzoimidazole-2-carboxylic acid (1-pyrimidin-4-yl-piperidin-4-yl)-amide

1-[5-(5-Chloro-thiophen-2-yl)-isoxazol-3-ylmethyl]-2-(1-isopropyl-piperidin-4- ylcarbamoyl)-1H-benzoimidazole-5-carboxylic acid methyl ester

3-[5-(5-Chloro-thiophen-2-yl)-isoxazol-3-ylmethyl]-2-(1-isopropyl-piperidin-4- ylcarbamoyl)-3H-benzoimidazole-5-carboxylic acid methyl ester

1-[5-(5-Chloro-thiophen-2-yl)-isoxazol-3-ylmethyl]-2-(isopropyl-piperidin-4- ylcarbamoyl)-1H-benzoimidazole-5-carboxylic acid

3-[5-(5-Chloro-thiophen-2-yl)-isoxazol-3-ylmethyl]-2-(1-isopropyl-piperidin-4- ylcarbamoyl)-3H-benzoimidazole-5-carboxylic acid

1-[2-(5-Chloro-thiophen-2-yl)-thiazol-5-ylmethyl]-2-(1-isopropyl-piperidin-4-ylcarbamoyl)-1H-benzoimidazole-5-carboxylic acid methyl ester

3-[2-(5-Chloro-thiophen-2-yl)-thiazol-5-ylmethyl]-2-(1-isopropyl-piperidin-4-ylcarbamoyl)- 3H-benzoimidazole-5-carboxylic acid methyl ester

1-[2-(5-Chloro-thiophen-2-yl)-thiazol-5-ylmethyl]-2-(1-isopropyl-piperidin-4-ylcarbamoyl)-1H-benzoimidazole-5-carboxylic acid

3-[2-(5-Chloro-thiophen-2-yl)-thiazol-5-yl methyl]-2-(1-isopropyl-piperidin-4-ylcarbamoyl)- 3H-benzoimidazole-5-carboxylic acid

1-[5-(5-Chloro-thiophen-2-yl)-isoxazol-3-ylmethyl]-2-(1-isopropyl-piperidin-4- ylcarbamoyl)-1H-benzoimidazole-4-carboxylic acid methyl ester

3-[5-(5-Chloro-thiophen-2-yl)-isoxazol-3-ylmethyl]-2-(1-isopropyl-piperidin-4- ylcarbamoyl)-3H-benzoimidazole-4-carboxylic acid methyl ester

1-[5-(5-Chloro-thiophen-2-yl)-isoxazol-3-ylmethyl]-2-(1-isopropyl-piperidin-4- ylcarbamoyl)-1H-benzoimidazole-4-carboxylic acid

3-[5-(5-Chloro-thiophen-2-yl)-isoxazol-3-ylmethyl]-2-(1-isopropyl-piperidin-4- ylcarbamoyl)-3H-benzoimidazole-4-carboxylic acid

3-[5-(5-Chloro-thiophen-2-yl)-isoxazol-3-ylmethyl]-3H-imidazo[4,5-b]pyridine-2- carboxylic acid (1-isopropyl-piperidin-4-yl)-amide

1-[(5-Chloro-pyridin-2-ylcarbamoyl)-methyl]-2-(1-isopropyl-piperidin-4-ylcarbamoyl)-1H-benzoimidazole-4-carboxylic acid methyl ester

1-[(5-Chloro-pyridin-2-ylcarbamoyl)-methyl]-2-(1-isopropyl-piperidin-4-ylcarbamoyl)-1H-benzoimidazole-4-carboxylic acid

1-[(5-Chloro-pyridin-2-ylcarbamoyl)-methyl]-2-(1-isopropyl-piperidin-4-ylcarbamoyl)-1H-benzoimidazole-5-carboxylic acid methyl ester

3-[(5-Chloro-pyridin-2-ylcarbamoyl)-methyl]-2-(1-isopropyl-piperidin-4-ylcarbamoyl)-3H-benzoimidazole-5-carboxylic acid methyl ester

1-[(5-Chloro-pyridin-2-ylcarbamoyl)-methyl]-2-(1-isopropyl-piperidin-4-ylcarbamoyl)-1H-benzoimidazole-5-carboxylic acid

3-[(5-Chloro-pyridin-2-ylcarbamoyl)-methyl]-2-(1-isopropyl-piperidin-4-ylcarbamoyl)-3H-benzoimidazole-5-carboxylic acid

1-[5-(5-Chloro-thiophen-2-yl)-isoxazol-3-ylmethyl]-2-(1-isopropyl-piperidin-4- ylcarbamoyl)-1H-benzoimidazole-4-carboxylic acid 1-ethoxycarbonyloxy-ethyl ester

1-[5-(5-Chloro-thiophen-2-yl)-isoxazol-3-ylmethyl]-2-(1-isopropyl-piperidin-4- ylcarbamoyl)-1H-benzoimidazole-4-carboxylic acid 1-cyclohexyloxycarbonyloxy-ethyl ester

1-[5-(5-Chloro-thiophen-2-yl)-isoxazol-3-ylmethyl]-1H-benzoimidazole-2,4-dicarboxylic acid 4-[(2-hydroxy-ethyl)-amide] 2-[(1-isopropyl-piperidin-4-yl)-amide]

1-[5-(5-Chloro-thiophen-2-yl)-isoxazol-3-ylmethyl]-4-(3-hydroxy-azetidine-1-carbonyl)-1H-benzoimidazole-2-carboxylic acid (1-isopropyl-piperidin-4-yl)-amide

1-[5-(5-Chloro-thiophen-2-yl)-isoxazol-3-ylmethyl]-1H-benzoimidazole-2,4-dicarboxylic acid 4-[(2-hydroxy-ethyl)-methyl-amide] 2-[(1-isopropyl-piperidin-4-yl)-amide]

1-[5-(5-Chloro-thiophen-2-yl)-isoxazol-3-ylmethyl]-1H-benzoimidazole-2,5-dicarboxylic acid 5-[(2-hydroxy-ethyl)-methyl-amide] 2-[(1-isopropyl-piperidin-4-yl)-amide]

1-[5-(5-Chloro-thiophen-2-yl)-isoxazol-3-ylmethyl]-5-(3-hydroxy-azetidine-1-carbonyl)-1H-benzoimidazole-2-carboxylic acid (1-isopropyl-piperidin-4-yl)-amide

1-[5-(5-Chloro-thiophen-2-yl)-isoxazol-3-ylmethyl]-2-(1-isopropyl-piperidin-4-ylcarbamoyl)-1H-benzoimidazole-5-carboxylic acid 1-ethoxycarbonyloxy-ethyl ester

1-[5-(5-Chloro-thiophen-2-yl)-isoxazol-3-ylmethyl]-2-(1-isopropyl-piperidin-4-ylcarbamoyl)-1H-benzoimidazole-5-carboxylic acid 1-cyclohexyloxycarbonyloxy-ethyl ester

1-[5-(5-Chloro-thiophen-2-yl)-isoxazol-3-ylmethyl]-5-(2-hydroxy-ethanesulfonyl)-1H-benzoimidazole-2-carboxylic acid (1-isopropyl-piperidin-4-yl)-amide

1-[5-(5-Chloro-thiophen-2-yl)-isoxazol-3-ylmethyl]-6-(2-hydroxy-ethanesulfonyl)-1H-benzoimidazole-2-carboxylic acid (1-isopropyl-piperidin-4-yl)-amide

1-[(5-Chloro-pyridin-2-ylcarbamoyl)-methyl]-1H-benzoimidazole-2,4-dicarboxylic acid 4- [(2-hydroxy-ethyl)-amide] 2-[(1-isopropyl-piperidin-4-yl)-amide]

1-[(5-Chloro-pyridin-2-ylcarbamoyl)-methyl]-1H-benzoimidazole-2,4-dicarboxylic acid 4- [(2-hydroxy-ethyl)-methyl-amide] 2-[(1-isopropyl-piperidin-4-yl)-amide]

1-[(5-Chloro-pyridin-2-ylcarbamoyl)-methyl]-4-(3-hydroxy-azetidine-1-carbonyl)-1H-benzoimidazole-2-carboxylic acid (1-isopropyl-piperidin-4-yl)-amide

1-[(5-Chloro-pyridin-2-ylcarbamoyl)-methyl]-2-(1-isopropyl-piperidin-4-ylcarbamoyl)-1H-benzoimidazole-4-carboxylic

acid 1-ethoxycarbonyloxy-ethyl ester

1-[(5-Chloro-pyridin-2-ylcarbamoyl)-methyl]-2-(1-isopropyl-piperidin-4-ylcarbamoyl)-1H-benzoimidazole-4-carboxylic acid 1-cyclohexyloxycarbonyloxy-ethyl ester

1-[(5-Chloro-pyridin-2-ylcarbamoyl)-methyl]-2-[4-(3-oxo-morpholin-4-yl)-phenylcarbamoyl]-1H-benzoimidazole-4-carboxylic acid methyl ester or

1-[(5-Chloro-pyridin-2-ylcarbamoyl)-methyl]-1H-benzoimidazole-2-carboxylic acid [4-(3- oxo-morpholin-4-yl)-phenyl]-amide.

In general, the meaning of any group, residue, heteroatom, number etc., which can occur more than once in the compounds of the formula I, is independent of the meaning of this group, residue, heteroatom, number etc. in any other occurrence. All groups, residues, heteroatoms, numbers etc., which can occur more than once in the compounds of the formula I can be identical or different.

As used herein, the term alkyl is to be understood in the broadest sense to mean hydrocarbon residues which can be linear, i. e. straight-chain, or branched and which can be acyclic or cyclic residues or comprise any combination of acyclic and cyclic subunits. Further, the term alkyl as used herein expressly includes saturated groups as well as unsaturated groups which latter groups contain one or more, for example one, two or three, double bonds and/or triple bonds, provided that the double bonds are not located within a cyclic alkyl group in such a manner that an aromatic system results. All these statements also apply if an alkyl group occurs as a substituent on another residue, for example in an alkyloxy residue, an alkyloxycarbonyl residue or an arylalkyl residue. Examples of "-$(C_1$-$C_8)$-alkyl" or "-$(C_1$-$C_8)$-alkylene" are alkyl residues containing 1, 2, 3, 4, 5, 6, 7 or 8 carbon atoms are methyl, methylene, ethyl, ethylene. propyl, propylene, butyl, butylene, pentyl, pentylene, hexyl, heptyl or octyl, the n-isomers of all these residues, isopropyl, isobutyl, 1-methylbutyl, isopentyl, neopentyl, 2,2-dimethylbutyl, 2-methylpentyl, 3-methylpentyl, isohexyl, sec-butyl, tBu, tert-pentyl, sec-butyl, tert-butyl or tert-pentyl. The term "-$(C_0$-$C_6)$-alkyl" or "-$(C_0$-$C_8)$-alkylene" is a hydrocarbon residues containing 1, 2, 3, 4, 5, 6, 7 or 8 carbon atoms. The term "-$C_0$-alkyl" or "-$C_0$-alkylene" is a covalent bond.

Unsaturated alkyl residues are, for example, alkenyl residues such as vinyl, 1-propenyl, 2-propenyl (= allyl), 2-butenyl, 3-butenyl, 2-methyl-2-butenyl, 3-methyl-2-butenyl, 5-hexenyl or 1,3-pentadienyl, or alkynyl residues such as ethynyl, 1-propynyl, 2-propynyl (= propargyl) or 2-butynyl. Alkyl residues can also be unsaturated when they are substituted.

Examples of -$(C_3$-$C_8)$-cycloalkyl cyclic alkyl residues are cycloalkyl residues containing 3, 4, 5, 6, 7 or 8 ring carbon atoms like cyclopropyl, cyclobutyl, cyclopentyl, cyclohexyl, cyloheptyl or cyclooctyl, which can also be substituted and/or unsaturated. Unsaturated cyclic alkyl groups and unsaturated cycloalkyl groups like, for example, cyclopentenyl or cyclohexenyl can be bonded via any carbon atom.

Of course, a cyclic alkyl group has to contain at least three carbon atoms, and an unsaturated alkyl group has to contain at least two carbon atoms. Thus, a group like $(C_1$-$C_8)$-alkyl is to be understood as comprising, among others, saturated acyclic $(C_1$-$C_8)$-alkyl, $(C_3$-$C_6)$-cycloalkyl, and unsaturated $(C_2$-$C_8)$-alkyl like $(C_2$-$C_8)$-alkenyl or $(C_2$-$C_8)$-alkynyl. Similarly, a group like $(C_1$-$C_4)$-alkyl is to be understood as comprising, among others, saturated acyclic $(C_1$-$C_4)$-alkyl, and unsaturated $(C_2$-$C_4)$-alkyl like $(C_2$-$C_4)$-alkenyl or $(C_2$-$C_4)$-alkynyl.

Unless stated otherwise, the term alkyl preferably comprises acyclic saturated hydro-carbon residues which have from one to six carbon atoms and which can be linear or branched. A particular group of saturated acyclic alkyl residues is formed by $(C_1$-$C_4)$-alkyl residues like methyl, ethyl, n-propyl, isopropyl, n-butyl, isobutyl, sec-butyl and tBu.

Unless stated otherwise, and irrespective of any specific substituents bonded to alkyl groups which are indicated in the definition of the compounds of the formula I, alkyl groups can in general be unsubstituted or substituted by one or more, for example one, two or three, identical or different substituents. Any kind of substituents present in substituted alkyl residues can be present in any desired position provided that the substitution does not lead to an unstable molecule. Examples of substituted alkyl residues are alkyl residues in which one or more, for example 1, 2 or 3, hydrogen atoms are replaced with halogen atoms, in particular fluorine atoms.

The terms "a monocyclic or bicyclic 6- to 14-membered aryl" or "-$(C_6$-$C_{14})$-aryl" are understood as meaning aromatic hydrocarbon radicals containing from 6 to 14 carbon atoms in the ring. Examples of -$(C_6$-$C_{14})$-aryl radicals are phenyl, naphthyl, for example 1-naphthyl and 2-naphthyl, biphenylyl, for example 2-biphenylyl, 3-biphenylyl and 4-biphenylyl, anthryl or fluorenyl. Biphenylyl radicals, naphthyl radicals and, in particular, phenyl radicals are preferred aryl radicals.

The terms "mono- or bicyclic 4- to 15-membered heterocyclyl" or "-$(C_4$-$C_{15})$-heterocyclyl" refer to heterocycles in which one or more of the 4 to 15 ring carbon atoms are replaced by heteroatoms such as nitrogen, oxygen or sulfur. Examples are acridinyl, azaindole ( 1H-pyrrolopyridinyl), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl

(benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahyd ropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1 ,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,3-triazolyl, 1 ,2,4-triazolyl, 1,2,5-triazolyl, 1,3,4-triazolyl and xanthenyl.

Preferred are heterocyclyls, such as benzimidazolyl, 1,3-benzodioxolyl, benzofuranyl, benzothiazolyl, benzothiophenyl, benzoxazolyl, chromanyl, cinnolinyl, 2-furyl, 3-furyl; imidazolyl, indolyl, indazolyl, isochromanyl, isoindolyl, isoquinolinyl, isothiazolyl, isoxazolyl, oxazolyl, phthalazinyl, pteridinyl, purinyl, pyrazinyl, pyrazolyl, pyridazinyl, pyridoimidazolyl, pyridopyridinyl, pyridopyrimidinyl, 2-pyridyl, 3-pyridyl, 4-pyridyl, pyrimidinyl, pyrrolyl; 2-pyrrolyl, 3-pyrrolyl, quinolinyl, quinazolinyl, quinoxalinyl, tetrazolyl, thiazolyl, 2-thienyl and 3-thienyl.

Also preferred are:

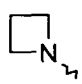 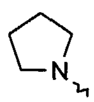 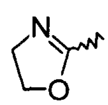 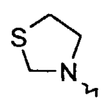 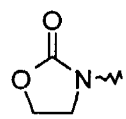 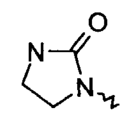

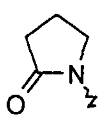 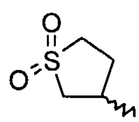 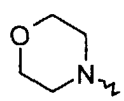 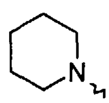 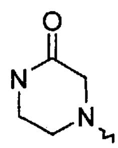 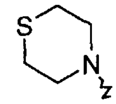 ,

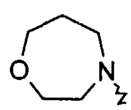 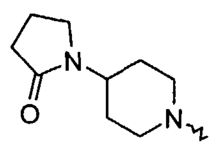 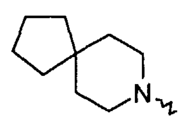

The terms "het" or "a 3- to 7-membered cyclic residue, containing up to 1, 2, 3 or 4 heteroatoms" refer to structures of heterocycles which can be derived from compounds such as azepine, azetidine, aziridine, azirine, 1,4 diazepane, 1,2-diazepine, 1,3-diazepine, 1,4-diazepine, diaziridine, diazirine, dioxazole, dioxazine, dioxole, 1 ,3-dioxolene, 1 ,3-dioxolane, furan, imidazole, imidazoline, imidazolidine, isothiazole, isothiazolidine, isothiazoline, isoxazole, isoxazoline, isoxazolidine, 2-isoxazoline, ketomorpholine, ketopiperazine, morpholine, 1,2-oxa-thiepane, 1,2-oxathiolane, 1,4-oxazepane, 1,2-oxazine, 1,3-oxazine, 1,4-oxazine, oxazole, oxaziridine, oxetan, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1 ,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole.

The term " $R^1$-N-$R^2$-V can form a 4- to 8-membered cyclic group " or "$R^{11}$ and $R^{12}$ together with the nitrogen atom to which they are bonded can form a 4- to 8-membered monocyclic heterocyclic ring which in addition to the nitrogen atom can contain one or two identical or different ring heteroatoms chosen from oxygen, sulfur and nitrogen" refer to structures of heterocycles which can be derived from compounds such as azepane, azepine, azetidine, dioxazole, dioxazine, 1,4-diazepane, 1,2-diazepine, 1,3-diazepine, 1,4-diazepine, imidazole, imidazoline, imidazolidine,

isothiazole, isothiazolidine, isothiazoline, isoxazole, isoxazoline, isoxazolidine, 2-isoxazoline, ketopiperazine, morpholine, [1,4]oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole.

The term "$R^{15}$ and $R^{16}$ together with the carbon atom to which they are bonded can form a 3-to 6 membered carbocyclic ring" refer to structures, which can be derived from compounds such as cyclopropyl, cyclobutyl, cyclopentyl or cyclohexyl.

The term "substructure

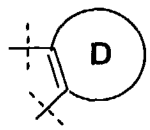

in formulae I" or the "substructure D" is
a 4-to 8 membered saturated, partially unsaturated or aromatic cyclic group containing zero, 1, 2, 3 or 4 heteroatoms chosen from nitrogen, sulfur or oxygen" refer to structures, which can be derived from compounds such as azepane, azetidine, azetine, azocane, azocane-2-one, cyclobutyl, cyclooctane, cyclooctene, cyclopentyl, cyclohexyl, cycloheptyl, cyclooctyl, 1,2-diazepane, 1,2-diazepine, 1,3-diazepine, 1,4-diazepine, [1,4]diazocane, [1,2]diazocan-3-one, [1,3]diazocan-2-one, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,4-oxaazepane, 1,2-oxa-thiepane, 1,2-oxathiolan, 1,2-oxazine, 1,3-oxazine, 1,4-oxazine, oxazole, [1,4]oxazocane, [1,3]oxazocan-2-one, oxetan, oxocane, oxocan-2-one, piperazine, piperidine, phenyl, pyran, pyrazine, pyrazole, pyrazoline, pyrazolidine, pyridazine, pyridine, pyrimidine, pyrrole, pyrrolidine, pyrrolidinone, pyrroline, 5,6,7,8-tetrahydro-1H-azocin-2-one, tetrahydrofuran, tetrahydropyran, tetrahydropyridine, tetrazine, thiadiazine thiadiazole, 1,2-thiazine, 1,3-thiazine, 1,4-thiazine, 1,3-thiazole, thiazole, thiazolidine, thiazoline, thietan, thiocane, thiocane-1,1-dioxide, thiocane-1-oxide, thiocan-2-one, thiomorpholine, thiopyran, 1,2,3-triazine, 1,2,4-triazine, 1,3,5-triazine, 1,2,3-triazole or 1,2,4-triazole.
The term "substructure D" is a 5 to 6 membered saturated, partially unsaturated or aromatic cyclic group containing zero, 1, 2, 3 or 4 heteroatoms chosen from nitrogen, sulfur or oxygen" refer to structures, which can be derived from compounds such as cyclopentyl, cyclohexyl, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketomorpholine, ketopiperazine, morpholine, 1,2-oxathiolan, 1,2-oxazine, 1,3-oxazine, 1,4-oxazine, oxazole, piperazine, piperidine, phenyl, pyran, pyrazine, pyrazole, pyrazoline, pyrazolidine, pyrazine, pyrazinone, pyridazine, pyridazone, pyridine, pyridone, pyrimidine, pyrimidone, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thiomorpholine, thiopyran, tetrazine, tetrazole, 1,2,3-triazine, 1,2,4-triazine, 1,3,5-triazine, 1,2,3-triazole or 1,2,4-triazole.

The term "$R^1$ and $R^3$ together with the atoms to which they are bonded can form a 6- to 8-membered cyclic group, containing up to 1, 2, 3 or 4 heteroatoms chosen from nitrogen, sulfur or oxygen" refers to structures of heterocycles which can be derived from compounds such as azocane, azocane-2-one, cyloheptyl cyclohexyl, cyclooctane, cyclooctene, 1,4-diazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,4-oxazepane, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxa-thiepane, 1,2-oxazine, 1,3-oxazine, 1,4-oxazine, [1,4]oxazocane, [1,3]oxazocan-2-one, oxocane, oxocan-2-one, phenyl, piperazine, piperidine, pyran, pyrazine, pyridazine, pyrimidine, 5,6,7,8-tetrahydro-1H-azocin-2-one or thiomorpholine.

The fact that many of the before-listed names of heterocycles are the chemical names of unsaturated or aromatic ring systems does not imply that the, the 4-15 membered mono- or polycyclic group could only be derived from the respective unsaturated ring system. The names here only serve to describe the ring system with respect to ring size and the number of the heteroatoms and their relative positions. As explained above, the 4-15 membered mono- or polycyclic group can be saturated or partially unsaturated or aromatic, and can thus be derived not only from the before-listed heterocycles themselves but also from all their partially or completely hydrogenated analogues and also from their more highly unsaturated analogues if applicable. As examples of completely or partially hydrogenated analogues of the before-listed heterocycles from which this group may be derived the following may be mentioned: pyrroline, pyrrolidine, tetrahydrofuran, tetrahydrothiophene, dihydropyridine, tetrahydropyridine, piperidine, 1,3-dioxolane, 2-imidazoline, imidazolidine, 4,5-dihydro-1,3-oxazol, 1,3-oxazolidine, 4,5-dihydro-1,3-thiazole, 1 ,3-thiazolidine, perhydro-1,4-dioxane, piperazine, perhydro-1,4-oxazine (= morpholine), perhydro-1,4-thiazine (= thiomorpholine), perhydroazepine, indoline, isoindoline, 1 ,2,3,4-tetrahydroquinoline, 1 ,2,3,4-tetrahydroisoquinoline, etc.

The 4-15 membered mono- or polycyclic group may be bonded via any ring carbon atom, and in the case of nitrogen heterocycles via any suitable ring nitrogen atom. Thus, for example, a pyrrolyl residue can be 1-pyrrolyl, 2-pyrrolyl or 3-pyrrolyl, a pyrrolidinyl residue can be pyrrolidin-1-yl (= pyrrolidino), pyrrolidin-2-yl or pyrrolidin-3-yl, a pyridinyl residue can be pyridin-2-yl, pyridin-3-yl or pyridin-4-yl, a piperidinyl residue can be piperidin-1-yl (= piperidino), piperidin-2-yl, piperidin-3-yl or piperidin-4-yl. Furyl can be 2-furyl or 3-furyl, thienyl can be 2-thienyl or 3-thienyl, imidazolyl can be imidazol-1-yl, imidazol-2-yl, imidazol-4-yl or imidazol-5-yl, 1,3-oxazolyl can be 1,3-oxazol-2-yl, 1,3-oxazol-4-yl or 1,3-oxazol-5-yl, 1,3-thiazolyl can be 1,3-thiazol-2-yl, 1,3-thiazol-4-yl or 1,3-thiazol-5-yl, pyrimidinyl can be pyrimidin-2-yl, pyrimidin-4-yl (= 6-pyrimidinyl) or 5-pyrimidinyl, piperazinyl can be piperazin-1-yl (= piperazin-4-yl = piperazino) or piperazin-2-yl. Indolyl can be indol-1-yl, indol-2-yl, indol-3-yl, indol-4-yl, indol-5-yl, indol-6-yl or indol-7-yl. Similarly benzimidazolyl, benzoxazolyl and benzothiazol residues can be bonded via the 2-position and via any of the positions 4, 5, 6, and 7. Quinolinyl can be quinolin-2-yl, quinolin-3-yl, quinolin-4-yl, quinolin-5-yl, quinolin-6-yl, quinolin-7-yl or quinolin-8-yl, isoqinolinyl can be isoquinol-1-yl, isoquinolin-3-yl, isoquinolin-4-yl, isoquinolin-5-yl, isoquinolin-6-yl, isoquinolin-7-yl or isoquinolin-8-yl. In addition to being bonded via any of the positions indicated for quinolinyl and isoquinolinyl, 1,2,3,4-tetrahydroquinolinyl and 1,2,3,4-tetrahydroisoquinolinyl can also be bonded via the nitrogen atoms in 1-position and 2-position, respectively.

Unless stated otherwise, and irrespective of any specific substituents bonded to the 4-15 membered mono- or polycyclic group or any other heterocyclic groups which are indicated in the definition of the compounds of the formula I, the 4-15 membered mono- or polycyclic group can be unsubstituted or substituted on ring carbon atoms with one or more, for example one, two, three, four or five, identical or different substituents like ($C_1$-$C_8$)-alkyl, in particular ($C_1$-$C_4$)-alkyl, ($C_1$-$C_8$)-alkyloxy, in particular ($C_1$-$C_4$)-alkyloxy, ($C_1$-$C_4$)-alkylthio, halogen, nitro, amino, (($C_1$-$C_4$)-alkyl) carbonylamino like acetylamino, trifluoromethyl, trifluoromethoxy, hydroxy, oxo, hydroxy-($C_1$-$C_4$)-alkyl such as, for example, hydroxymethyl or 1-hydroxyethyl or 2-hydroxyethyl, methylenedioxy, ethylenedioxy, formyl, acetyl, cyano, aminosulfonyl, methylsulfonyl, hydroxycarbonyl, aminocarbonyl, ($C_1$-$C_4$)-alkyloxycarbonyl, optionally substituted phenyl, optionally substituted phenoxy, benzyl optionally substituted in the phenyl group, benzyloxy optionally substituted in the phenyl group, etc. The substituents can be present in any desired position provided that a stable molecule results. Of course an oxo group cannot be present in an aromatic ring. Each suitable ring nitrogen atom in the 4-15 membered mono- or polycyclic group can independently of each other be unsubstituted, i. e. carry a hydrogen atom, or can be substituted, i. e. carry a substituent like ($C_1$-$C_8$)-alkyl, for example ($C_1$-$C_4$)-alkyl such as methyl or ethyl, optionally substituted phenyl, phenyl-($C_1$-$C_4$)-alkyl, for example benzyl, optionally substituted in the phenyl group, hydroxy-($C_2$-$C_4$)-alkyl such as, for example 2-hydroxyethyl, acetyl or another acyl group, methylsulfonyl or another sulfonyl group, aminocarbonyl, ($C_1$-$C_4$)-alkyloxycarbonyl, etc. In general, in the compounds of the formula I nitrogen heterocycles can also be present as N-oxides or as quaternary salts. Ring sulfur atoms can be oxidized to the sulfoxide or to the sulfone. Thus, for example a tetrahydrothienyl residue may be present as S,S-dioxotetrahydro-thienyl residue or a thiomorpholinyl residue like thiomorpholin-4-yl may be present as 1-oxo-thiomorpholin-4-yl or 1,1-dioxo-thiomorpholin-4-yl. A substituted 4 to 15 membered mono- or polycyclic group that can be present in a specific position of the compounds of formula I can independently of other groups be substituted by substituents selected from any desired subgroup of the substituents listed before and/or in the definition of that group.

The 3-7 membered monocyclic group may be bonded via any ring carbon atom, and in the case of nitrogen heterocycles via any suitable ring nitrogen atom. Thus, for example, a pyrrolyl residue can be 1-pyrrolyl, 2-pyrrolyl or 3-pyrrolyl, a pyrrolidinyl residue can be pyrrolidin-1-yl (= pyrrolidino), pyrrolidin-2-yl or pyrrolidin-3-yl, a pyridinyl residue can be pyridin-2-yl, pyridin-3-yl or pyridin-4-yl, a piperidinyl residue can be piperidin-1-yl (= piperidino), piperidin-2-yl, piperidin-3-yl or piperidin-4-yl. Furyl can be 2-furyl or 3-furyl, thienyl can be 2-thienyl or 3-thienyl, imidazolyl can be imidazol-1-yl, imidazol-2-yl, imidazol-4-yl or imidazol-5-yl, 1,3-oxazolyl can be 1,3-oxazol-2-yl, 1,3-oxazol-4-yl or 1,3-oxazol-5-yl, 1,3-thiazolyl can be 1,3-thiazol-2-yl, 1,3-thiazol-4-yl or 1,3-thiazol-5-yl, pyrimidinyl can be pyrimidin-2-yl, pyrimidin-4-yl (= 6-pyrimidinyl) or 5-pyrimidinyl, piperazinyl can be piperazin-1-yl (= piperazin-4-yl = piperazino) or piperazin-2-yl. Unless stated otherwise, and irrespective of any specific substituents bonded to the 3-7 membered monocyclic group or any other heterocyclic groups which are indicated in the definition of the compounds of the formula I, can be unsubstituted or substituted on ring carbon atoms with one or more, for example one, two, three, four or five, identical or different substituents like ($C_1$-$C_8$)-alkyl, in particular ($C_1$-$C_4$)-alkyl, ($C_1$-$C_8$)-alkyloxy, in particular ($C_1$-$C_4$)-alkyloxy, ($C_1$-$C_4$)-alkylthio, halogen, nitro, amino, (($C_1$-$C_4$)-alkyl)carbonylamino like acetylamino, trifluoromethyl, trifluoromethoxy, hydroxy, oxo, hydroxy-($C_1$-$C_4$)-alkyl such as, for example, hydroxymethyl or 1-hydroxyethyl or 2-hydroxyethyl, methylenedioxy, ethylenedioxy, formyl, acetyl, cyano, aminosulfonyl, methylsulfonyl, hydroxycarbonyl, aminocarbonyl, ($C_1$-$C_4$)-alkyloxycarbonyl, optionally substituted phenyl, optionally substituted phenoxy, benzyl optionally substituted in the phenyl group, benzyloxy optionally substituted in the phenyl group, etc. The substituents can be present in any desired position provided that a stable molecule results. Of course an oxo group cannot be present in an aromatic

ring. Each suitable ring nitrogen atom in the 3-7 membered monocyclic group can independently of each other be unsubstituted, i. e. carry a hydrogen atom, or can be substituted, i. e. carry a substituent like ($C_1$-$C_8$)-alkyl, for example ($C_1$-$C_4$)-alkyl such as methyl or ethyl, optionally substituted phenyl, phenyl-($C_1$-$C_4$)-alkyl, for example benzyl, optionally substituted in the phenyl group, hydroxy-($C_2$-$C_4$)-alkyl such as, for example 2-hydroxyethyl, acetyl or another acyl group, methylsulfonyl or another sulfonyl group, aminocarbonyl, ($C_1$-$C_4$)-alkyloxycarbonyl, etc. In general, in the compounds of the formulae I nitrogen heterocycles can also be present as N-oxides or as quaternary salts. Ring sulfur atoms can be oxidized to the sulfoxide or to the sulfone. Thus, for example a tetrahydrothienyl residue may be present as S,S-dioxotetrahydrothienyl residue or a thiomorpholinyl residue like thiomorpholin-4-yl may be present as 1-oxo-thiomorpholin-4-yl or 1,1-dioxo-thiomorpholin-4-yl. A substituted 3-7 membered monocyclic group that can be present in a specific position of the compounds of formulae I can independently of other groups be substituted by substituents selected from any desired subgroup of the substituents listed before and/or in the definition of that group.

The term "-($C_1$-$C_3$)-perfluoroalkyl" is a partial or totally fluorinated alkyl-residue, which can be derived from residues such as $-CF_3$, $-CHF_2$, $-CH_2F$, $-CHF-CF_3$, $-CHF-CHF_2$, $-CHF-CH_2F$, $-CH_2-CF_3$, $-CH_2-CHF_2$, $-CH_2-CH_2F$, $-CF_2-CF_3$, $-CF_2-CHF_2$, $-CF_2-CH_2F$, $-CH_2-CHF-CF_3$, $-CH_2-CHF-CHF_2$, $-CH_2-CHF-CH_2F$, $-CH_2-CH_2-CF_3$, $-CH_2-CH_2-CHF_2$, $-CH_2-CH_2-CH_2F$, $-CH_2-CF_2-CF_3$, $-CH_2-CF_2-CHF_2$, $-CH_2-CF_2-CH_2F$, $-CHF-CHF-CF_3$, $-CHF-CHF-CHF_2$, $-CHF-CHF-CH_2F$, $-CHF-CH_2-CF_3$, $-CHF-CH_2-CHF_2$, $-CHF-CH_2-CH_2F$, $-CHF-CF_2-CF_3$, $-CHF-CF_2-CHF_2$, $-CHF-CF_2-CH_2F$, $-CF_2-CHF-CF_3$, - $CF_2-CHF-CHF_2$, $-CF_2-CHF-CH_2F$, $-CF_2-CH_2-CF_3$, $-CF_2-CH_2-CHF_2$, $-CF_2-CH_2-CH_2F$, $-CF_2-CF_2-CF_3$, $-CF_2-CF_2-CHF_2$ or $-CF_2-CF_2-CH_2F$.

Halogen is fluorine, chlorine, bromine or iodine, preferably fluorine, chlorine or bromine, particularly preferably chlorine or bromine.

Optically active carbon atoms present in the compounds of the formula I can independently of each other have R configuration or S configuration. The compounds of the formula I can be present in the form of pure enantiomers or pure diastereomers or in the form of mixtures of enantiomers and/or diastereomers, for example in the form of racemates. The present invention relates to pure enantiomers and mixtures of enantiomers as well as to pure diastereomers and mixtures of diastereomers. The invention comprises mixtures of two or of more than two stereoisomers of the formula I, and it comprises all ratios of the stereoisomers in the mixtures. In case the compounds of the formula I can be present as E isomers or Z isomers (or cis isomers or trans isomers) the invention relates both to pure E isomers and pure Z isomers and to E/Z mixtures in all ratios. The invention also comprises all tautomeric forms of the compounds of the formula I.

Diastereomers, including E/Z isomers, can be separated into the individual isomers, for example, by chromatography. Racemates can be separated into the two enantiomers by customary methods, for example by chromatography on chiral phases or by resolution, for example by crystallization of diastereomeric salts obtained with optically active acids or bases. Stereochemically uniform compounds of the formula I can also be obtained by employing stereochemically uniform starting materials or by using stereoselective reactions.

Physiologically tolerable salts of the compounds of formula I are nontoxic salts that are physiologically acceptable, in particular pharmaceutically utilizable salts. Such salts of compounds of the formula I containing acidic groups, for example a carboxyl group COOH, are for example alkali metal salts or alkaline earth metal salts such as sodium salts, potassium salts, magnesium salts and calcium salts, and also salts with physiologically tolerable quaternary ammonium ions such as tetramethylammonium or tetraethylammonium, and acid addition salts with ammonia and physiologically tolerable organic amines, such as methylamine, dimethylamine, trimethylamine, ethylamine, triethylamine, ethanolamine or tris-(2-hydroxyethyl)amine. Basic groups contained in the compounds of the formula I, for example amino groups or guanidino groups, form acid addition salts, for example with inorganic acids such as hydrochloric acid, hydrobromic acid, sulfuric acid, nitric acid or phosphoric acid, or with organic carboxylic acids and sulfonic acids such as formic acid, acetic acid, oxalic acid, citric acid, lactic acid, malic acid, succinic acid, malonic acid, benzoic acid, maleic acid, fumaric acid, tartaric acid, methanesulfonic acid or p-toluenesulfonic acid. Compounds of the formula I, which simultaneously contain a basic group and an acidic group, for example a guanidino group and a carboxyl group, can also be present as zwitterions (betaines) which are likewise included in the present invention.

Salts of compounds of the formula I can be obtained by customary methods known to those skilled in the art, for example by combining a compound of the formula I with an inorganic or organic acid or base in a solvent or dispersant, or from other salts by cation exchange or anion exchange. The present invention also includes all salts of the compounds of the formula I which, because of low physiologically tolerability, are not directly suitable for use in pharmaceuticals but are suitable, for example, as intermediates for carrying out further chemical modifications of the compounds of the formula I or as starting materials for the preparation of physiologically tolerable salts.

The present invention furthermore includes all solvates of compounds of the formula I, for example hydrates or adducts with alcohols.

The invention also includes derivatives and modifications of the compounds of the formula I, for example prodrugs, protected forms and other physiologically tolerable derivatives, as well as active metabolites of the compounds of the formula I. The invention relates in particular to prodrugs and protected forms of the compounds of the formula I, which

can be converted into compounds of the formula I under physiological conditions. Suitable prodrugs for the compounds of the formula I, i. e. chemically modified derivatives of the compounds of the formula I having properties which are improved in a desired manner, for example with respect to solubility, bioavailability or duration of action, are known to those skilled in the art. More detailed information relating to prodrugs is found in standard literature like, for example, Design of Prodrugs, H. Bundgaard (ed.), Elsevier, 1985; Fleisher et al., Advanced Drug Delivery Reviews 19 (1996) 115-130; or H. Bundgaard, Drugs of the Future 16 (1991) 443 which are all incorporated herein by reference. Suitable prodrugs for the compounds of the formula I are especially acyl prodrugs and carbamate prodrugs of acylatable nitrogen-containing groups such as amino groups and the guanidino group and also ester prodrugs and amide prodrugs of carboxylic acid groups which may be present in compounds of the formula I. In the acyl prodrugs and carbamate prodrugs one or more, for example one or two, hydrogen atoms on nitrogen atoms in such groups are replaced with an acyl group or a carbamate, preferably a -($C_1$-$C_6$)-alkyloxycarbonyl group. Suitable acyl groups and carbamate groups for acyl prodrugs and carbamate prodrugs are, for example, the groups $R^{p1}$-CO- and $R^{p2}$O-CO-, in which $R^{p1}$ is hydrogen, ($C_1$-$C_{18}$)-alkyl, ($C_3$-$C_8$)-cycloalkyl, ($C_3$-$C_8$)-cycloalkyl-($C_1$-$C_4$)-alkyl)-, ($C_6$-$C_{14}$)-aryl, Het-, ($C_6$-$C_{14}$)-aryl-($C_1$-$C_4$)-alkyl- or Het-($C_1$-$C_4$)-alkyl- and in which $R^{p2}$ has the meanings indicated for $R^{p1}$ with the exception of hydrogen.

Especially preferred compounds of the formula I are those wherein two or more residues are defined as indicated before for preferred compounds of the formula I, or residues can have one or some of the specific denotations of the residues given in their general definitions or in the definitions of preferred compounds before. All possible combinations of definitions given for preferred definitions and of specific denotations of residues explicitly are a subject of the present invention.

Also with respect to all preferred compounds of the formula I all their stereoisomeric forms and mixtures thereof in any ratio and their physiologically acceptable salts explicitly are a subject of the present invention, as well as are their prodrugs. Similarly, also in all preferred compounds of the formula I, all residues that are present more than one time in the molecule are independent of each other and can be identical or different.

The compounds of the formula I can be prepared by utilising procedures and techniques, which per se are well known and appreciated by one of ordinary skill in the art. Starting materials or building blocks for use in the general synthetic procedures that can be applied in the preparation of the compounds of formula I are readily available to one of ordinary skill in the art. In many cases they are commercially available or have been described in the literature. Otherwise they can be prepared from readily available precursor compounds analogously to procedures described in the literature, or by procedures or analogously to procedures described in this application.

In general, compounds of the formula I can be prepared, for example in the course of a convergent synthesis, by linking two or more fragments which can be derived retrosynthetically from the formula I . More specifically, suitably substituted starting benzoimidazole derivatives are employed as building blocks in the preparation of the compounds of formula I. If not commercially available, such benzoimidazole derivatives can be prepared according to the well-known standard procedures for the formation of the benzoimidazole ring system. By choosing suitable precursor molecules, these benzoimidazole syntheses allow the introduction of a variety of substituents into the various positions of the benzoimidazole system, which can be chemically modified in order to finally arrive at the molecule of the formula I having the desired substituent pattern. As one of the comprehensive reviews in which numerous details and literature references on the chemistry of benzoimidazole and on synthetic procedures for their preparation can be found, J. Backes, B. Heinz, W. G. Ried in Houben-Weyl, "Methoden der Organischen Chemie" (Methods of Organic Chemistry), Georg Thieme Verlag, Stuttgart, Germany 1994, Vol. E8c Hetarene.

If starting benzoimidazole derivatives are not commercially available and have to be synthesized this can be done, for example, according to the well-known benzoimidazole syntheses mentioned above. In the following procedures of particuluar interest for the embodiment of this invention are listed and referenced briefly, however, they are standard procedures comprehensively discussed in the literature, and are well known to one skilled in the art. Although not always shown explicitly, in certain cases positional isomers will occur during the synthesis of the below mentioned reactions. Nevertheless such mixtures of positional isomers, can be separated by modern separation techniques like, for example, preparative HPLC.

1) J. H. Musser et al., Synth. Commun. 1984, 10, 947.

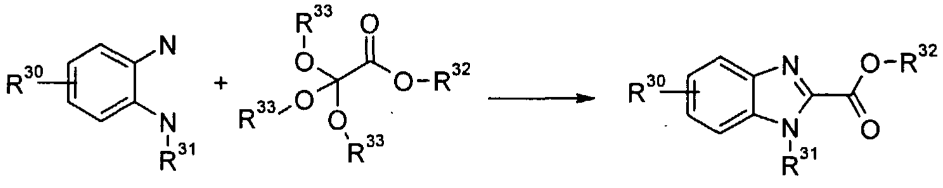

2) Reissert et al., Chem. Ber. 1905, 38, 93.

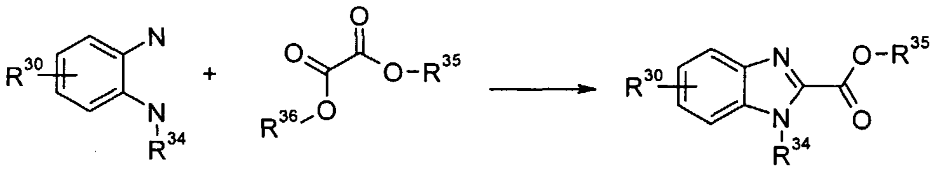

3)

a) Usherwood et al., J. Chem. Soc. 1923, 123, 1082
b) J. R. Young et al., Bioorg. Med. Chem Lett. 2002, 12, 827.
c) H. Yukawa et al., Bioorg. Med. Chem Lett. 1997, 10, 1267.

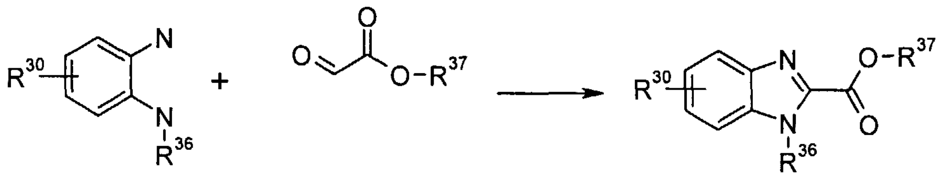

4) Z.-T. Huang et al., Tetrahedron 1992, 48, 2325.

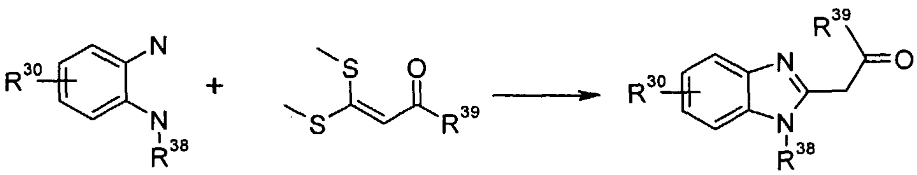

5) A. 0. Abdelhamid et al., J. Heterocycl. Chem. 1988, 25, 403.

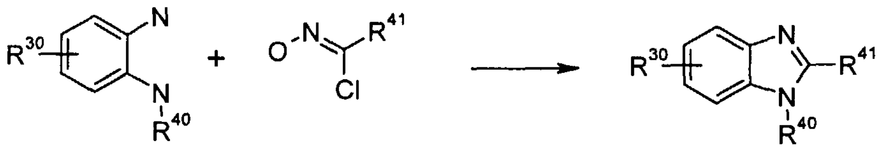

6)

a) G. Holan et al., J. Chem. Soc. 1967, 20.
b) G. Crank et al., Aust. J. Chem. 1982, 35, 775.
c) G. Dannhardt et al., Arch. Pharm. 2000, 333, 123.
d) P. Louvet et al., Eur. J. Med. Chem. 1993, 28, 71.
e) E. L. Samuel et al., J. Chem. Soc. C, 1967, 25.

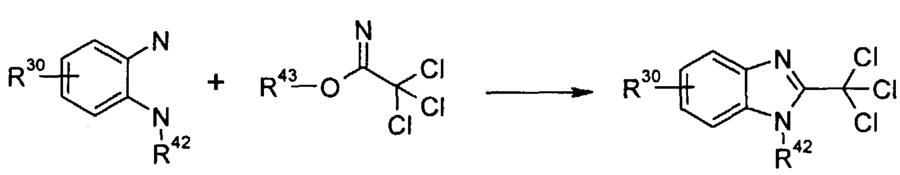

7) P. A. Petyunin et al., Khim. Geterosikl Soedin 19982, 5, 684.

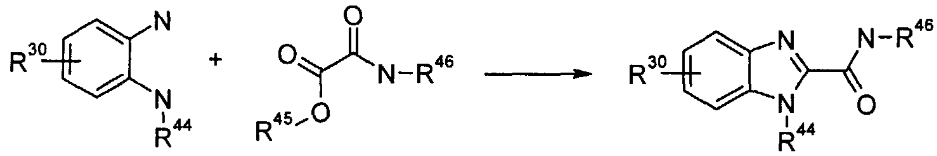

8) C. T. Brain et al.,Tetrahedron Lett., 2002,43,1893.

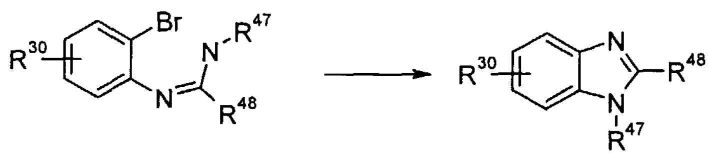

9) E. L. Samuel et al., J. Chem. Soc. C, 1967, 25.

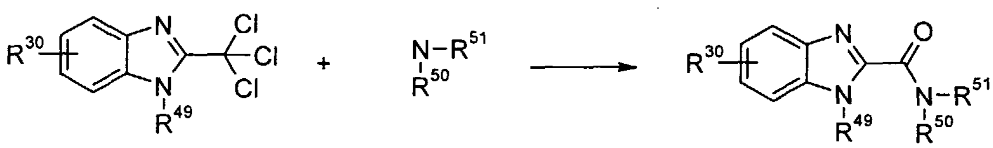

10) E. L. Samuel et al., J. Chem. Soc. C, 1967, 25.

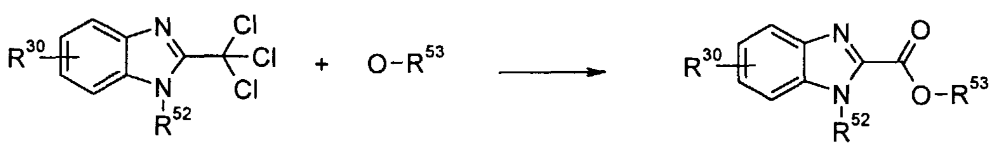

11) G. Dannhardt et al., Arch. Pharm. 2000, 333, 123.

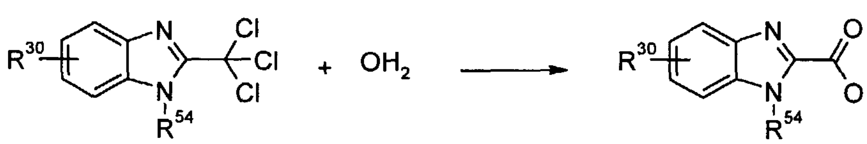

12) A. Orjales et al. , Eur. J. Med. Chem. 1999, 34, 415.

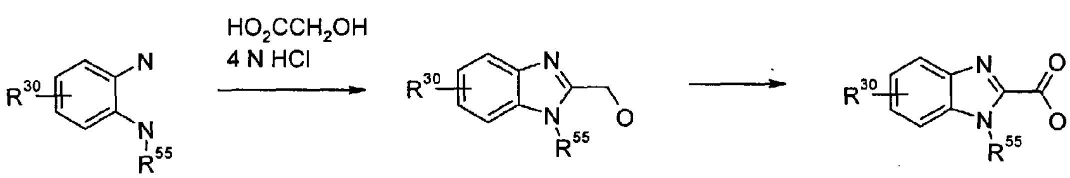

13) H. Göker et al. , Arch. Pharm. Pharm. Med. Chem. 2001, 334, 148.

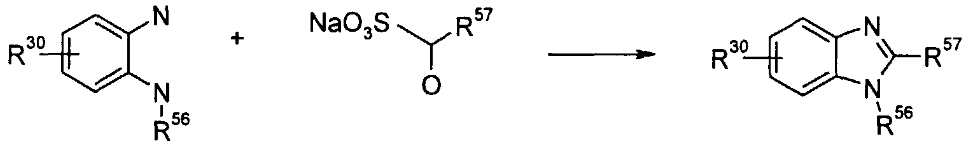

14)

a) R. B. Baudy et al., J. Med. Chem. 2001, 44, 1516.
b) Y. K. Yun et al. , Synlett 2002, No. 5, 739.

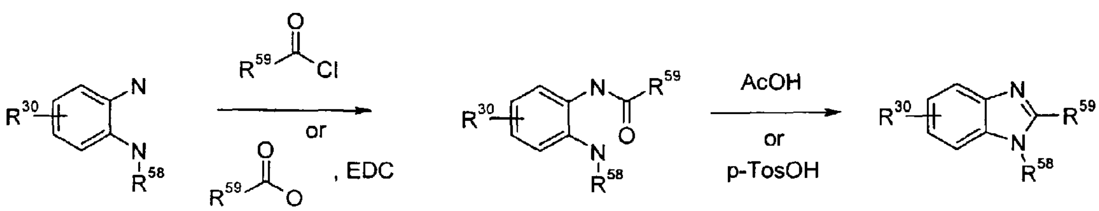


15) N. Nabulsi, R. Gandour, J. Org. Chem. 1991, 56, 2260.

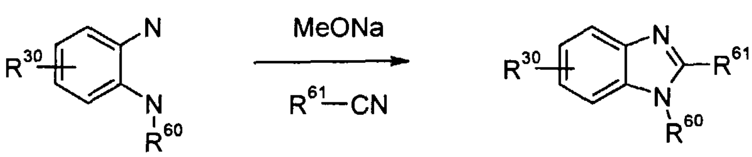


Depending on the substituents in the starting materials, in certain benzoimidazole syntheses mixtures of positional isomers may be obtained, which, however, can be separated by modern separation techniques like, for example, preparative HPLC.

Further, in order to obtain the desired substituents at the benzoimidazole ring system in the formula I, the functional groups introduced into the ring system during the benzoimidazole synthesis can be chemically modified. Especially the groups present in the benzoimidazole ring system can be modified by a variety of reactions and thus the desired residues $R^{30}$ be obtained. For example, a benzoimidazole carrying a hydrogen atom in the 2-position can also be obtained by saponification and subsequent decarboxylation of benzoimidazole carrying an ester group in the respective position. Carboxylic acid groups and acetic acid groups in the 2-position, the 4-position, 5-position, 6-position and the 7-position can be converted into their homologues by usual reactions for chain elongation of carboxylic acids. Halogen atoms can be introduced, for example according to procedures described in the literature like the following. For the fluorination of benzoimidazoles N-fluoro-2,4,6-trimethylpyridinium triflate is the reagent of choice (T. Umemoto, S. Fukami, G. Tomizawa, K. Harasawa, K. Kawada, K. Tomita, J. Am. Chem. Soc. 1990, 112, 8563) but is not limited to this reagent. The chlorination, bromination, or iodination of benzoimidazoles can be accomplished by the reaction of the elemental halogens or by the use of NCS, NBS or NIS and many other reagents well known to those skilled in the art. These procedures are for example referred in Y. Shi et al., Synth. Commun. 1993, 23, 2623; H. Rapoport et al., Synthesis 1988, 767; R. Jones et al., J. Org. Chem. 1999, 64, 6575; J. Sessler et al., Chem. Eur. J. 2001, 7, 721. Depending on the reaction conditions, reagent, stochiometry and substitution pattern the halogen is introduced in the 2-position and/or 4-position and/or 5-position and/or 6-position and/or 7-position. By selective halogen/metal exchange or metalation by selective hydrogen/metal exchange and subsequent reaction with a wide range of electrophiles various substituents can be introduced at the heterocyclic nucleus. (R. Breslow et al., J. Am. Chem. Soc. 1983, 105, 5337; P. Knochel et al., J. Org. Chem. 2000, 65, 4618; S. Ohta et al., Chem. Pharm. Bull. 1996, 44, 1831).

Halogens or hydroxy groups - via the triflate or nonaflate - or primary amines - via its diazonium salt - or after

interconversion to the corresponding stannane, or boronic acid - present in the benzoimidazole structure can be converted into a variety of other functional groups like for example -CN, $-CF_3$, $-C_2F_5$, ethers, acids, amides, amines, alkyl- or aryl- groups mediated by means of transition metals, namely palladium or nickel catalysts or copper salts and reagents for example referred to below (F. Diederich, P. Stang, Metal-catalyzed Cross-coupling Reactions, Wiley-VCH, 1998; or M. Beller, C. Bolm, Transition Metals for Organic Synthesis, Wiley-VCH, 1998; J. Tsuji, Palladium Reagents and Catalysts, Wiley, 1996; J. Hartwig, Angew. Chem. 1998, 110, 2154; B. Yang, S. Buchwald, J. Organomet. Chem. 1999, 576, 125; T. Sakamoto, K. Ohsawa, J. Chem. Soc. Perkin Trans I, 1999, 2323; D. Nichols, S. Frescas, D. Marona-Lewicka, X. Huang, B. Roth, G. Gudelsky, J. Nash, J. Med. Chem, 1994, 37, 4347; P. Lam, C. Clark, S. Saubern, J. Adams, M. Winters, D. Chan, A. Combs, Tetrahedron Lett., 1998, 39, 2941; D. Chan, K. Monaco, R. Wang, M. Winters, Tetrahedron Lett. 1998, 39, 2933; V. Farina, V. Krishnamurthy, W. Scott, The Stille Reaction, Wiley, 1994; F. Qing et al. J. Chem. Soc. Perkin Trans. I 1997, 3053; S. Buchwald et al. J. Am. Chem Soc. 2001, 123, 7727; S. Kang et al. Synlett 2002, 3, 427; S. Buchwald et al. Organic Lett. 2002, 4, 581; T. Fuchikami et al. Tetrahedron Lett. 1991, 32, 91; Q. Chen et al. Tetrahedron Lett. 1991, 32, 7689).
For example, nitro groups can be reduced to amino group with various reducing agents, such as sulfides, dithionites, complex hydrides or by catalytic hydrogenation. A reduction of a nitro group may also be carried out at a later stage of the synthesis of a compound of the formula I, and a reduction of a nitro group to an amino group may also occur simultaneously with a reaction performed on another functional group, for example when reacting a group like a cyano group with hydrogen sulfide or when hydrogenating a group. In order to introduce the residues $R^{30}$, amino groups can then be modified according to standard procedures for alkylation, for example by reaction with (substituted) alkyl halogenides or by reductive amination of carbonyl compounds, according to standard procedures for acylation, for example by reaction with activated carboxylic acid derivatives such as acid chlorides, anhydrides, activated esters or others or by reaction with carboxylic acids in the presence of an activating agent, or according to standard procedures for sulfonylation, for example by reaction with sulfonyl chlorides.

Ester groups present in the benzoimidazole nucleus can be hydrolyzed to the corresponding carboxylic acids, which after activation can then be reacted with amines or alcohols under standard conditions. Furthermore these ester or acid groups can be reduced to the corresponding alcohols by many standard procedures. Ether groups present at the benzoimidazole nucleus, for example benzyloxy groups or other easily cleavable ether groups, can be cleaved to give hydroxy groups which then can be reacted with a variety of agents, for example etherification agents or activating agents allowing replacement of the hydroxy group by other groups. Sulfur-containing groups can be reacted analogously.

During the course of the synthesis in order to modify the groups $R^{62}$ or $R^{8'}$ attached to the benzoimidazole ring system by application of parallel synthesis methodology, beside a variety of reactions, palladium, nickel or copper catalysis can be extremely useful. Such reactions are described for example in F. Diederich, P. Stang, Metal-catalyzed Cross-coupling Reactions, Wiley-VCH, 1998; or M. Beller, C. Bolm, Transition Metals for Organic Synthesis, Wiley-VCH, 1998; J. Tsuji, Palladium Reagents and Catalysts, Wiley, 1996; J. Hartwig, Angew. Chem. 1998, 110, 2154; B. Yang, S. Buchwald, J. Organomet. Chem. 1999, 576, 125; P. Lam, C. Clark, S. Saubern, J.
Adams, M. Winters, D. Chan, A. Combs, Tetrahedron Lett. 1998, 39, 2941; D. Chan, K. Monaco, R. Wang, M. Winters, Tetrahedron Lett. 1998, 39, 2933; J. Wolfe, H. Tomori, J. Sadight, J. Yin, S. Buchwald, J. Org. Chem. 2000, 65, 1158; V. Farina, V. Krishnamurthy, W. Scott, The Stille Reaction, Wiley, 1994; S. Buchwald et al., J. Am. Chem Soc. 2001, 123, 7727; S. Kang et al., Synlett 2002, 3, 427; S. Buchwald et al., Org. Lett. 2002, 4, 581.

The previously-mentioned reactions for the conversion of functional groups are furthermore, in general, extensively described in textbooks of organic chemistry like M. Smith, J. March, March's Advanced Organic Chemistry, Wiley-VCH, 2001 and in treatises like Houben-Weyl, "Methoden der Organischen Chemie" (Methods of Organic Chemistry), Georg Thieme Verlag, Stuttgart, Germany, or "Organic Reactions", John Wiley & Sons, New York, or R. C. Larock, " Comprehensive Organic Transformations", Wiley-VCH, 2$^{nd}$ ed (1999), B. Trost, I. Fleming (eds.) Comprehensive Organic Synthesis, Pergamon,1991; A. Katritzky, C. Rees, E. Scriven Comprehensive Heterocyclic Chemistry II, Elsevier Science, 1996) in which details on the reactions and primary source literature can be found. Due to the fact that in the present case the functional groups are attached to a benzoimidazole ring it may in certain cases become necessary to specifically adapt reaction conditions or to choose specific reagents from a variety of reagents that can in principle be employed into a conversion reaction, or otherwise to take specific measures for achieving a desired conversion, for example to use protection group techniques. However, finding out suitable reaction variants and reaction conditions in such cases does not cause any problems for one skilled in the art.
The structural elements present in the residues in the 1-position of the benzoimidazole ring in the compounds of the formula and in the $COR^{8'}$ group present in the 2-position of the benzoimidazole ring can be introduced into the starting benzoimidazole derivative obtainable as outlined above by consecutive reaction steps using parallel synthesis methodologies like those outlined below using procedures which per se are well known to one skilled in the art.

The residues $R^{8'}$ that can be introduced in formula **2**, for example, by condensing a corresponding carboxylic acid of the formula **2** with a compound of the formula $HR^{8'}$,

i. e. with an amine of the formula $HN(R^{1'})R^{2'}$-V-G-M to give a compound of the formula **3**. The compound of the formula **3** thus obtained can already contain the desired final groups, i. e. the groups $R^{8'}$ and $R^{62}$ can be the groups-$N(R^1)$-$R^2$-V-G-M and $R^0$-Q- as defined in the formula I, or optionally in the compound of the formula **3** thus obtained subsequently the residue or the residues $R^{8'}$ and the residue $R^{62}$ are converted into the residues -$N(R^1)$-$R^2$-V-G-M and $R^0$-Q- , respectively, to give the desired compound of the formula I.

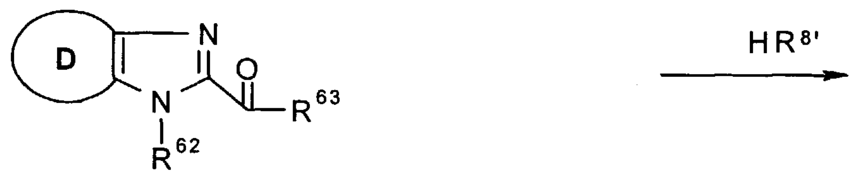


**2**

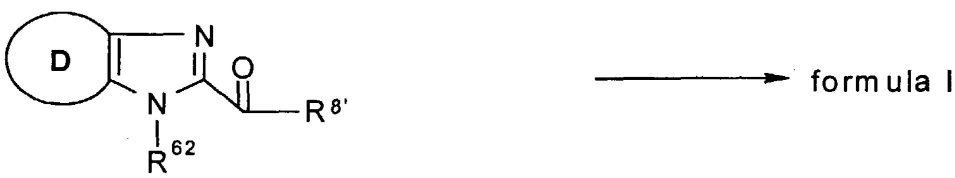


**3**

Thus, the residues $R^{8'}$ and the residues $R^{1'}$ and $R^{2'}$-V-G-M contained therein can have the denotations of $R^1$ and $R^2$-V-G-M, respectively, given above or in addition in the residues $R^{1'}$ and $R^{2'}$-V-G-M functional groups can also be present in the form of groups that can subsequently be transformed into the final groups $R^1$ and $R^2$-V-G-M, i.e. functional groups can be present in the form of precursor groups or of derivatives, for example in protected form. In the course of the preparation of the compounds of the formula I it can generally be advantageous or necessary to introduce functional groups which reduce or prevent undesired reactions or side reactions in the respective synthesis step, in the form of precursor groups which are later converted into the desired functional groups, or to temporarily block functional groups by a protective group strategy suited to the synthesis problem. Such strategies are well known to those skilled in the art (see, for example, Greene and Wuts, Protective Groups in Organic Synthesis, Wiley, 1991, or P. Kocienski, Protecting Groups, Thieme 1994). As examples of precursor groups cyano groups and nitro groups may be mentioned. The cyano group can in a later step be transformed into carboxylic acid derivatives or by reduction into aminomethyl groups, or the nitro groups may be transformed by reduction like catalytic hydrogenation into amino groups. Protective groups can also have the meaning of a solid phase, and cleavage from the solid phase stands for the removal of the protective group. The use of such techniques is known to those skilled in the art (Burgess K (Ed.) Solid Phase Organic Synthesis, New York, Wiley, 2000). For example, a phenolic hydroxy group can be attached to a trityl-polystyrene resin, which serves as a protecting group, and the molecule is cleaved from this resin by treatment with TFA at a later stage of the synthesis.

The residue $R^{62}$ in the compounds of the formulae **2** and **3** can denote the group -Q-$R^0$ as defined above which finally is to be present in the desired target molecule of the formula I, or it can denote a group which can subsequently be transformed into the group -Q-$R^0$, for example a precursor group or a derivative of the group -Q-$R^0$ in which functional groups are present in protected form, or $R^{62}$ can denote a hydrogen atom or a protective group for the nitrogen atom of the benzoimidazole ring. Similarly, the residues $R^{30}$ have the corresponding definitions of $R^3$ in formula I as defined above, however, for the synthesis of the compounds of the formula I these residues, too, can in principle be present at the stage of the condensation of a compound of the formula **2** with a compound of the formula $HR^{8'}$ giving a compound of the formula **3** in the form of precursor groups or in protected form.

The residues $R^{63}$ in the compounds of the formula **2** which can be identical or different, can be, for example, hydroxy or ($C_1$-$C_4$)-alkoxy, i. e., the groups $COR^{63}$ present in the compounds of the formula **2** can be, for example, the free carboxylic acids or esters thereof like alkyl esters as can be the groups $COR^{8'}$ in the compounds of the formula I. The groups $COR^{63}$ can also be any other activated derivative of a carboxylic acid which allows amide formation, ester formation or thioester formation with a compound of the formula $HR^{8'}$. The group $COR^{63}$ can be, for example, an acid

chloride, an activated ester like a substituted phenyl ester, an azolide like an imidazolide, an azide or a mixed anhydride, for example a mixed anhydride with a carbonic acid ester or with a sulfonic acid, which derivatives can all be prepared from the carboxylic acid by standard procedures and can be reacted with an amine, an alcohol or a mercaptan of the formula $HR^{8'}$ under standard conditions. A carboxylic acid group COOH representing $COR^{63}$ in a compound of the formula **2** can be obtained, for example, from an ester group introduced into the benzoimidazole system during a benzoimidazole synthesis by standard hydrolysis procedures.

Compounds of the formula I in which a group $COR^{8'}$ is an ester group can also be prepared from compounds of the formula **2** in which $COR^{63}$ is a carboxylic acid group by common esterification reactions like, for example, reacting the acid with an alcohol under acid catalysis, or alkylation of a salt of the carboxylic acid with an electrophile like an alkyl halogenide, or by transesterification from another ester. Compounds of the formula I in which a group $COR^{8'}$ is an amide group can be prepared from amines and compounds of the formula **2** in which $COR^{63}$ is a carboxylic acid group or an ester thereof by common amination reactions. Especially for the preparation of amides the compounds of the formula **2** in which $COR^{63}$ is a carboxylic acid group can be condensed under standard conditions with compounds of the formula $HR^{8'}$ which are amines by means of common coupling reagents used in peptide synthesis. Such coupling reagents are, for example, carbodiimides like dicyclohexylcarbodiimide (DCC) or diisopropylcarbodiimide, carbonyldiazoles like carbonyldiimidazole (CDI) and similar reagents, propylphosphonic anhydride, O-((cyano-(ethoxycarbonyl)-methylene)amino)-N,N,N',N'-tetramethyluronium tetrafluoroborate (TOTU), diethylphosphoryl cyanide (DEPC) or bis-(2-oxo-3-oxazolidinyl)-phosphoryl chloride (BOP-Cl) and many others.

If the residue $-Q-R^0$ present in a benzoimidazole of the formula I or the residue $R^{63}$ present in a benzoimidazole of the formula **2**, or a residue in which functional groups within the residue $-Q-R^0$ or $R^{63}$ are present in protected form or in the form of a precursor group, have not already been introduced during a preceding step, for example during a synthesis of the benzoimidazole nucleus, these residues can, for example, be introduced into the 1-position of the benzoimidazole system by conventional literature procedures well known to one skilled in the art for N-alkylation, reductive amination, N-arylation, N-acylation or N-sulfonylation of ring nitrogen atoms of heterocycles. The starting benzoimidazole derivative that is to be employed in such a reaction carries a hydrogen atom in the 1-position. N-Alkylation of a ring nitrogen atom can, for example, be performed under standard conditions, preferably in the presence of a base like $K_2CO_3$, $Cs_2CO_3$, NaH or $KO^tBu$, using an alkylating compound of the formula $LG-Q-R^0$ or of the formula $R^{62}-LG$, wherein the atom in the group Q or in the group $R^{62}$ bonded to the group LG in this case is an aliphatic carbon atom of an alkyl moiety and LG is a leaving group, for example halogen like chlorine, bromine or iodine, or a sulfonyloxy group like tosyloxy, mesyloxy or trifluormethylsulfonyloxy. LG may, for example, also be a hydroxy group which, in order to achieve the alkylation reaction, is activated by a conventional activating agent. For the preparation of compounds in which A is a direct linkage and an aromatic group is directly bonded to the 1-position of the benzoimidazole system, conventional arylation procedures can be used. For example aryl fluorides like alkyl fluorobenzoates or 4-fluorophenyl methyl sulfones can be employed as arylating agents. Such processes are described, for example, by M. Yamada et al. J. Med. Chem. 1996, 39, 596; J. Ohmori et al. J. Med. Chem. 1996, 39, 3971. Alternatively a wide variety of substituted aryl iodides, aryl bromides or aryl triflates can serve as arylating agents at the 1-position of the heterocyclic nitrogen in a copper salt or palladium mediated reaction according for example to P. Cozzi et al. Farmaco 1987, 42, 205; P. Unangst, D. Connor, R. Stabler, R. Weikert, J. Heterocycl. Chem. 1987, 24, 811; G. Tokmakov, I. Grandberg, Tetrahedron 1995, 51, 2091; D. Old, M. Harris, S. Buchwald, Org. Lett. 2000, 2, 1403, G. Mann, J. Hartwig, M. Driver, C. Fernandez-Rivas, J. Am. Chem. Soc. 1998, 120, 827; J. Hartwig, M. Kawatsura, S. Hauk, K. Shaughnessy, L. J. Org. Chem. 1999, 64, 5575; S. Buchwald et al., J. Am. Chem. Soc. 2001, 123, 7727. Moreover such arylations can also be accomplished by reaction of a wide range of substituted aryl boronic acids as demonstrated for example by W. Mederski, M. Lefort, M. Germann, D. Kux, Tetrahedron 1999, 55, 12757; J. Collman et al., J. Org. Chem. 2001, 66, 7892. During the above-mentioned transformations positional isomers may occur, nevertheless these mixtures of positional isomers can be separated by modern separation techniques like, for example, preparative HPLC.

Preferred methods include, but are not limited to those described in the examples.

The compounds of the present invention are serine protease inhibitors, which inhibit the activity of the blood coagulation enzyme factors Xa and/or factor VIIa. In particular, they are highly active inhibitors of factor Xa. They are specific serine protease inhibitors inasmuch as they do not substantially inhibit the activity of other proteases whose inhibition is not desired. The activity of the compounds of the formula I can be determined, for example, in the assays described below or in other assays known to those skilled in the art. With respect to factor Xa inhibition, a preferred embodiment of the invention comprises compounds which have a Ki < 1 mM for factor Xa inhibition as determined in the assay described below, with or without concomitant factor VIIa inhibition, and which preferably do not substantially inhibit the activity of other proteases involved in coagulation and fibrinolysis whose inhibition is not desired (using the same concentration of the inhibitor). The compounds of the invention inhibit factor Xa catalytic activity either directly, within the prothrombinase complex or as a soluble subunit, or indirectly, by inhibiting the assembly of factor Xa into the prothrombinase complex.

As inhibitors of factor Xa and/or factor VIIa the compounds of the formula I and their physiologically tolerable

salts and their prodrugs are generally suitable for the therapy and prophylaxis of conditions in which the activity of factor Xa and/or factor VIIa plays a role or has an undesired extent, or which can favorably be influenced by inhibiting factor Xa and/or factor VIIa or decreasing their activities, or for the prevention, alleviation or cure of which an inhibition of factor Xa and/or factor VIIa or a decrease in their activity is desired by the physician. As inhibition of factor Xa and/or factor VIIa influences blood coagulation and fibrinolysis, the compounds of the formula I and their physiologically tolerable salts and their prodrugs are generally suitable for reducing blood clotting, or for the therapy and prophylaxis of conditions in which the activity of the blood coagulation system plays a role or has an undesired extent, or which can favorably be influenced by reducing blood clotting, or for the prevention, alleviation or cure of which a decreased activity of the blood coagulation system is desired by the physician. A specific subject of the present invention thus are the reduction or inhibition of unwanted blood clotting, in particular in an individual, by administering an effective amount of a compound I or a physiologically tolerable salt or a prodrug thereof, as well as pharmaceutical preparations therefor.

The present invention also relates to the compounds of the formula I and/or their physiologically tolerable salts and/or their prodrugs for use as pharmaceuticals (or medicaments), to the use of the compounds of the formula I and/or their physiologically tolerable salts and/or their prodrugs for the production of pharmaceuticals for inhibition of factor Xa and/or factor VIIa or for influencing blood coagulation, inflammatory response or fibrinolysis or for the therapy or prophylaxis of the diseases mentioned above or below, for example for the production of pharmaceuticals for the therapy and prophylaxis of cardiovascular disorders, thromboembolic diseases or restenoses. The invention also relates to the use of the compounds of the formula I and/or their physiologically tolerable salts and/or their prodrugs for the inhibition of factor Xa and/or factor VIIa or for influencing blood coagulation or fibrinolysis or for the therapy or prophylaxis of the diseases mentioned above or below, for example for use in the therapy and prophylaxis of cardiovascular disorders, thromboembolic diseases or restenoses, and to methods of treatment aiming at such purposes including methods for said therapies and prophylaxis. The present invention also relates to pharmaceutical preparations (or pharmaceutical compositions) which contain an effective amount of at least one compound of the formula I and/or its physiologically tolerable salts and/or its prodrugs in addition to a customary pharmaceutically acceptable carrier, i. e. one or more pharmaceutically acceptable carrier substances or excipients and/or auxiliary substances or additives.

The invention also relates to the treatment of disease states such as abnormal thrombus formation, acute myocardial infarction, unstable angina, thromboembolism, acute vessel closure associated with thrombolytic therapy or percutaneous transluminal coronary angioplasty (PTCA), transient ischemic attacks, stroke, intermittent claudication or bypass grafting of the coronary or peripheral arteries, vessel luminal narrowing, restenosis post coronary or venous angioplasty, maintenance of vascular access patency in long-term hemodialysis patients, pathologic thrombus formation occurring in the veins of the lower extremities following abdominal, knee or hip surgery, pathologic thrombus formation occurring in the veins of the lower extremities following abdominal, knee and hip surgery, a risk of pulmonary thromboembolism, or disseminated systemic intravascular coagulatopathy occurring in vascular systems during septic shock, certain viral infections or cancer.

The compounds of the present invention can also be used to reduce an inflammatory response. Examples of specific disorders for the treatment or prophylaxis of which the compounds of the formula I can be used are coronary heart disease, myocardial infarction, angina pectoris, vascular restenosis, for example restenosis following angioplasty like PTCA, adult respiratory distress syndrome, multi-organ failure and disseminated intravascular clotting disorder. Examples of related complications associated with surgery are thromboses like deep vein and proximal vein thrombosis, which can occur following surgery.

The compounds of the formula I and their physiologically tolerable salts and their prodrugs can be administered to animals, preferably to mammals, and in particular to humans as pharmaceuticals for therapy or prophylaxis. They can be administered on their own, or in mixtures with one another or in the form of pharmaceutical preparations, which permit enteral or parenteral administration.

The pharmaceuticals can be administered orally, for example in the form of pills, tablets, lacquered tablets, coated tablets, granules, hard and soft gelatin capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The pharmaceutical preparations according to the invention are prepared in a manner known per se and familiar to one skilled in the art, pharmaceutically acceptable inert inorganic and/or organic carriers being used in addition to the compound(s) of the formula I and/or its (their) physiologically tolerable salts and/or its (their) prodrugs. For the production of pills, tablets, coated tablets and hard gelatin capsules it is possible to use, for example, lactose, cornstarch or derivatives thereof, talc, stearic acid or its salts, etc. Carriers for soft gelatin capsules and suppositories are, for example, fats, waxes, semisolid and liquid polyols, natural or hardened oils, etc. Suitable carriers for the production of solutions, for example injection solutions, or of emulsions or syrups are, for example, water, saline, alcohols, glycerol, polyols,

sucrose, invert sugar, glucose, vegetable oils, etc. Suitable carriers for microcapsules, implants or rods are, for example, copolymers of glycolic acid and lactic acid. The pharmaceutical preparations normally contain about 0.5 % to 90 % by weight of the compounds of the formula I and/or their physiologically tolerable salts and/or their prodrugs. The amount of the active ingredient of the formula I and/or its physiologically tolerable salts and/or its prodrugs in the pharmaceutical preparations normally is from about 0.5 mg to about 1000 mg, preferably from about 1 mg to about 500 mg.

In addition to the active ingredients of the formula I and/or their physiologically acceptable salts and/or prodrugs and to carrier substances, the pharmaceutical preparations can contain additives such as, for example, fillers, disintegrants, binders, lubricants, wetting agents, stabilizers, emulsifiers, preservatives, sweeteners, colorants, flavorings, aromatizers, thickeners, diluents, buffer substances, solvents, solubilizers, agents for achieving a depot effect, salts for altering the osmotic pressure, coating agents or antioxidants. They can also contain two or more compounds of the formula I, and/or their physiologically tolerable salts and/or their prodrugs. In case a pharmaceutical preparation contains two or more compounds of the formula I, the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in the compounds of the formula I allows a great deal of control over the biological and physico-chemical properties of the compounds and thus allows the selection of such desired compounds. Furthermore, in addition to at least one compound of the formula I and/or a physiologically tolerable salt and/or its prodrug, the pharmaceutical preparations can also contain one or more other therapeutically or prophylactically active ingredients.

When using the compounds of the formula I the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily dose for achieving the desired results in an adult weighing about 75 kg is from 0.01 mg/kg to 100 mg/kg, preferably from 0.1 mg/kg to 50 mg/kg, in particular from 0.1 mg/kg to 10 mg/kg, (in each case in mg per kg of body weight). The daily dose can be divided, in particular in the case of the administration of relatively large amounts, into several, for example 2, 3 or 4, part administrations. As usual, depending on individual behavior it may be necessary to deviate upwards or downwards from the daily dose indicated.

A compound of the formula I can also advantageously be used as an anticoagulant outside an individual. For example, an effective amount of a compound of the invention can be contacted with a freshly drawn blood sample to prevent coagulation of the blood sample. Further, a compound of the formula I or its salts can be used for diagnostic purposes, for example in in vitro diagnoses, and as an auxiliary in biochemical investigations. For example, a compound of the formula I can be used in an assay to identify the presence of factor Xa and/or factor VIIa or to isolate factor Xa and/or factor VIIa in a substantially purified form. A compound of the invention can be labeled with, for example, a radioisotope, and the labeled compound bound to factor Xa and/or factor VIIa is then detected using a routine method useful for detecting the particular label. Thus, a compound of the formula I or a salt thereof can be used as a probe to detect the location or amount of factor Xa and/or factor VIIa activity in vivo, in vitro or ex vivo.

Furthermore, the compounds of the formula I can be used as synthesis intermediates for the preparation of other compounds, in particular of other pharmaceutical active ingredients, which are obtainable from the compounds of the formula I, for example by introduction of substituents or modification of functional groups.

The general synthetic sequences for preparing the compounds useful in the present invention our outlined in the examples given below. Both an explanation of, and the actual procedure for, the various aspects of the present invention are described where appropriate. The following examples are intended to be merely illustrative of the present invention, and not limiting thereof in either scope or spirit. Those with skill in the art will readily understand that known variations of the conditions and processes described in the examples can be used to synthesize the compounds of the present invention.

It is understood that changes that do not substantially affect the activity of the various embodiments of this invention are included within the invention disclosed herein. Thus, the following examples are intended to illustrate but not limit the present invention.

Examples

When in the final step of the synthesis of a compound an acid such as trifluoroacetic acid or acetic acid was used, for example when trifluoroacetic acid was employed to remove a tBu group or when a compound was purified by chromatography using an eluent which contained such an acid, in some cases, depending on the work-up procedure, for example the details of a freeze-drying process, the compound was obtained partially or completely in the form of a salt of the acid used, for example in the form of the acetic acid salt or trifluoroacetic acid salt or hydrochloric acid salt.

Abbreviations used:

| | |
|---|---|
| tert-Butyl | tBu |
| 2,2'-bis(diphenylphoshino-1,1'-binaphthyl | Binap |
| Bis-(oxo-3-oxazolidinyl)-phosphoryl chloride | BOP-Cl |
| dibenzylidenacetone | dba |
| Dichloromethane | DCM |
| Dicyclohexyl-carbodiimide | DCC |
| Diethylphosphoryl cyanide | DEPC |
| Diisopropylethyl amine | DIPEA |
| 4-Dimethyaminopyridine | DMAP |
| N,N-Dimethylformamide | DMF |
| Dimethylsulfoxide | DMSO |
| 1,1'-Bis(diphenylphosphino)ferrocene | DPPF |
| O-(7-Azabenzotriazol-1-yl)-N,N,N',N'-tetramethyluronium-hexafluorophosphate | HATU |
| N-Bromosuccinimide | NBS |
| N-Chlorosuccinimide | NCS |
| N-Iodosuccinimide | NIS |
| N-Ethylmorpholine | NEM |
| Methanol | MeOH |
| Room temperature 20 °C to 25 °C | RT |
| Saturated | sat. |
| Tetrahydrofuran | THF |
| Trifluoroacetic acid | TFA |
| O-((Ethoxycarbonyl)cyanomethyleneamino)-N,N,N',N'-tetramethyluronium tetrafluoroborate | TOTU |

Example 1: 1-[5-(5-Chloro-thiophen-2-yl)-isoxazol-3-ylmethyl]-1H-benzoimidazole-2-carboxylic acid (1-isopropyl-piperidin-4-yl)-amide

(i) (1-Isopropyl-piperidin-4-yl)-carbamic acid tert-butyl ester

To a solution of 5.0 g Piperidin-4-yl-carbamic acid tert-butyl ester in 15 ml methanol, 7.34 ml acetone, 3.14 g $Na(CN)BH_3$ and 0.3 ml acetic acid were added. After stirring for 16h at room temperature the solvent was removed under reduced pressure and the residue was partitioned between 30 ml water and 30 ml ethyl acetate. The organic layer was washed with saturated $Na_2CO_3$ solution, water and then dried over $Na_2SO_4$. The solvent was removed under reduced pressure to give the product as a white solid. Yield: 4.8g MS ($ES^+$): m/e= 243.

(ii) 1-Isopropyl-piperidin-4-ylamine

To 4.8 g (1-Isopropyl-piperidin-4-yl)-carbamic acid tert-butyl ester in 15 ml methanol, 20 ml methanolic hydrochloric acid (8M) were added and the mixture was stirred for 16h. Removal of the solvent under reduced pressure, followed by removal of residual volatiles by twice coevaporating with toluene, gave the product.
Yield: 5.42 g MS ($ES^+$): m/e= 143.

(iii) 1H-Benzoimidazole-2-carboxylic acid (1-isopropyl-piperidin-4-yl)-amide

To a solution of 300 mg 1H-Benzoimidazole-2-carboxylic acid in 3 ml DMF and 1 ml $NEt_3$, 398 mg 1-Isopropyl-piperidin-4-ylamine hydrochloride and 470 mg BOP-Cl were added and the mixture was stirred for 3 h. Finally, 3 ml saturated $NaHCO_3$ solution were added and the mixture was filtered through a chem elut® cartridge by elution with ethyl acetate. After removal of the solvent under reduced pressure the crude product was subjected to the next reaction step without further purification.
Yield: 604 mg.

(iv) 1-[5-(5-Chloro-thiophen-2-yl)-isoxazol-3-ylmethyl]-1H-benzoimidazole-2-carboxylic acid (1-isopropyl-piperidin-4-yl)-amide

To a solution of 200 mg 1H-Benzoimidazole-2-carboxylic acid (1-isopropyl-piperidin-4-yl)-amide in 2 ml DMF, 227 mg $Cs_2CO_3$ and 194 mg 3-Bromomethyl-5-(5-chloro-thiophen-2-yl)-isoxazole [prepared by adopting a procedure described by Ewing, William R.; Becker, Michael R.; Choi-Sledeski, Yong Mi; Pauls, Heinz W.; He, Wei; Condon, Stephen M.; Davis, Roderick S.; Hanney, Barbara A.; Spada, Alfred P.; Burns, Christopher J.; Jiang, John Z.; Li, Aiwen; Myers, Michael R.; Lau, Wan F.; Poli, Gregory B; PCT Int. Appl. (2001), 460 pp. WO 0107436 A2] were added at RT and the mixture was stirred for 16 h. After addition of 5 ml water the mixture was filtered through a chem elut® cartridge by elution with ethyl acetate. After removal of the solvent under reduced pressure the residue was purified by preparative HPLC (C18 reverse phase column, elution with a $H_2O$/MeCN gradient with 0.1% TFA). The fractions containing the product were evaporated and lyophilized to yield a white solid. The product was obtained as its trifluoroacetate salt. Yield: 29 mg MS ($ES^+$): m/e= 484, chloro pattern.

Example 2: 1-[(4-Chloro-phenylcarbamoyl)-methyl]-1H-benzoimidazole-2-carboxylic acid (1-isopropyl- piperidin-4-yl)-amide

(i) 2-Bromo-N-(4-chloro-phenyl)-acetamide

To a solution of 5 g 4-Chloro-phenylamine and 1.5 ml pyridine in 30 ml toluene, 8 g bromoacetyl bromide dissolved in 10 ml toluene were added dropwise under ice cooling. After 2 h the precipitate was isolated by filtration and recrystallized from toluene to yield a white solid.
Yield: 10 g.

(ii) 1-[(4-Chloro-phenylcarbamoyl)-methyl]-1H-benzoimidazole-2-carboxylic acid (1-isopropyl-piperidin-4-yl)-amide

To a solution of 200 mg 1H-Benzoimidazole-2-carboxylic acid (1-isopropyl-piperidin-4-yl)-amide in 2 ml DMF, 227 mg $C_{S2}CO_3$ and 173 mg 2-Bromo-N-(4-chloro-phenyl)-acetamide were added at RT and the mixture was stirred for 16 h. After addition of 5 ml water the mixture was filtered through a chem elut® cartridge by elution with ethyl acetate. After removal of the solvent under reduced pressure the residue was purified by preparative HPLC (C18 reverse phase column, elution with a $H_2O$/MeCN gradient with 0.1% TFA). The fractions containing the product were evaporated and lyophilized to yield a white solid. The product was obtained as its trifluoroacetate salt. Yield: 63 mg MS ($ES^+$): m/e= 454, chloro pattern.

Example 3: 1-[(5-Chloro-pyridin-2-ylcarbamoyl)-methyl]-1H-benzoimidazole-2-carboxylic acid (1-isopropyl-piperidin-4-yl)-amide

(i) 2-Bromo-N-(5-chloro-pyridin-2-yl)-acetamide

To a solution of 5 g 5-Chloro-pyridin-2-ylamine and 1.5 ml pyridine in 30 ml toluene, 8 g bromoacetyl bromide dissolved in 10 ml toluene were added dropwise under ice cooling. After 2 h the precipitate was isolated by filtration and recrystallized from toluene to yield a white solid.
Yield: 12 g.

(ii) 1-[(5-Chloro-pyridin-2-ylcarbamoyl)-methyl]-1H-benzoimidazole-2-carboxylic acid (1-isopropyl-piperidin-4-yl)-amide

To a solution of 200 mg 1H-Benzoimidazole-2-carboxylic acid (1-isopropyl-piperidin-4-yl)-amide in 2 ml DMF, 227 mg $Cs_2CO_3$ and 174 mg 2-Bromo-N-(5-chloro-pyridin-2-yl)-acetamide were added at RT and the mixture was stirred for 16 h. After addition of 5 ml water the mixture was filtered through a chem elut® cartridge by elution with ethyl acetate. After removal of the solvent under reduced pressure the residue was purified by preparative HPLC (C18 reverse phase column, elution with a $H_2O$/MeCN gradient with 0.1% TFA). The fractions containing the product were evaporated and lyophilized to yield a white solid. The product was obtained as its trifluoroacetate salt. Yield: 34 mg MS ($ES^+$): m/e= 455, chloro pattern.

Example 4: 1-[5-(5-Chloro-thiophen-2-yl)-isoxazol-3-ylmethyl]-1H-benzoimidazole-2-carboxylic acid (1-pyrimidin-4-yl-piperidin-4-yl)-amide

(i) (1-Pyrimidin-4-yl-piperidin-4-yl)-carbamic acid tert-butyl ester

To a solution of 395 mg [1-(2-Chloro-pyrimidin-4-yl)-piperidin-4-yl]-carbamic acid tert-butyl ester in 10 ml ethanol and 0.3 ml acetic acid, 20 mg Pd/C (10%) were added and the mixture purged with argon for 10 min. Then the mixture was stirred under a hydrogen atmosphere for 5 h at RT. After addition of 10 ml ethyl acetate the reaction mixture was filtered through a pad of celite. The solvent was evaporated under reduced pressure and the residue codistilled twice with toluene to give the product as a white solid. Yield: 468 mg.

(ii) 1-Pyrimidin-4-yl-piperidin-4-ylamine

To a solution of 468 mg (1-Pyrimidin-4-yl-piperidin-4-yl)-carbamic acid tert-butyl ester in 2 ml DCM, 2 ml TFA were added and the mixture was stirred for 2 h at RT. Then, 10 ml toluene were added and the solvents were removed under reduced pressure. The residue was codistilled twice with toluene to yield a yellow oil. The product was obtained as its trifluoroacetate salt.
Yield: 703 mg.

(iii) 1H-Benzoimidazole-2-carboxylic acid (1-pyrimidin-4-yl-piperidin-4-yl)-amide

To a solution of 80 mg 1H-Benzoimidazole-2-carboxylic acid in 1 ml DMF and 0.2 ml $NEt_3$, 200 mg 1-Pyrimidin-4-yl-piperidin-4-ylamine trifluoroacetate and 125 mg BOP-Cl were added and the mixture was stirred for 3 h. Finally, 3 ml saturated $NaHCO_3$ solution were added and the mixture was filtered through a chem elut® cartridge by elution with ethyl acetate. After removal of the solvent under reduced pressure the crude product was subjected to the next reaction step without further purification. Yield: 160 mg.

(iv) 1-[5-(5-Chloro-thiophen-2-yl)-isoxazol-3-ylmethyl]-1H-benzoimidazole-2-carboxylic acid (1-pyrimidin-4-yl-piperidin-4-yl)-amide

To a solution of 160 mg 1H-Benzoimidazole-2-carboxylic acid (1-pyrimidin-4-yl-piperidin-4-yl)-amide in 2 ml DMF, 161 mg $Cs_2CO_3$ and 138 mg 3-Bromomethyl-5-(5-chloro-thiophen-2-yl)-isoxazole were added at RT and the mixture was stirred for 16 h. After addition of 5 ml water the mixture was filtered through a chem elut® cartridge by elution with ethyl acetate. After removal of the solvent under reduced pressure the residue was purified by preparative HPLC (C18 reverse phase column, elution with a $H_2O$/MeCN gradient with 0.1% TFA). The fractions containing the product were evaporated and lyophilized to yield a white solid. The product was obtained as its trifluoroacetate salt.
Yield: 114 mg MS ($ES^+$): m/e= 520, chloro pattern.

Example 5: a) 1-[5-(5-Chloro-thiophen-2-yl)-isoxazol-3-ylmethyl]-2-(1-isopropyl-piperidin-4-ylcarbamoyl)-1H-benzoimidazole-5-carboxylic acid methyl ester

b) 3-[5-(5-Chloro-thiophen-2-yl)-isoxazol-3-ylmethyl]-2-(1-isopropyl-piperidin-4-ylcarbamoyl)-3H-benzoimidazole-5-carboxylic acid methyl ester

(i) Methyl-2-trichloromethyl-1H-benzoimidazole-5-carboxylate

2.00 g (12.0 mmol) Methyl-3,4-diamino-benzoate were dissolved in 50 ml concentrated acetic acid. Then 2.09 ml (1.4 equiv.) methyl-2,2,2-trichloroacetimidate were added slowly and the resulting mixture was stirred at room temperature for 2 h. The mixture was diluted with 100 ml toluene and the solvent was removed under reduced pressure. The residue was taken up in dichloromethane and washed once with a saturated $NaHCO_3$-solution and once with brine. The organic layer was dried over $MgSO_4$ and the solvent was removed under reduced pressure to give pure methyl-2-trichloromethyl-1H-benzoimidazole-5-carboxylate as a light brown amorphous solid. Yield: 3.64 g MS ($ES^+$): m/e = 293, chloro pattern.

(ii) 2-(1-Isopropyl-piperidin-4-ylcarbamoyl)-1H-benzoimidazole-5-carboxylic acid methyl ester

500 mg (1.7 mmol) Methyl-2-trichloromethyl-1H-benzoimidazole-5-carboxylate were added to a mixture of 548 mg (1.8 equiv.) 1-isopropyl-piperidine-4-ylamine-dihydrochloride and 1.43 g (10 equiv.) $NaHCO_3$ in 15 ml THF and

7.5 ml $H_2O$ and stirred vigorously for 4 h at room temperature.

The reaction mixture was diluted with dichloromethane and washed with a saturated $NaHCO_3$-solution and brine. The organic layer was dried over $MgSO_4$ and concentrated. Preparative HPLC ($CH_3CN/H_2O$ gradient + 0.05 % formic acid) gave pure 2-(1-isopropyl-piperidin-4-ylcarbamoyl)-1H-benzoimidazole-5-carboxylic acid methyl ester as a white solid.

Yield: 300 mg MS ($ES^+$): m/e = 345.

(iii) a) 1-[5-(5-Chloro-thiophen-2-yl)-isoxazol-3-ylmethyl]-2-(1-isopropyl-piperidin-4-ylcarbamoyl)-1H-benzoimidazole-5-carboxylic acid methyl ester

b) 3-[5-(5-Chloro-thiophen-2-yl)-isoxazol-3-ylmethyl]-2-(1-isopropyl-piperidin-4-ylcarbamoyl)-3H-benzoimidazole-5-carboxylic acid methyl ester

115 mg ( 0.33 mmol) 2-(1-Isopropyl-piperidin-4-ylcarbamoyl)-1H-benzoimidazole-5-carboxylic acid methyl ester were dissolved in 10 ml DMF. Subsequently 69.2 mg (1. 5 equiv.) $K_2CO_3$ and 111.6 mg (1.2 equiv.) 3-bromomethyl-5-(5-chloro-thiophen-2-yl)-isoxazole were added and the resulting mixture was stirred for 2 h at 80° C. The reaction mixture was diluted with toluene and washed twice with a saturated $NaHCO_3$-solution and once with brine, dried over anhydrous $MgSO_4$ and concentrated under reduced pressure. Preparative RP-HPLC ($CH_3CN/H_2O$ gradient + 0.05 % formic acid) gave a 6:4 mixture of both isomers described in the title. These isomers could be separated by NP-HPLC using a chiral stationary phase and a mixture of heptane, ethanol, methanol and diethyl amine as solvent. Structural assignment of both isomers was achieved by NOE-spectroscopy.

Yield of 1-[5-(5-chloro-thiophen-2-yl)-isoxazo[-3-ylmethyl]-2-(1-isopropyl-piperidin-4-ylcarbamoyl)-1H-benzoimidazole-5-carboxylic acid methyl ester: 52 mg; MS ($ES^+$): m/e =542, chloro pattern.

Yield of 3-[5-(5-chloro-thiophen-2-yl)-isoxazol-3-ylmethyl]-2-(1-isopropyl-piperidin-4-ylcarbamoyl)-3H-benzoimidazole-5-carboxylic acid methyl ester: 34 mg; MS ($ES^+$): m/e =542, chloro pattern.

Example 6: 1-[5-(5-Chloro-thiophen-2-yl)-isoxazol-3-ylmethyl]-2-(1-isopropyl-piperidin-4-ylcarbamoyl)-1H-benzoimidazole-5-carboxylic acid

To a suspension of 43.6 mg (0.080 mmol) 1-[5-(5-chloro-thiophen-2-yl)-isoxazol-3-ylmethyl]-2-(1-isopropyl-piperidin-4-ylcarbamoyl)-1H-benzoimidazole-5-carboxylic acid methyl ester in 4 ml MeOH 0.4 ml of a 1 M aqueous LiOH-solution were added and the resulting mixture was stirred at 60 °C for 5 h. The mixture was acidified by the addition of a 1 M HCl-solution (pH ≈ 2-3) and concentrated under reduced pressure. Final purification by preparative RP-HPLC ($CH_3CN/H_2O$ gradient + 0.05 % formic acid) gave pure 1-[5-(5-chloro-thiophen-2-yl)-isoxazol-3-ylmethyl]-2-(1-isopropyl-piperidin-4-ylcarbamoyl)-1H-benzoimidazole-5-carboxylic acid. The corresponding dihydrochloride was obtained by treatment of the product with a 0.1 M HCl-solution followed by lyophilization. Yield: 43 mg MS ($ES^+$): m/e =528, chloro pattern.

Example 7: 3-[5-(5-Chloro-thiophen-2-yl)-isoxazol-3-ylmethyl]-2-(1-isopropyl-piperidin-4-ylcarbamoyl)-3H-benzoimidazole-5-carboxylic acid

3-[5-(5-Chloro-thiophen-2-yl)-isoxazol-3-ylmethyl]-2-(1-isopropyl-piperidin-4-ylcarbamoyl)-3H-benzoimidazole-5-carboxylic acid was prepared as described in example 6 from 20 mg ( 0.037 mmol) 3-[5-(5-chloro-thiophen-2-yl)-isoxazol-3-ylmethyl]-2-(1-isopropyl-piperidin-4-ylcarbamoyl)-3H-benzoimidazole-5-carboxylic acid methyl ester.
Yield: 18 mg MS ($ES^+$): m/e =528, chloro pattern.

Example 8: a) 1-[2-(5-Chloro-thiophen-2-yl)-thiazol-5-ylmethyl]-2-(1-isopropyl-piperidin-4-ylcarbamoyl)-1H-benzoimidazole-5-carboxylic acid methyl ester

b) 3-[2-(5-Chloro-thiophen-2-yl)-thiazol-5-ylmethyl]-2-(1-isopropyl-piperidin-4-ylcarbamoyl)-3H-benzoimidazole-5-carboxylic acid methyl ester

To a solution of 80.0 mg (0.23 mmol) 2-(1-isopropyl-piperidin-4-ylcarbamoyl)-1H-benzoimidazole-5-carboxylic acid methyl ester in 5 ml DMF 9.3 mg sodium hydride (60% supension in mineral oil) were added. The mixture was stirred for 30 min at room temperature and subsequently 68.4 mg (0.23 mmol) 5-bromomethyl-2-(5-chloro-thiophen-2-yl)-thiazole [prepared by adopting a procedure described by Ewing, William R. et al.; PCT Int. Appl. (2001), 460 pp. WO 0107436 A2] were added. After 1H another 17.1 mg (0.25 equiv.) 5-bromomethyl-2-(5-chloro-thiophen-2-yl)-thiazole were added and the reaction mixture was stirred for further 2 h. The reaction was stopped by the careful addition

of MeOH and water. The solvent was removed under reduced pressure and the residue was purified by preparative RP-HPLC ($CH_3CN/H_2O$ gradient + 0.05 % formic acid). The two isomers were then separated using NP-HPLC on a chiral stationary phase with a mixture of heptane, ethanol, methanol and diethyl amine as solvent. Structural assignment of both isomers was achieved by NOE-spectroscopy.
Yield of 1-[2-(5-chloro-thiophen-2-yl)-thiazol-5-ylmethyl]-2-(1-isopropyl-piperidin-4-ylcarbamoyl)-1H-benzoimidazole-5-carboxylic acid methyl ester: 40 mg; MS ($ES^+$): m/e =558, chloro pattern.
Yield of 3-[2-(5-chloro-thiophen-2-yl)-thiazol-5-ylmethyl]-2-(1-isopropyl-piperidin-4-ylcarbamoyl)-3H-benzoimidazole-5-carboxylic acid methyl ester: 26 mg; MS ($ES^+$): m/e =558, chloro pattern.

Example 9: 1-[2-(5-Chloro-thiophen-2-yl)-thiazol-5-ylmethyl]-2-(1-isopropyl-piperidin-4-ylcarbamoyl)-1H-benzoimidazole-5-carboxylic acid

1-[2-(5-Chloro-thiophen-2-yl)-thiazol-5-ylmethyl]-2-(1-isopropyl-piperidin-4-ylcarbamoyl)-1H-benzoimidazole-5-carboxylic acid was prepared by a procedure according to example 6 starting from 40 mg (0.072 mmol) 1-[2-(5-chloro-thiophen-2-yl)-thiazol-5-ylmethyl]-2-(1-isopropyl-piperidin-4- ylcarbamoyl)-1H-benzoimidazole-5-carboxylic acid methyl ester. The title compound was obtained as its dihydrochloride. Yield: 23 mg MS ($ES^+$): m/e =544, chloro pattern.

Example 10: 3-[2-(5-Chloro-thiophen-2-yl)-thiazol-5-ylmethyl]-2-(1-isopropyl-piperidin-4-ylcarbamoyl)-3H-benzoimidazole-5-carboxylic acid

3-[2-(5-Chloro-thiophen-2-yl)-thiazol-5-ylmethyl]-2-(1-isopropyl-piperidin-4-ylcarbamoyl)-3H-benzoimidazole-5-carboxylic acid was prepared by a procedure according to example 6 starting from 26 mg (0.047 mmol) 3-[2-(5-chloro-thiophen-2-yl)-thiazol-5-ylmethyl]-2-(1-isopropyl-piperidin-4- ylcarbamoyl)-3H-benzoimidazole-5-carboxylic acid methyl ester. The title compound was obtained as its dihydrochloride. Yield: 19 mg MS ($ES^+$): m/e =544, chloro pattern.

Example 11: a) 1-[5-(5-Chloro-thiophen-2-yl)-isoxazol-3-ylmethyl]-2-(1-isopropyl-piperidin-4-ylcarbamoyl)-1H-benzoimidazole-4-carboxylic acid methyl ester

b) 3-[5-(5-Chloro-thiophen-2-yl)-isoxazol-3-ylmethyl]-2-(1-isopropyl-piperidin-4-ylcarbamoyl)-3H-benzoimidazole-4-carboxylic acid methyl ester

(i) Methyl-2,3-diamino-benzoate

3.0 g (15.3 mmol) Methyl-2-amino-3-nitro-benzoate were dissolved in 200 ml abs. MeOH. The solution was evacuated and rinsed with argon several times. 300 mg palladium on charcoal (10%) were added and again the mixture was evacuated and rinsed with argon a several times. Finally argon was exchanged by hydrogen (balloon filled with hydrogen) and the mixture was stirred for 4 h at room temperature. The reaction mixture was filtered over "Celite" and the filter residue was washed with 150 ml methanol. The filtrate was concentrated under vacuo to give pure methyl-2,3-diamino-benzoate as a brown oil.
Yield: 2.53 g MS ($ES^+$): m/e = 167.

(ii) Methyl-2-trichloromethyl-1H-benzoimidazole-4-carboxylate

Methyl-2-trichloromethyl-1H-benzoimidazole-4-carboxylate was prepared similarly to methyl-2-trichloromethyl-1H-benzoimidazole-5-carboxylate as described in example 5 i) starting from 2.53 g (15.1 mmol) methyl-2,3-diamino-benzoate.
Yield: 4.15 g MS ($ES^+$): m/e =293, chloro pattern.

(iii) 2-(1-Isopropyl-piperidin-4-ylcarbamoyl)-1H-benzoimidazole-4-carboxylic acid methyl ester

2-(1-Isopropyl-piperidin-4-ylcarbamoyl)-1H-benzoimidazole-4-carboxylic acid methyl ester was prepared from 1.82 g (6.2 mmol) methyl-2-trichloromethyl-1H-benzoimidazole-4-carboxylate as described in example 5 ii). Yield: 1.10 g MS ($ES^+$): m/e = 345.

(iv) a) 1-[5-(5-Chloro-thiophen-2-yl)-isoxazol-3-ylmethyl]-2-(1-isopropyl-piperidin-4-ylcarbamoyl)-1H-benzoimidazole-4-carboxylic acid methyl ester

b) 3-[5-(5-Chloro-thiophen-2-yl)-isoxazol-3-ylmethyl]-2-(1-isopropyl-piperidin-4-ylcarbamoyl)-3H-benzoimidazole-4-carboxylic acid methyl ester

Both isomers were obtained by an analogous procedure as described for example 5 iii) starting from 150.0 mg (0.44 mmol) 2-(1-isopropyl-piperidin-4-ylcarbamoyl)-1H-benzoimidazole-4-carboxylic acid methyl ester and 145.6 mg (1.2 equiv.) 3-bromomethyl-5-(5-chloro-thiophen-2-yl)-isoxazole. In this case the ratio of both isomers was 3:1. As described in example 5 iii) the isomers were separated by NP-HPLC using a chiral stationary phase and a mixture of heptane, ethanol, methanol and diethyl amine as solvent. Again structural assignment of both isomers was achieved by NOE-spectroscopy.
Yield of 1-[5-(5-chloro-thiophen-2-yl)-isoxazol-3-ylmethyl]-2-(1-isopropyl-piperidin-4-ylcarbamoyl)-1H-benzoimidazole-4-carboxylic acid methyl ester: 105 mg; MS ($ES^+$): m/e =542, chloro pattern.
Yield of 3-[5-(5-chloro-thiophen-2-yl)-isoxazol-3-ylmethyl]-2-(1-isopropyl-piperidin-4-ylcarbamoyl)-3H-benzoimidazole-4-carboxylic acid methyl ester: 45 mg; MS ($ES^+$): m/e =542, chloro pattern.

Example 12: 1-[5-(5-Chloro-thiophen-2-yl)-isoxazol-3-ylmethyl]-2-(1-isopropyl-piperidin-4-ylcarbamoyl)-1H-benzoimidazole-4-carboxylic acid

1-[5-(5-Chloro-thiophen-2-yl)-isoxazol-3-ylmethyl]-2-(1-isopropyl-piperidin-4-ylcarbamoyl)-1H-benzoimidazole-4-carboxylic acid was prepared from 60 mg (0.111 mmol) 1-[5-(5-chlorothiophen-2-yl)-isoxazol-3-ylmethyl]-2-(1-isopropyl-piperidin-4-ylcarbamoyl)-1H-benzoimidazole-4-carboxylic acid methyl ester as described in example 6. The title compound was obtained as its dihydrochloride. Yield: 34 mg MS ($ES^+$): m/e = 528, chloro pattern.

Example 13: 3-[5-(5-Chloro-thiophen-2-yl)-isoxazol-3-ylmethyl]-2-(1-isopropyl-piperidin-4-ylcarbamoyl)-3H-benzoimidazole-4-carboxylic acid

3-[5-(5-Chloro-thiophen-2-yl)-isoxazo)-3-ylmethyl]-2-(1-isopropy)-piperidin-4-ylcarbamoyl)-3H-benzoimidazole-4-carboxylic acid was prepared from 20 mg (0.037 mmol) 3-[5-(5-chloro-thiophen-2-yl)-isoxazol-3-ylmethyl]-2-(1-isopropyl-piperidin-4-ylcarbamoyl)-3H-benzoimidazole-4-carboxylic acid methyl ester as described in example 6. The title compound was obtained as its dihydrochloride. Yield: 16 mg MS ($ES^+$): m/e = 528, chloro pattern.

Example 14: 3-[5-(5-Chloro-thiophen-2-yl)-isoxazol-3-ylmethyl]-3H-imidazo[4,5-b]pyridine-2-carboxylic acid (1-isopropyl-piperidin-4-yl)-amide

(i) 3H-Imidazo[4,5-b]pyridine-2-carboxylic acid methyl ester

1.76 g (9.16 mmol) methyl-dichloro-methoxy-acetate were added to a solution of 1.00 g (9.16 mmol) 2,3-diamino-pyridine in 40 ml methanol and stirred at room temperature. 1.85 g (18.32 mmol) triethyl amine were added dropwise . After complete addition the reaction mixture was stirred for 15 h at 80°C. The reaction was not complete resulting in the additon of another 1.76 g methyl-dichloro-methoxy-acetate and 1.85 g triethyl amine. Again the reaction mixture was stirred for 8 h at 80°C. The mixture was concentrated under reduced pressure and the residue was digerated sequentially with diethyl ether and a saturated $NaHCO_3$-solution and then washed with water yielding almost pure 3H-imidazo[4,5-b]pyridine-2-carboxylic acid methyl ester.
Yield: 210 mg MS ($ES^+$): m/e = 178.

(ii) 3-[5-(5-Chloro-thiophen-2-yl)-isoxazol-3-ymethyl]-3H-imidazo[4,5-b]pyridine-2- carboxylic acid methyl ester

100 mg (0.56 mmol) 3H-Imidazo[4,5-b]pyridine-2-carboxylic acid methyl ester were dissolved in 3 ml DMF. 22.6 mg (0.56 mmol) sodium hydride (60% in mineral oil) were added and the mixture was stirred 30 minutes at room temperature. 157.2 mg (0.56 mmol) 3-Bromomethyl-5-(5-chloro-thiophen-2-yl)-isoxazole were added and the resulting mixture was stirred for 1H at 80°C. The reaction mixture was cooled to room temperature and after the addition of a few drops of water concentrated under reduced pressure. HPLC-MS-analysis showed the presence of another isomer (ratio of isomers ≈ 7:1). Final purification by preparative RP-HPLC ($CH_3CN/H_2O$ gradient + 0.05 % formic acid) gave pure 3-[5-(5-chloro-thiophen-2-yl)-isoxazol-3-ylmethyl]-3H-imidazo[4,5-b]pyridine-2- carboxylic acid methyl ester as a brown amorphous solid.
Yield: 203 mg MS ($ES^+$): m/e = 375, chloro pattern.

(iii) 3-[5-(5-Chloro-thiophen-2-yl)-isoxazol-3-ylmethyl]-3H-imidazo[4,5-b] pyridine-2-carboxylic acid

175.0 mg (0.46 mmol) 3-[5-(5-Chloro-thiophen-2-yl)-isoxazol-3-ylmethyl]-3H-imidazo[4,5-b]pyridine-2- carboxylic acid methyl ester were added to a solution of 19.6 mg (0.82 mmol) LiOH in 6 ml THF and 2 ml $H_2O$. The reaction mixture was stirred for 2 h at 60°C, cooled to room temperature and acidified (pH= 2) by the addition of a half concentrated HCl-solution. The precipitate was filtered off and washed with water to give pure 3-[5-(5-chloro-thiophen-2-yl)-isoxazol-3-ylmethyl]-3H-imidazo[4,5-b] pyridine-2-carboxylic acid as a brown crystalline solid. Yield: 150 mg MS ($ES^+$): m/e = 361, chloro pattern.

(iv) 3-[5-(5-Chloro-thiophen-2-yl)-isoxazol-3-ylmethyl]-3H-imidazo[4,5-b]pyridine-2- carboxylic acid (1-isopropyl-piperidin-4-yl)-amide

150.0 mg (0.41 mmol) 3-[5-(5-Chloro-thiophen-2-yl)-isoxazol-3-ylmethyl]-3H-imidazo[4,5-b] pyridine-2-carboxylic acid were dissolved in 4 ml DMF. Sequentially 142 µl DIPEA and 151.8 mg (0.41 mmol) HATU were added and the mixture was stirred for 20 min at room temperature. 74.3 mg (0.41 mmol) 1-Isopropyl-piperidine-4-ylamine-hydrochloride and another 71 µl DIPEA were added and the resulting mixture was stirred 3 h at room temperature. Concentration under reduced pressure and final purification by preparative RP-HPLC ($CH_3CN/H_2O$ gradient + 0.05 % formic acid) gave pure 3-[5-(5-chloro-thiophen-2-yl)-isoxazol-3-ylmethyl]-3H-imidazo[4,5-b]pyridine-2- carboxylic acid (1-isopropyl-piperidin-4-yl)-amide as a light brown amorphous solid. The title compound was obtained as its hydroformiate. Yield: 17 mg MS ($ES^+$): m/e = 485, chloro pattern.

Example 15: 1-[(5-Chloro-pyridin-2-ylcarbamoyl)-methyl]- 2-(1-isopropyl-piperidin-4-ylcarbamoyl)-1H-benzoimidazole-4-carboxylic acid methyl ester

100.8 mg (0.29 mmol) 2-(Isopropyl-piperidin-4-ylcarbamoyl)-1H-benzoimidazole-4-carboxylic acid methyl ester were dissolved in 6 ml DMF. Subsequently 60.6 mg (0.44 mmol) $K_2CO_3$ and 87.5 mg (0.35 mmol) 2-bromo-*N*-(5-chloro-pyridin-2-yl-)-acetamide were added and the resulting mixture was stirred for 3h at 80°C. The reaction mixture was diluted with 60 ml toluene and washed with a sat. $NaHCO_3$ solution and brine. The organic layer was dried over $MgSO_4$ and the solvent was removed under reduced pressure. The residue was purified by preparative HPLC ($CH_3CN/H_2O$ gradient + 0.05 % formic acid) to give 1-[(5-chloro-pyridin-2-ylcarbamoyl)-methyl]-2-(1-isopropyl-piperidin-4- ylcarbamoyl)-1H-benzoimidazole-4-carboxylic acid methyl ester as a white amorphous solid. The product was obtained as its hydroformiate.
Yield: 106 mg MS ($ES^+$): m/e = 513, chloro pattern.

Example 16: 1-[(5-Chloro-pyridin-2-ylcarbamoyl)-methyl]- 2-(1-isopropyl-piperidin-4-ylcarbamoyl)-1H-benzoimidazole-4-carboxylic acid

30 mg (0.058 mmol) 1-[(5-Chloro-pyridin-2-ylcarbamoyl)-methyl]- 2-(1-isopropyl-piperidin-4-ylcarbamoyl)-1H-benzoimidazole-4-carboxylic acid methyl ester were dissolved in 3 ml dichloromethane and cooled to 0°C. 234 µl of a 1 M $BBr_3$-solution (4 equiv.) in dichloromethane were carefully added and the resulting mixture was stirred at room temperature overnight. Under cooling 3 ml water were added dropwise followed by the addition of 0.7 ml of a 1 M NaOH-solution. The mixture was concentrated under reduced pressure. Purification by preparative HPLC ($CH_3CN/H_2O$ gradient + 0.05 % formic acid) gave pure 1-[(5-chloro-pyridin-2-ylcarbamoyl)-methyl]- 2-(1-isopropyl-piperidin-4-ylcarbamoyl)-1H-benzoimidazole-4-carboxylic acid. The corresponding dihydrochloride was obtained by treatment of the product with a 0.1 M HCl-solution and following lyophilization.
Yield: 26 mg MS ($ES^+$): m/e = 499, chloro pattern.

Example 17: a) 1-[(5-Chloro-pyridin-2-ylcarbamoyl)-methyl]-2-(1-isopropyl-piperidin-4-ylcarbamoyl)-1H-benzoimidazole-5-carboxylic acid methyl ester

b) 3-[(5-Chloro-pyridin-2-ylcarbamoyl)-methyl]-2-(1-isopropyl-piperidin-4-ylcarbamoyl)-3H-benzoimidazole-5-carboxylic acid methyl ester

102.3 mg (0.297 mmol) 2-(Isopropyl-piperidin-4-ylcarbamoyl)-1H-benzoimidazole-5-carboxylic acid methyl ester were dissolved in 6 ml DMF. Subsequently 61.6 mg (0.446 mmol) $K_2CO_3$ and 88.9 mg (0.356 mmol) 2-bromo-*N*-(5-chloro-pyridin-2-yl-)-acetamide were added and the resulting mixture was stirred for 2h at 80°C. The reaction mixture was diluted with 60 ml toluene and washed with sat. $NaHCO_3$ solution and brine. The organic layer was dried over $MgSO_4$ and the solvent was removed under reduced pressure. The residue was purified by preparative HPLC

($CH_3CN/H_2O$ gradient + 0.05 % formic acid) to give both isomers described in the title in a 1.3:1 ratio. Both isomers could be separated by NP-HPLC using a chiral stationary phase and a mixture of heptane, ethanol and methanol as solvent. Structural assignment of both isomers was achieved by NOE-spectroscopy. Both isomers were transformed into their dihydrochloride by treatment with a 0.1 M HCl-solution and following lyophilization.
Yield of 1-[(5-chloro- pyridin-2-ylcarbamoyl)-methyl]- 2-(1-isopropyl-piperidin-4-ylcarbamoyl)-1H-benzoimidazole-5-carboxylic acid methyl ester: 50 mg MS ($ES^+$): m/e = 513, chloro pattern. Yield of 3-[(5-chloro-pyridin-2-ylcarbamoyl)-methyl]- 2-(1-isopropyl-piperidin-4-ylcarbamoyl)-3H-benzoimidazole-5-carboxylic acid methyl ester: 51 mg MS ($ES^+$): m/e = 513, chloro pattern.

Example 18: 1-[(5-Chloro-pyridin-2-ylcarbamoyl)-methyl]- 2-(1-isopropyl-piperidin-4-ylcarbamoyl)-1H-benzoimidazole-5-carboxylic acid

1-[(5-Chloro-pyridin-2-ylcarbamoyl)-methyl]- 2-(1-isopropyl-piperidin-4-ylcarbamoyl)-1H-benzoimidazole-5-carboxylic acid was prepared by a procedure according to example 16 starting from 27.8 mg (0.047 mmol) 1-[(5-chloro-pyridin-2-ylcarbamoyl)-methyl]- 2-(1-isopropyl-piperidin-4-ylcarbamoyl)-1H-benzoimidazole-5-carboxylic acid methyl ester. The title compound was obtained as its dihydrochloride. Yield: 15 mg MS ($ES^+$): m/e = 499, chloro pattern.

Example 19: 3-[(5-Chloro-pyridin-2-ylcarbamoyl)-methyl]- 2-(1-isopropyl-piperidin-4-ylcarbamoyl)-3H-benzoimidazole-5-carboxylic acid

3-[(5-Chloro-pyridin-2-ylcarbamoyl)-methyl]- 2-(1-isopropyl-piperidin-4-ylcarbamoyl)-3H-benzoimidazole-5-carboxylic acid was prepared by a procedure according to example 16 starting from 28 mg (0.048 mmol) 3-[(5-chloro-pyridin-2-ylcarbamoyl)-methyl]- 2-(1-isopropyl-piperidin-4-ylcarbamoyl)-3H-benzoimidazole-5-carboxylic acid methyl ester. The title compound was obtained as its dihydrochloride. Yield: 22 mg MS ($ES^+$): m/e = 499, chloro pattern.

Example 20: 1-[5-(5-Chloro-thiophen-2-yl)-isoxazol-3-ylmethyl]-2-(1-isopropyl-piperidin-4-ylcarbamoyl)-1H-benzoimidazole-4-carboxylic acid 1-ethoxycarbonyloxy-ethyl ester

50 mg (0.08 mmol) 1-[5-(5-Chloro-thiophen-2-yl)-isoxazol-3-ylmethyl]-2-(1-isopropyl-piperidin-4-ylcarbamoyl)-1H-benzoimidazole-4-carboxylic acid were dissolved in 3 ml DMF. Subsequently 27.6 mg (0.16 mmol) KI, 46 mg (0.33 mmol) $K_2CO_3$ and 45 µl (0.32 mmol) 1-chloroethyl-ethylcarbonate were added and the reaction mixture was stirred at 60°C for 4 h. The reaction mixture was concentrated and the resulting residue purified by preparative HPLC ($CH_3CN/H_2O$ gradient + 0.05 % formic acid) to give pure 1-[5-(5-chloro-thiophen-2-yl)-isoxazol-3-ylmethyl]-2-(1-isopropyl-piperidin-4- ylcarbamoyl)-1H-benzoimidazole-4-carboxylic acid 1-ethoxycarbonyloxy-ethyl ester as a white amorphous solid. The corresponding dihydrochloride was obtained by treatment with a 0.1 M HCl-solution and following lyophilization.
Yield: 46 mg MS ($ES^+$): m/e = 644, chloro pattern.

Example 21: 1-[5-(5-Chloro-thiophen-2-yl)-isoxazol-3-ylmethyl]-2-(1-isopropyl-piperidin-4-ylcarbamoyl)-1H-benzoimidazole-4-carboxylic acid 1-cyclohexyloxycarbonyloxy-ethyl ester

1-[5-(5-Chloro-thiophen-2-yl)-isoxazol-3-ylmethyl]-3-(1-isopropyl-piperidin-4- ylcarbamoyl)-1H-benzoimidazole-4-carboxylic acid 1-cyclohexyloxycarbonyloxy-ethyl ester was prepared by adopting the procedure described for example 20 starting from 50 mg (0.08 mmol) 1-[5-(5-chloro-thiophen-2-yl)-isoxazol-3-ylmethyl]-2-(1-isopropyl-piperidin-4-ylcarbamoyl)-1H-benzoimidazole-4-carboxylic acid and 61 µl (0.32 mmol) cyclohexyl-1-chloroethyl carbonate. The title compound was obtained as its dihydrochloride.
Yield: 47 mg MS (ES+): m/e = 698, chloro pattern.

Example 22: 1-(5-(5-Chloro-thiophen-2-yl)-isoxazol-3-ylmethyl]-1H-benzoimidazole-2,4-dicarboxylic acid 4-[(2-hydroxy-ethyl)-amide] 2-[(1-isopropyl-piperidin-4-yl)-amide]

30 mg (0.057 mmol) 1-[5-(5-Chloro-thiophen-2-yl)-isoxazol-3-ylmethyl]-2-(1-isopropyl-piperidin-4-ylcarbamoyl)-1H-benzoimidazole-4-carboxylic acid were dissolved in 3 ml DMF. Subsequently 29 µl (0.171 mmol) DIPEA and 21.6 mg (0.057 mmol) HATU were added. After 30 minutes 3.5 µl (0.057 mmol) 2-amino-ethanol and 10 µl DIPEA were added and the resulting mixture was stirred at room temperature for 16 h. The reaction mixture was concentrated and the residue was purified by preparative HPLC ($CH_3CN/H_2O$ gradient + 0.05 % formic acid). The title compound

was obtained as its hydroformiate in form of a white amorphous solid.
Yield: 29 mg MS ($ES^+$): m/e = 571, chloro pattern.

Example 23: 1-[5-(5-Chloro-thiophen-2-yl)-isoxazol-3-ylmethyl]-4-(3-hydroxy-azetidine-1-carbonyl)-1H-benzoimidazole-2-carboxylic acid (1-isopropyl-piperidin-4-yl)-amide

1-[5-(5-Chloro-thiophen-2-yl)-isoxazol-3-ylmethyl]-4-(3-hydroxy-azetidine-1-carbonyl)-1H-benzoimidazole-2-carboxylic acid (1-isopropyl-piperidin-4-yl)-amide was prepared by a procedure according to example 22 starting from 50 mg (0.08 mmol) 1-[5-(5-chloro-thiophen-2-yl)-isoxazol-3-ylmethyl]-2-(1-isopropyl-piperidin-4-ylcarbamoyl)-1H-benzoimidazole-4-carboxylic acid and 6.7 mg (0.09 mmol) 3-hydroxy-acetidine. The title compound was obtained as its hydroformiate in form of a white amorphous solid.
Yield: 51 mg MS ($ES^+$): m/e = 583, chloro pattern.

Example 24: 1-[5-(5-Chloro-thiophen-2-yl)-isoxazol-3-ylmethyl]-1H-benzoimidazole-2,4-dicarboxylic acid 4-[(2-hydroxy-ethyl)-methyl-amide] 2-[(1-isopropyl-piperidin-4-yl)-amide]

1-[5-(5-Chloro-thiophen-2-yl)-isoxazol-3-ylmethyl]-1H-benzoimidazole-2,4-dicarboxylic acid 4-[(2-hydroxy-ethyl)-methyl-amide] 2-[(1-isopropyl-piperidin-4-yl)-amide] was prepared by a procedure according to example 22 starting from 40 mg (0.067 mmol) 1-[5-(5-chloro-thiophen-2-yl)-isoxazol-3-ylmethyl]-2-(1-isopropyl-piperidin-4-ylcarbarnoyl)-1H-benzoimidazole-4-carboxylic acid and 5.6 mg (0.075 mmol) N-methyl-2-aminoethanol. The title compound was obtained as its hydroformiate in form of a white amorphous solid.
Yield: 35 mg MS ($ES^+$): m/e = 585, chloro pattern.

Example 25: 1-[5-(5-Chloro-thiophen-2-yl)-isoxazol-3-ylmethyl]-1H-benzoimidazole-2,5-dicarboxylic acid 5-[(2-hydroxy-ethyl)-methyl-amide] 2-[(1-isopropyl-piperidin-4-yl)-amide]

1-[5-(5-Chloro-thiophen-2-yl)-isoxazol-3-ylmethyl-1H-benzoimidazole-2,5-dicarboxylic acid 5-[(2-hydroxy-ethyl)-methyl-amide] 2-[(1-isopropyl-piperidin-4-yl)-amide] was prepared by a procedure according to example 22 starting from 50 mg (0.08 mmol) 1-[5-(5-chloro-thiophen-2-yl)-isoxazol-3-ylmethyl]-2-(1-isopropyl-piperidin-4-ylcarbamoyl)-1H-benzoimidazole-5-carboxylic acid and 6.8 mg (0.09 mmol) N-methyl-2-aminoethanol. The title compound was obtained as its hydroformiate in form of a white amorphous solid.
Yield: 43 mg MS ($ES^+$): m/e = 585, chloro pattern.

Example 26: 1-[5-(5-Chloro-thiophen-2-yl)-isoxazol-3-ylmethyl]-5-(3-hydroxy-azetidine-1-carbonyl)-1H-benzoimidazole-2-carboxylic acid (1-isopropyl-piperidin-4-yl)-amide

1-[5-(5-Chloro-thiophen-2-yl)-isoxazol-3-ylmethyl]-5-(3-hydroxy-azetidine-1-carbonyl)- 1H-benzoimidazole-2-carboxylic acid (1-isopropyl-piperidin-4-yl)-amide was prepared by a procedure according to example 22 starting from 50 mg (0.08 mmol) 1-[5-(5-chloro-thiophen-2-yl)-isoxazol-3-ylmethyl]-2-(1-isopropyl-piperidin-4-ylcarbamoyl)-1H-benzoimidazole-5-carboxylic acid and 6.7 mg (0.09 mmol) 3-hydroxy-acetidine. The title compound was obtained as its hydroformiate in form of a white amorphous solid.
Yield: 37 mg MS ($ES^+$): m/e = 583, chloro pattern.

Example 27: 1-[5-(5-Chloro-thiophen-2-yl)-isoxazol-3-ylmethyl]-2-(1-isopropyl-piperidin-4-ylcarbamoyl)-1H-benzoimidazole-5-carboxylic acid 1-ethoxycarbonyloxy-ethyl ester

1-[5-(5-Chloro-thiophen-2-yl)-isoxazol-3-ylmethyl]-2-(1-isopropyl-piperidin-4- ylcarbamoyl)-1H-benzoimidazole-5-carboxylic acid 1-ethoxycarbonyloxy-ethyl ester was prepared by adopting the procedure described for example 20 starting from 50 mg (0.08 mmol) 1-[5-(5-chloro-thiophen-2-yl)-isoxazol-3-ylmethyl]-2-(1-isopropyl-piperidin-4-ylcarbamoyl)-1H-benzoimidazole-5-carboxylic acid and 45 µl (0.32 mmol) 1-chloroethyl-ethylcarbonate. The title compound was obtained as its dihydrochloride. Yield: 57 mg MS ($ES^+$): m/e = 644, chloro pattern.

Example 28: 1-[5-(5-Chloro-thiophen-2-yl)-isoxazol-3-ylmethyl]-2-(1-isopropyl-piperidin-4-ylcarbamoyl)-1H-benzoimidazole-5-carboxylic acid 1-cyclohexyloxycarbonyloxy-ethyl ester

1-[5-(5-Chloro-thiophen-2-yl)-isoxazol-3-ylmethyl]-2-(1-isopropyl-piperidin-4-ylcarbamoyl)-1H-benzoimidazole-5-carboxylic acid 1-cyclohexyloxycarbonyloxy-ethyl ester was prepared by adopting the procedure described for example 20 starting from 50 mg (0.08 mmol) 1-[5-(5-chloro-thiophen-2-yl)-isoxazol-3-ylmethyl]-2-(1-isopropyl-piperidin-4- ylcarbamoyl)-1H-benzoimidazole-5-carboxylic acid and 61 µl (0.32 mmol) cyclohexyl-1-chloroethyl carbonate. The title compound was obtained as its dihydrochloride.
Yield: 59 mg MS ($ES^+$): m/e = 698, chloro pattern.

Example 29: a) 1-[5-(5-Chloro-thiophen-Z-yl)-isoxazol-3-ylmethyl]-5-(2-hydroxy-ethanesulfony!)-1H- benzoimidazole-2-carboxylic acid (1-isopropyl-piperidin-4-yl)-amide

b) 1-[5-(5-Chloro-thiophen-2-yl)-isoxazol-3-ylmethyl]-6-(2-hydroxy-ethanesulfonyl)-1H-benzoimidazole-2-carboxylic acid (1-isopropyl-piperidin-4-yl)-amide

(i) 2-(2-Trichloromethyl-1H-benzoimidazole-5-sulfonyl)-ethanol

500 mg (2.31 mmol) 3,4-diamino-benzene-sulfonylethan-2-ol were dissolved in 10 ml concentrated acetic acid. 0.4 ml (1.4 equiv.) methyl-2,2,2-trichloroacetimidate were added slowly and the resulting mixture was stirred at room temperature for 4 h. The mixture was diluted with 100 ml toluene and the solvent was removed under reduced pressure. The residue was rinsed with toluene, filtered and dried under vacuo to give a brown crystalline solid pure enough for all further reactions. Yield: 680 mg MS ($ES^+$): m/e = 345, chloro pattern.

(ii) 5-(2-Hydroxy-ethanesulfonyl)-1H-benzoimidazole-2-carboxylic acid (1-isopropyl-piperidin-4-yl)-amide

360 mg (1.05 mmol) 2-(2-Trichloromethyl-1H-benzoimidazole-5-sulfonyl)-ethanol were added to a mixture of 225.4 mg (1.05 equiv.) 1-isopropyl-piperidine-4-ylamine-dihydrochloride and 880 mg (10 equiv.) $NaHCO_3$ in 6 ml THF and 3 ml $H_2O$ and stirred vigorously for 2 h at room temperature. The reaction mixture was diluted with dichloromethane and washed with a saturated $NaHCO_3$-solution and brine. The organic layer was dried over $MgSO_4$ and concentrated. The obtained product was pure enough for further reactions.
Yield: 207 mg MS ($ES^+$): m/e = 395.

(iii) a) 1-[5-(5-Chloro-thiophen-2-yl)-isoxazol-3-ylmethyl]-5-(2-hydroxy-ethanesulfonyl)-1H-benzoimidazole-2-carboxylic acid (1-isopropyl-piperidin-4-yl)-amide

b) 1-[5-(5-Chloro-thiophen-2-yl)-isoxazol-3-ylmethyl]-6-(2-hydroxy-ethanesulfonyl)-1H-benzoimidazole-2-carboxylic acid (1-isopropyl-piperidin-4-yl)-amide

126.2 mg (0.32 mmol) 5-(2-Hydroxy-ethanesulfonyl)-1H-benzoimidazole-2-carboxylic acid (1-isopropyl-piperidin- 4-yl)-amide were dissolved in 10 ml DMF. Subsequently 48.6 mg (1.1 equiv.) $K_2CO_3$ and 89.0 mg (1.0 equiv.) 3-bromomethyl-5-(5-chloro-thiophen-2-yl)-isoxazole were added and the resulting mixture was stirred for 3 h at 55° C. The reaction mixture was diluted with toluene and washed twice with a saturated $NaHCO_3$-solution and once with brine, dried over anhydrous $MgSO_4$ and concentrated under reduced pressure. Preparative RP-HPLC ($CH_3CN/H_2O$ gradient + 0.05 % formic acid) gave a 6:4 mixture of both isomers described in the title. These isomers could be separated by NP-HPLC using a chiral stationary phase and a mixture of heptane, ethanol, methanol and diethyl amine as solvent. Structural assignment of both isomers was achieved by NOE-spectroscopy.
Yield of 1-[5-(5-chloro-thiophen-2-yl)-isoxazol-3-ylmethyl]-5-(2-hydroxy-ethanesulfonyl)-1H-benzo-imidazole-2-carboxylic acid (1-isopropyl-piperidin-4-yl)-amide: 79 mg
MS ($ES^+$): m/e =591, chloro pattern.
Yield of 1-[5-(5-Chloro-thiophen-2-yl)-isoxazol-3-ylmethyl]-6-(2-hydroxy-ethanesulfonyl)-1H-benzo-imidazole-2-carboxylic acid (1-isopropyl-piperidin-4-yl)-amide: 59 mg
MS ($ES^+$): m/e =591, chloro pattern.

Example 30:1-[(5-Chloro-pyridin-2-ylcarbamoyl)-methyl]-1H-benzoimidazole-2,4-dicarboxylic acid 4- [(2-hydroxy-ethyl)-amide] 2-[(1-isopropyl-piperidin-4-yl)-amide]

1-[(5-Chloro-pyridin-2-ylcarbamoyl)-methyl]-1H-benzoimidazole-2,4-dicarboxylic acid 4- [(2-hydroxy-ethyl)-amide] 2-[(1-isopropyl-piperidin-4-yl)-amide] was prepared by a procedure according to example 22 starting from 40 mg (0.08 mmol) 1-[(5-chloro-pyridin-2-ylcarbamoyl)-methyl]- 2-(1-isopropyl-piperidin-4- ylcarbamoyl)-1H-benzoimidazole-4-carboxylic acid and 5.4 mg (0.088 mmol) 2-aminoethanol. The title compound was obtained as its hydroformiate in form of a white amorphous solid. Yield: 34 mg MS ($ES^+$): m/e = 542, chloro pattern.

Example 31: 1-[(5-Chloro-pyridin-2-ylcarbamoyl)-methyl]-1H-benzoimidazole-2,4-dicarboxylic acid 4- [(2-hydroxy-ethyl)-methyl-amide] 2-[(1-isopropyl-piperidin-4-yl)-amide]

1-[(5-Chloro-pyridin-2-ylcarbamoyl)-methyl]-1H-benzoimidazole-2,4-dicarboxylic acid 4- [(2-hydroxy-ethyl)-methyl-amide] 2-[(1-isopropyl-piperidin-4-yl)-amide] was prepared by a procedure according to example 22 starting from 50 mg (0.10 mmol) 1-[(5-chloro-pyridin-2-ylcarbamoyl)-methyl]- 2-(1-isopropyl-piperidin-4-ylcarbamoyl)-1H-benzoimidazole-4-carboxylic acid and 8.3 mg (0.11 mmol) *N*-methyl-2-amino-ethanol. The title compound was obtained as its hydroformiate in form of a white amorphous solid.
Yield: 30 mg MS ($ES^+$): m/e = 556, chloro pattern.

Example 32: 1-[(5-Chloro-pyridin-2-ylcarbamoyl)-methyl]-4-(3-hydroxy-azetidine-1-carbonyl)-1H-benzoimidazole-2-carboxylic acid (1-isopropyl-piperidin-4-yl)-amide

1-[(5-Chloro-pyridin-2-ylcarbamoyl)-methyl]-4-(3-hydroxy-azetidine-1-carbonyl)-1H-benzoimidazole-2-carboxylic acid (1-isopropyl-piperidin-4-yl)-amide was prepared by a procedure according to example 22 starting from 50 mg (0.10 mmol) 1-[(5-chloro-pyridin-2-ylcarbamoyl)-methyl]- 2-(1-isopropyl-piperidin-4-ylcarbamoyl)-1H-benzoimidazole-4-carboxylic acid and 8.1 mg (0.11 mmol) 3-hydroxy-acetidine. The title compound was obtained as its hydroformiate in form of a white amorphous solid.
Yield: 28 mg MS ($ES^+$): m/e = 554, chloro pattern.

Example 33: 1-[(5-Chloro-pyridin-2-ylcarbamoyl)-methyl]-2-(1-isopropyl-piperidin-4-ylcarbamoyl)-1H-benzoimidazole-4-carboxylic acid 1-ethoxycarbonyloxy-ethyl ester

1-[(5-Chloro-pyridin-2-ylcarbamoyl)-methyl]-2-(1-isopropyl-piperidin-4-ylcarbamoyl)-1H-benzoimidazole-4-carboxylic acid 1-ethoxycarbonyloxy-ethyl ester was prepared by a procedure according to example 20 starting from 50 mg (0.10 mmol) 1-[(5-chloro-pyridin-2-ylcarbamoyl)-methyl]- 2-(1-isopropyl-piperidin-4-ylcarbamoyl)-1H-benzoimidazole-4-carboxylic acid and 61.2 mg (0.401 mmol) 1-chloroethyl-ethylcarbonate. The title compound was obtained as its hydroformiate in form of a white amorphous solid.
Yield: 38 mg MS ($ES^+$): m/e = 615, chloro pattern.

Example 34: 1-[(5-Chloro-pyridin-2-ylcarbamoyl)-methyl]-2-(1-isopropyl-piperidin-4-ylcarbamoyl)-1H-benzoimidazole-4-carboxylic acid 1-cyclohexyloxycarbonyloxy-ethyl ester

1-[(5-Chloro-pyridin-2-ylcarbamoyl)-methyl]-2-(1-isopropyl-piperidin-4-ylcarbamoyl)-1H-benzoimidazole-4-carboxylic acid 1-cyclohexyloxycarbonyloxy-ethyl ester was prepared by a procedure according to example 20 starting from 50 mg (0.10 mmol) 1-[(5-chloro-pyridin-2-ylcarbamoyl)-methyl]- 2-(1-isopropyl-piperidin-4-ylcarbamoyl)-1H-benzoimidazole-4-carboxylic acid and 82.8 mg (0.401 mmol) cyclohexyl-1-chloroethyl carbonate. The title compound was obtained as its hydroformiate in form of a white amorphous solid.
Yield: 46 mg MS ($ES^+$): m/e = 669, chloro pattern.

Example 35: 1-[(5-Chloro-pyridin-2-ylcarbamoyl)-methyl]-1H-benzoimidazole-2-carboxylic acid [4-(3- oxo-morpholin-4-yl)-phenyl]-amide

(i) 4-(4-Nitro-phenyl)-morpholine

A mixture of 24.5 g morpholine and 13.3 g 1-Fluoro-4-nitro-benzene in 30 ml DMSO was heated to 100°C for 4 h. This solution was poured on to 300 ml of water and the resulting precipitate was collected by filtration to yield a bright yellow crystalline product, which was dried in vacuo.
Yield: 19.7g.

(ii) 4-(4-Nitro-phenyl)-morpholin-3-one

To a solution of 10 g 4-(4-Nitro-phenyl)-morpholine in 200 ml DCM, 32 g Benzyl-triethyl-ammonium chloride and 22.7 g potassium permanganate (325 mesh) were cautiously added at RT. After stirring for 1H at RT the reaction mixture was heated to reflux for 10 h. Then a solution of 95 g $Na_2SO_3$ in 450 ml water were added under ice cooling and vigourous stirring. The mixture was filtered trough a pad of celite and the filtrate was concentrated under reduced pressure. The yellow solid was stirred with 250 ml water and the precipitated product was collected by filtration. This crude product was purified by chromatography on silica gel eluting with a gradient of DCM/MeOH 100%->50%. The

fractions containing the product were combined and the solvent evaporated under reduced pressure. Yield: 2.6 g.

(iii) 4-(4-Amino-phenyl)-morpholin-3-one

To a solution of 2.6 g 4-(4-Nitro-phenyl)-morpholin-3-one in 350 ml ethyl acetate and 17 ml ethanol, 13.2 g $SnCl_2$ dihydrate were added and the reaction mixture was heated to reflux for 2 h. Then after cooling to RT the mixture was stirred for 16 h. The precipitated product was collected by filtration and was pure enough for the next reaction step. Yield: 2.07 g.

(iv) 1H-Benzoimidazole-2-carboxylic acid [4-(3-oxo-morpholin-4-yl)-phenyl]-amide

To a solution of 100 mg 1H-Benzoimidazole-2-carboxylic acid and 118 mg 4-(4-Amino-phenyl)-morpholin-3-one in 2 ml DCM, 157 mg BOP-Cl and 0.3 ml $NEt_3$ were added and the mixture was stirred for 16 h at RT. Then, after the reaction mixture was diluted with 20 ml water, the precipitated product was collected by filtration. The crude product was subjected to the next reaction step without further purification. Yield: 122 mg.

(V) 1-[(5-Chloro-pyridin-2-ylcarbamoyl)-methyl]-1H-benzoimidazole-2-carboxylic acid [4-(3-oxo-morpholin-4-yl)-phenyl]-amide

To a solution of 50 mg 1H-Benzoimidazole-2-carboxylic acid [4-(3-oxo-morpholin-4-yl)-phenyl]-amide in 2 ml DMF, 49 mg $Cs_2CO_3$ and 37 mg 2-Bromo-N-(5-chloro-pyridin-2-yl)-acetamide were added at RT and the reaction mixture was stirred for 2 h. Then additional 20 mg $Cs_2CO_3$ and 30 mg 2-Bromo-N-(5-chloro-pyridin-2-yl)-acetamide were added at RT and the reaction mixture was stirred for additional 2 h. The reaction mixture was diluted with 20 ml water and the precipitated product was collected by filtration. The product was taken-up in 3 ml diluted HCl and lyophilized to yield a white solid. The product was obtained as its hydrochloride.
Yield: 128 mg MS ($ES^+$): m/e = 505, chloro pattern.

Example 36: 1-[(5-Chloro-pyridin-2-ylcarbamoyl)-methyl]-2-[4-(3-oxo-morpholin-4-yl)-phenylcarbamoyl]- 1H-benzoimidazole-4-carboxylic acid methyl ester

(i) 2-[4-(3-Oxo-morpholin-4-yl)-phenylcarbamoyl]-1H-benzoimidazole-4-carboxylic acid methyl ester

A solution of 69.4 mg (0.236mmol) methyl-2-trichloromethyl-1H-benzoimidazole-4-carboxylate in 2 ml THF were slowly added to a mixture containing 50 mg (0.26 mmol) 4-(4-amino-phenyl)-morpholin-3-one, 220 mg (2.62 mmol) $NaHCO_3$, 8 ml THF and 3 ml $H_2O$. The mixture was stirred vigorously for 3 h at room temperature, diluted with 50 ml $CH_2Cl_2$ and washed with 30 ml of a saturated $NaHCO_3$-solution. The aqueous solution was extracted with 50 ml $CH_2Cl_2$ and the combined organic layers were dried over $MgSO_4$ and concentrated under reduced pressure to give a brown solid, which was pure enough for the following reactions.
Yield: 72 mg MS (ES+): m/e = 395.

(ii) 1-[(5-Chloro-pyridin-2-ylcarbamoyl)-methyl]-2-[4-(3-oxo-morpholin-4-yl)-phenylcarbamoyl]-1H-benzoimidazole-4-carboxylic acid methyl ester

72.0 mg (0.183 mmol) 2-[4-(3-Oxo-morpholin-4-yl)-phenylcarbamoyl]-1H-benzoimidazole-4-carboxylic acid methyl ester were dissolved in 7 ml DMF. Sequentially 37.8 mg (0.274 mmol) $K_2CO_3$ and 54.6 mg (0.219 mmol) 2-bromo-*N*-(5-chloro-pyridin-2-yl-)-acetamide were added and the resulting mixture was stirred for 4h at 80°. The mixture was diluted with 200 ml toluene and washed with 50 ml of a saturated $NaHCO_3$-solution. The product was not completely soluble in toluene, so ethyl acetate had to be added. The organic layer was washed with brine, dried over anhydrous $MgSO_4$ and concentrated under reduced pressure. The residue was purified by preparative HPLC ($CH_3CN/H_2O$ gradient + 0.05 % formic acid) to give 1-[(5-chloro-pyridin-2-ylcarbamoyl)-methyl]-2-[4-(3-oxo-morpholin-4-yl)-phenylcarbamoyl]- 1H-benzoimidazole-4-carboxylic acid methyl ester as a light brown amorphous solid.
Yield: 46 mg MS ($ES^+$): m/e = 563, chloro pattern.

Pharmacological testing

The ability of the compounds of the formula I to inhibit factor Xa or factor VIIa or other enzymes like thrombin, plasmin, or trypsin can be assessed by determining the concentration of the compound of the formula I that inhibits enzyme activity by 50 %, i. e. the IC50 value, which was related to the inhibition constant Ki. Purified enzymes were

used in chromogenic assays. The concentration of inhibitor that causes a 50 % decrease in the rate of substrate hydrolysis was determined by linear regression after plotting the relative rates of hydrolysis (compared to the uninhibited control) versus the log of the concentration of the compound of formula I. For calculating the inhibition constant Ki, the IC50 value was corrected for competition with substrate using the formula

$$Ki = IC50 / \{1 + (\text{substrate concentration} / Km)\}$$

wherein Km is the Michaelis-Menten constant (Chen and Prusoff, Biochem. Pharmacol. 22 (1973) 3099-3108; I. H. Segal, Enzyme Kinetics, 1975, John Wiley & Sons, New York, 100-125; which were incorporated herein by reference).

a) Factor Xa Assay

In the assay for determining the inhibition of factor Xa activity TBS-PEG buffer (50 mM Tris-HCl, pH 7.8, 200 mM NaCl, 0.05 % (w/v) PEG-8000, 0.02 % (w/v) NaN3) was used. The IC50 was determined by combining in appropriate wells of a Costar half-area microtiter plate 25 μl human factor Xa (Enzyme Research Laboratories, Inc.; South Bend, Indiana) in TBS-PEG; 40 μl 10 % (v/v) DMSO in TBS-PEG (uninhibited control) or various concentrations of the compound to be tested diluted in 10 % (v/v) DMSO in TBS-PEG; and substrate S-2765 (N(α)-benzyloxycarbonyl-D-Arg-Gly-L-Arg-p-nitroanilide; Kabi Pharmacia, Inc.; Franklin, Ohio) in TBS-PEG.
The assay was performed by pre-incubating the compound of formula I plus enzyme for 10 min. Then the assay was initiated by adding substrate to obtain a final volume of 100 μl. The initial velocity of chromogenic substrate hydrolysis was measured by the change in absorbance at 405 nm using a Bio-tek Instruments kinetic plate reader (Ceres UV900HDi) at 25 °C during the linear portion of the time course (usually 1.5 min after addition of substrate). The enzyme concentration was 0.5 nM and substrate concentration was 140 μM.

b) Factor VIIa Assay

The inhibitory activity towards factor VIIa/tissue factor activity was determined using a chromogenic assay essentially as described previously (J. A. Ostrem et al., Biochemistry 37 (1998) 1053-1059 which was incorporated herein by reference). Kinetic assays were conducted at 25 °C in half-area microtiter plates (Costar Corp., Cambridge, Massachusetts) using a kinetic plate reader (Molecular Devices Spectramax 250). A typical assay consisted of 25 μl human factor VIIa and TF (5 nM and 10 nM, respective final concentration) combined with 40 μl of inhibitor dilutions in 10% DMSO/TBS-PEG buffer (50 mM Tris, 15 mM NaCl, 5 mM $CaCl_2$, 0.05 % PEG 8000, pH 8.15). Following a 15 minutes preincubation period, the assay was initiated by the addition of 35 μl of the chromogenic substrate S-2288 (D-Ile-Pro-Arg-p-nitroanilide, Pharmacia Hepar Inc., 500 μM final concentration). The results (inhibition constants Ki (FXa) for inhibition of factor Xa) are shown in Table 1.

Table1:

| Example | Ki(FXa) [μM] | Example | Ki(FXa) [μM] | Example | Ki(FXa) [μM] |
|---|---|---|---|---|---|
| 1 | 0,0007 | 13 | 0,2990 | 25 | 0,0650 |
| 2 | 0,0031 | 14 | 2,3900 | 26 | 0,0185 |
| 3 | 0,0014 | 15 | 0,0115 | 27 | 0,0125 |
| 4 | 0,0592 | 16 | 0,0110 | 28 | 0,0480 |
| 5a | 0,0025 | 17a | 0,0195 | 29a | 0,0255 |
| 5b | 0,1750 | 17b | 0,3710 | 29b | 0,0115 |
| 6 | 0,0030 | 18 | 0,0225 | 30 | 0,0090 |
| 7 | 0,0165 | 19 | 0,0235 | 31 | 0,0155 |
| 9 | 0,0940 | 20 | 0,0085 | 32 | 0,0105 |
| 10 | 1,357 | 21 | 0,0310 | 33 | 0,0125 |
| 11a | 0,004 | 22 | 0,0007 | 34 | 0,0195 |
| 11b | 0,001 | 23 | 0,0080 | 35 | 0,041 |

Table1: (continued)

| Example | Ki(FXa) [µM] | Example | Ki(FXa) [µM] | Example | Ki(FXa) [µM] |
|---|---|---|---|---|---|
| 12 | 0,0030 | 24 | 0,0260 | 36 | 0,0315 |

## Claims

**1.** A compound of the formula I

(I) 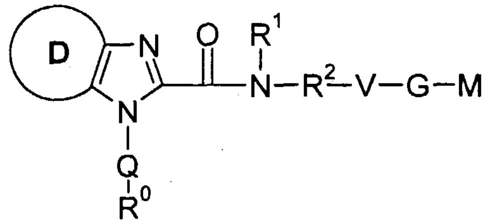

wherein

$R^0$ is 1) a monocyclic or bicyclic 6- to 14-membered aryl, wherein aryl is mono-, di- or trisubstituted independently of one another by R8,
2) a monocyclic or bicyclic 4- to 15-membered heterocyclyl out of the group pyridinyl, pyrimidinyl, indolyl, isoindolyl, indazolyl, phthalazinyl, quinolyl, isoquinolyl, benzothiophen, quinazolinyl and phenylpyridyl, wherein said heterocyclyl is unsubstituted or mono-, di- or trisubstituted independently of one another by R8, or
3) a monocyclic or bicyclic 4- to 15-membered heterocyclyl, containing one, two, three or four heteroatoms chosen from nitrogen, sulfur or oxygen,

wherein said heterocyclyl is unsubstituted or mono-, di- or trisubstituted independently of one another by R8, and which is additionally substituted by a monocyclic or bicyclic 4- to 15-membered heterocyclyl, containing one, two, three or four heteroatoms chosen from nitrogen, sulfur or oxygen,
wherein heterocyclyl is unsubstituted or mono-, di- or trisubstituted independently of one another by R8,

R8 is 1) halogen,
2) $-NO_2$,
3) -CN,
4) $-C(O)-NH_2$,
5) -OH,
6) $-NH_2$,
7) $-O-CF_3$
8) a monocyclic or bicyclic 6- to 14-membered aryl, wherein aryl is mono-, di- or trisubstituted independently of one another by halogen or $-O-(C_1-C_8)$-alkyl,
9) $-(C_1-C_8)$-alkyl, wherein alkyl is unsubstituted or mono-, di- or trisubstituted independently of one another by halogen, $NH_2$, -OH or a methoxy residue,
10) $-O-(C_1-C_8)$-alkyl, wherein alkyl is unsubstituted or mono-, di- or trisubstituted independently of one another by halogen, $NH_2$, -OH or a methoxy residue,
11) $-SO_2-CH_3$ or
12) $-SO_2-CF_3$,

provided that R8 is at least one halogen, $-C(O)-NH_2$ or$-O-(C_1-C_8)$-alkyl residue, if $R^0$ is a monocyclic or bicyclic 6- to 14-membered aryl,
the substructure

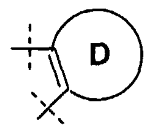

in formulae I is

a 4-to 8 membered saturated, partially unsaturated or aromatic cyclic group containing zero, 1,2, 3 or 4 heteroatoms chosen from nitrogen, sulfur or oxygen and is unsubstituted or substituted 1, 2, 3, 4, 5 or 6 times by R3,

Q is a direct bond, $-(C_0-C_2)$-alkylene-C(O)-$NR^{10}$-, -$NR^{10}$-C(O)-$NR^{10}$-, -$NR^{10}$-C(O)-, -$SO_2$-, $-(C_1-C_6)$-alkylene, $-(CH_2)_m$-$NR^{10}$-C(O)-$NR^{10}$-$(CH_2)_n$-, $-(CH_2)_m$-$NR^{10}$-C(O)-$(CH_2)_n$-, $-(CH_2)_m$-S-$(CH_2)_n$-, $-(CH_2)_m$-C(O)-$(CH_2)_n$-, $-(CH_2)_m$-$SO_2$-$NR^{10}$-$(CH_2)_n$-, $-(CH_2)_m$-$NR^{10}$-$SO_2$-$(CH_2)_n$-, $-(CH_2)_m$-$NR^{10}$-$SO_2$-$NR^{10}$-$(CH_2)_n$-, $-(CH_2)_m$-CH (OH)-$(CH_2)_n$-, $-(CH_2)_m$-O-C(O)-$NR^{10}$-$(CH_2)_n$-, $-(C_2-C_3)$-alkylene-O-$(C_0-C_3)$-alkylene-, $-(C_2-C_3)$-alkylene-S (O)-, $-(C_2-C_3)$-alkylene-S$(O)_2$-, $-(CH_2)_m$-$NR_{10}$-C(O)-O-$(CH_2)_n$-, $-(C_2-C_3)$-alkylene-S$(O)_2$-NH-$(R^{10})$-, $-(C_2-C_3)$-alkylene-N$(R^{10})$- or $-(C_0-C_3)$-alkylene-C(O)-O-,

wherein $R^{10}$ is as defined below, and wherein n and m are independently of one another identical or different and are the integers zero, 1, 2, 3, 4, 5 or 6, wherein the alkylene residues which are formed by $-(CH_2)_m$- or $-(CH_2)_n$- are unsubstituted or mono-, di- or trisubstituted independently of one another by halogen, -$NH_2$ or -OH; or $-(C_3-C_6)$-cycloalkylen, wherein cycloalkylen is unsubstituted or mono-, di- or trisubstituted independently of one another by halogen, -$NH_2$ or-OH;

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-$R^{10}$, a monocyclic or bicyclic 6- to 14-membered aryl, wherein aryl is mono-, di- or trisubstituted independently of one another by R8, wherein R8 is as defined above; a monocyclic or bicyclic 4- to 15-membered heterocyclyl, containing one, two, three or four heteroatoms chosen from nitrogen, sulfur or oxygen; $-(C_1-C_3)$-perfluoroalkylene, $-(C_1-C_3)$-alkylene-S(O)-$(C_1-C_4)$-alkyl, $-(C_1-C_3)$-alkylene-S$(O)_2$-$(C_1-C_3)$-alkyl, $-(C_1-C_3)$-alkylene-S$(O)_2$-N$(R^{4'})$-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a 3- to 7- membered cyclic residue, containing up to 1, 2, 3 or 4 heteroatoms chosen from nitrogen, sulfur or oxygen, wherein said cyclic residue is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or$-(C_1-C_4)$-alkylene, or

$R^1$ and $R^3$ together with the atoms to which they are bonded can form a 6- to 8-membered cyclic group, containing 1, 2, 3 or 4 heteroatoms chosen from nitrogen, sulfur or oxygen, wherein said cyclic group is unsubstituted or mono-, di- or trisubstituted independently of one another by R14, or

$R^1$-N-$R^2$-V can form a 4- to 8-membered cyclic group, containing 1, 2, 3 or 4 heteroatoms chosen from nitrogen, sulfur or oxygen, wherein said cyclic group is unsubstituted or mono-, di- or trisubstituted independently of one another by R14,

R14 is halogen, -OH, =O, $-(C_1-C_8)$-alkyl, $-(C_1-C_4)$-alkoxy, -$NO_2$, -C(O)-OH, -CN, -$NH_2$, -C(O)-O-$(C_1-C_4)$-alkyl, $-(C_1-C_8)$-alkylsulfonyl, -$SO_2$-N$(R^{18})$-$R^{21}$, -C(O)-NH-$(C_1-C_8)$-alkyl, -C(O)-N-$[(C_1-C_8)\text{-alkyl}]_2$, -$NR^{18}$-C(O)-NH-$(C_1-C_8)$-alkyl, -C(O)-$NH_2$, -S-$R^{18}$, or -$NR^{18}$-C(O)-NH-$[(C_1-C_8)\text{-alkyl}]_2$,

wherein $R^{18}$ and $R^{21}$ are independently from each other hydrogen atom, $-(C_1-C_3)$-perfluoroalkyl or$-(C_1-C_6)$-alkyl,

V is 1) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14,

2) a 6- to 14-membered aryl, wherein aryl is unsubstituted or mono-, di- or trisubstituted independently of one another by R14, or

3) a monocyclic or bicyclic 4- to 15-membered heterocyclyl, wherein said heterocyclyl is unsubstituted or mono-, di- or trisubstituted independently of one another by R14,

G is a direct bond, $-(CH_2)_m$-$NR^{10}$-$SO_2$-$NR^{10}$-$(CH_2)_n$-, $-(CH_2)_m$-CH(OH)-$(CH_2)_n$-, $-(CH_2)_m$-, $-(CH_2)_m$-O-$(CH_2)_n$-, $-(CH_2)_m$-C(O)-$NR^{10}$-$(CH_2)_n$-, $-(CH_2)$-$SO_2$-$(CH_2)_n$-, $-(CH_2)_m$-$NR^{10}$-C(O)-$NR^{10}$-$(CH_2)_n$-,

$-(CH_2)_m-NR^{10}-C(O)-(CH_2)_n-(CH_2)_m-C(O)-(CH_2)_n-$, $-(CH_2)-S-(CH_2)_n-$, $-(CH_2)_m-SO_2-NR^{10}-(CH_2)_n-$, $-(CH_2)_m-NR^{10}-SO_2-(CH_2)_n-$, $-(CH_2)_m-NR^{10}-$, $-(CH_2)_m-O-C(O)-NR^{10}-(CH_2)_n-$ or $-(CH_2)_m-NR^{10}-C(O)-O-(CH_2)_n-$,

n and m are independently of one another identical or different and are the integers zero, 1, 2, 3, 4, 5 or 6,

M is
1) a hydrogen atom,
2) $-(C_1-C_8)$-alkyl, wherein alkyl is unsubstituted or mono-, di- or trisubstituted independently of one another by R14,
3) -C(O)-N(R11)-R12,
4) $-(CH_2)_m-NR^{10}$,
5) a 6- to 14-membered aryl, wherein aryl is unsubstituted or mono-, di- or trisubstituted independently of one another by R14,
6) a monocyclic or bicyclic 4- to 15-membered heterocyclyl, wherein heterocyclyl is unsubstituted or mono-, di- or trisubstituted independently of one another by R14,
7) $-(C_3-C_8)$-cycloalkyl, wherein said cycloalkyl is unsubstituted or mono-, di- or trisubstituted independently of one another by R14, or
8) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14, wherein R14 is defined above,

R3 is
1) hydrogen atom,
2) halogen,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_4)$-alkylene-O-R19, wherein R19 is

a) hydrogen atom,
b) $-(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) $-CF_3$,
e) $-CHF_2$,

7) $-NO_2$,
8) -CN,
9) $-SO_s-R^{11}$, wherein s is 1 or 2,
10) $-SO_t-N(R^{11})-R^{12}$, wherein t is 1 or 2,
11) $-(C_0-C_4)$-alkylene-C(O)-$R^{11}$,
12) $-(C_0-C_4)$-alkylene-C(O)-O-$R^{11}$,
13) $-(C_0-C_4)$-alkylene-C(O)-N($R^{11}$)-$R^{12}$,
14) $-(C_0-C_4)$-alkylene-N($R^{11}$)-$R^{12}$,
15) $-NR^{10}-SO_2R^{10}$,
16) $-S-R^{10}$,
17) $-(C_0-C_2)$alkylene-C(O)-O-$(C_2-C_4)$-alkylene-O-C(O)-$(C_1-C_4)$-alkyl,
18) -C(O)-O-C(R15, R16)-O-C(O)-R17,
19) $-(C_0-C_2)$alkylene-C(O)-O-$(C_2-C_4)$-alkylene-O-C(O)-O-$(C_1-C_6)$-alkyl,
20) -C(O)-O- C(R15, R16)-O-C(O)-O-R17,
21) $-(C_0-C_4)$-alkylene-$(C_6-C_{14})$-aryl, wherein aryl is mono-, di- or trisubstituted independently of one another by R13,
22) $-(C_0-C_4)$-alkylene-$(C_4-C_{15})$-heterocyclyl, wherein heterocyclyl is unsubstituted or mono-, di- or trisubstituted independently of one another by R13
23) $-(C_0-C_4)$-alkylene-$(C_3-C_8)$-cycloalkyl, wherein cycloalkyl is unsubstituted or mono-, di- or trisubstituted independently of one another by R13,
24) $-(C_0-C_4)$-alkylene-het, wherein het is unsubstituted or mono-, di- or trisubstituted independently of one another by R13, or

25) a residue from the following list

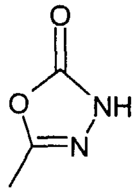 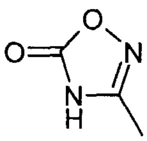 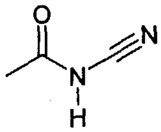 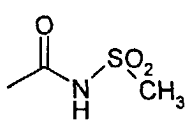 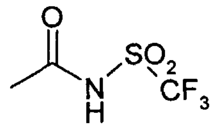 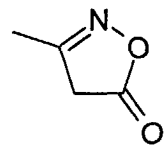

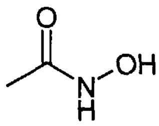 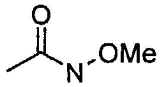 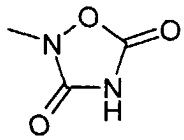 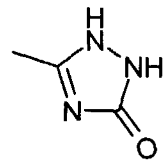 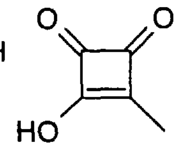

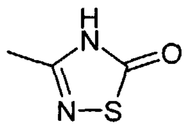 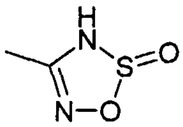 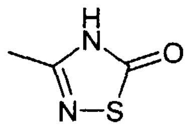 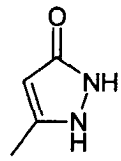 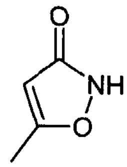 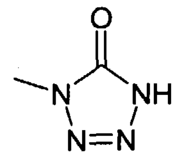

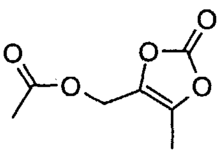 and 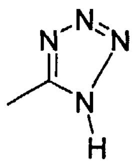

wherein Me is methyl, or

if two -OR19 residues are attached to adjacent atoms they can form together with the atoms which they are attached to a 5- or 6- membered ring, which is unsubstituted or substituted one, two, three or four times by R13,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_3$-$C_8$)-cycloalkyl,
4) -$SO_t$-$R^{10}$, wherein t is 1 or 2,
5) -($C_0$-$C_6$)-alkyl-($C_6$-$C_{14}$)-aryl, wherein alkyl and aryl independently from one another are unsubstituted or mono-, di- or trisubstituted by R13,
6) -($C_1$-$C_3$)-perfluoroalkyl,
7) -O-$R^{17}$, or
8) -($C_0$-$C_6$)-alkyl-($C_4$-$C_{15}$)-heterocyclyl, wherein alkyl and heterocyclyl independently from one another are unsubstituted or mono-, di- or trisubstituted by R13, or

R11 and R12 together with the nitrogen atom to which they are bonded can form a 4-to 8-membered monocyclic heterocyclic ring which in addition to the nitrogen atom can contain one or two identical or different ring heteroatoms chosen from oxygen, sulfur and nitrogen; wherein said heterocyclic ring is unsubstituted or mono-, di- or trisubstituted independently of one another by R13,

R13 is halogen, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_3$-$C_8$)-cycloalkyl, -($C_0$-$C_3$)-alkylene-O-$R^{10}$, -Si-$(CH_3)_3$, -N($R^{10}$)-$S(O)_u$-$R^{10}$, wherein u is 1 or 2, -S-$R^{10}$, -$SO_r$-$R^{10}$, wherein r is 1 or 2, -$S(O)_v$-N($R^{10}$)-$R^{20}$, wherein v is 1 or 2, -C(O)-$R^{10}$, -($C_1$-$C_8$)-alkyl, -($C_1$-$C_8$)-alkoxy, phenyl, phenyloxy-, -O-$CF_3$, -($C_0$-$C_4$)-alkyl-C(O)-O-C(R15, R16)-O-C(O)-R17, -($C_1$-$C_4$)-alkoxy-phenyl, -($C_0$-$C_4$)-alkyl-C(O)-O-C(R15, R16)-O-C(O)-O-R17, -($C_1$-$C_3$)-perfluoroalkyl, -O-R15, -NH-C(O)-NH-$R^{10}$, -NH-C(O)-O-$R^{10}$, or a residue from the following list

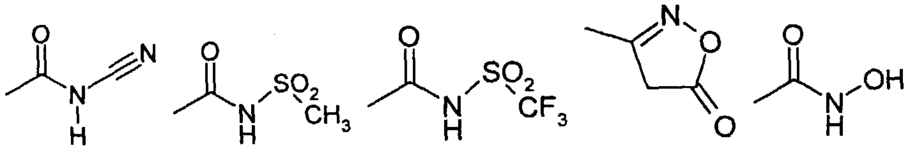

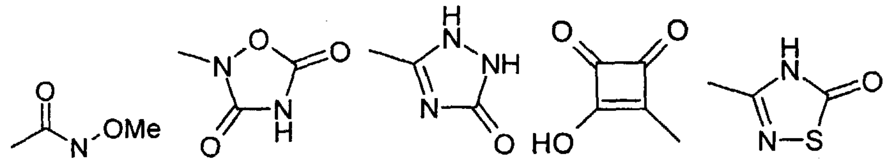

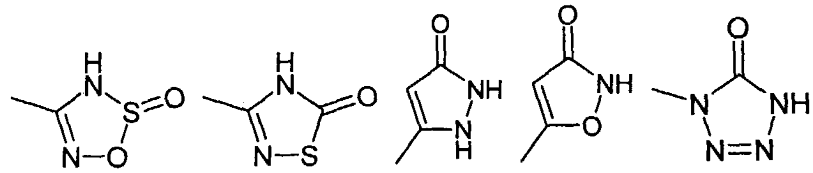

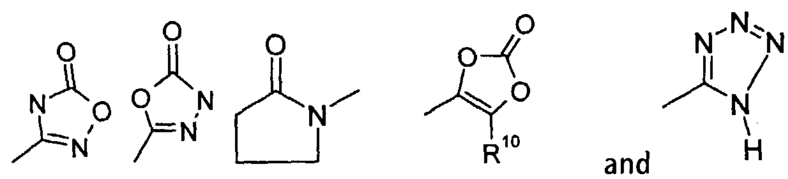

and ,

R10 and R20 are independently of one another hydrogen, $-(C_1-C_6)$-alkyl, $-(C_0-C_4)$-alkyl-OH, $-(C_0-C_4)$-alkyl-O-$(C_1-C_4)$-akyl or $-(C_1-C_3)$-perfluoroalkyl,

R15 and R16 are independently of one another hydrogen, $-(C_1-C_6)$-alkyl, or together with the carbon atom to which they are bonded they can form a 3- to 6 membered carbocyclic ring which is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by-OH, -O-$(C_1-C_4)$-alkyl or $R^{10}$,

in all its stereoisomeric forms and mixtures thereof in any ratio, and its physiologically tolerable salts.

**2.** A copound of formula I as claimed in claim 1, wherein

$R^0$ is 1) a monocyclic or bicyclic 6- to 14-membered aryl, wherein aryl is mono-, di- or trisubstituted independently of one another by R8,
2) a monocyclic or bicyclic 4- to 15-membered heterocyclyl out of the group benzothiophen, indazolyl, indolyl, isoindolyl, isoquinolyl, phenylpyridyl, phthalazinyl, pyridyl, pyridinyl, pyrimidinyl, quinazolinyl and quinolyl, wherein said heterocyclyl is unsubstituted or mono-, di- or trisubstituted independently of one another by R8, or
3) a monocyclic or bicyclic 4- to 15-membered heterocyclyl, containing one, two, three or four heteroatoms chosen from nitrogen, sulfur or oxygen,
wherein said heterocyclyl is unsubstituted or mono-, di- or trisubstituted independently of one another by R8, and which is additionally substituted by a monocyclic or bicyclic 4- to 15-membered heterocyclyl, containing one, two, three or four heteroatoms chosen from nitrogen, sulfur or oxygen,
wherein heterocyclyl is unsubstituted or mono-, di- or trisubstituted independently of one another by R8,

R8 is 1) halogen,
2) $-NO_2$,
3) -CN,
4) $-C(O)-NH_2$,
5) -OH,

6) $-NH_2$,
7) $-O-CF_3$
8) a monocyclic or bicyclic 6- to 14-membered aryl, wherein aryl is mono-, di- or trisubstituted independently of one another by halogen or-O-$(C_1-C_8)$-alkyl,
9) -$(C_1-C_8)$-alkyl, wherein alkyl is unsubstituted or mono-, di- or trisubstituted independently of one another by halogen, $NH_2$, -OH or a methoxy residue, or
10) -O-$(C_1-C_8)$-alkyl, wherein alkyl is unsubstituted or mono-, di- or trisubstituted independently of one another by halogen, $NH_2$, -OH or a methoxy residue,
11) $-SO_2-CH_3$ or
12) $-SO_2-CF_3$,

provided that R8 is at least one halogen, -C(O)-$NH_2$ or -O-$(C_1-C_8)$-alkyl residue, if $R^0$ is a monocyclic or bicyclic 6- to 14-membered aryl,
substructure D is a 5-to 6 membered saturated, partially unsaturated or aromatic cyclic group containing zero, 1, 2, 3 or 4 heteroatoms chosen from nitrogen, sulfur or oxygen and is substituted 1, 2, 3, 4, 5 or 6 times by R3,

Q is a direct bond, -$(C_0$ -$C_2)$-alkylene-C(O)-$NR^{10}$-, -$NR^{10}$-C(O)-$NR^{10}$-, -$NR^{10}$-C(O)-, $-SO_2-$, -$(C_1-C_6)$-alkylene, $-(CH_2)_m-NR^{10}-C(O)-NR^{10}-(CH_2)_n-$, $-(CH_2)_m-NR^{10}-C(O)-(CH_2)_n-$, $-(CH_2)_m-S-(CH_2)_n-$, $-(CH_2)_m-C(O)-(CH_2)_{n-}$ $-(CH_2)_m-SO_2-NR^{10}-(CH_2)_n-$, $-(CH_2)_m-NR^{10}-SO_2-(CH_2)_{n-}$ $-(CH_2)_n-NR^{10}-SO_2-NR^{10}-(CH_2)_m-$, $-(CH_2)_m-CH(OH)-(CH_2)n-$, $-(CH_2)_m-O-C(O)-NR^{10}-(CH_2)_n-$, -$(C_2-C_3)$-alkylene-O-$(C_2-C_3)$-alkylene-, -$(C_2-C_3)$-alkylene-S(O)-, -$(C_2-C_3)$-alkylene-$S(O)_2$-, $-(CH_2)_m-NR^{10}-C(O)-O-(CH_2)_n-$, $(C_2-C_3)$-alkylene-$S(O)_2$-NH-$(R^{10})$-, -$(C_2-C_3)$-alkylene-N$(R^{10})$- or -$(C_0-C_3)$-alkylene-C(O)-O-,
wherein $R^{10}$ is as defined below, and wherein n and m are independently of one another identical or different and are the integers zero, 1, 2, 3, 4, 5 or 6, wherein the alkylene residues which are formed by $-(CH_2)_m-$ or $-(CH_2)_n-$ are unsubstituted or mono-, di- or trisubstituted independently of one another by halogen, $-NH_2$ or -OH; or -$(C_3-C_6)$-cycloalkylen, wherein cycloalkylen is unsubstituted or mono-, di- or trisubstituted independently of one another by halogen, $-NH_2$ or-OH;

$R^1$ is a hydrogen atom, -$(C_1-C_4)$-alkyl, wherein alkyl is unsubstituted or substituted one to three times by R13; -$(C_1-C_3)$-alkylene-C(O)-NH-$R^0$, -$(C_1-C_3)$-alkylene-C(O)-O-R15, a monocyclic or bicyclic 6- to 14-membered aryl, wherein aryl is mono-, di- or trisubstituted independently of one another by R8, wherein R8 is as defined above; a monocyclic or bicyclic 4- to 15-membered heterocyclyl, containing one, two, three or four heteroatoms chosen from nitrogen, sulfur or oxygen; -$(C_1-C_3)$-perfluoroalkylene, -$(C_1-C_3)$-alkylene-S(O)-$(C_1-C_4)$-alkyl, -$(C_1-C_3)$-alkylene-$S(O)_2$-$(C_1-C_3)$-alkyl, -$(C_1-C_3)$-alkylene-$S(O)_2$-N$(R^{4'})$-$R^{5'}$, -$(C_1-C_3)$-alkylene-O-$(C_1-C_4)$-alkyl, -$(C_0-C_3)$-alkylene-$(C_3-C_8)$-cycloalkyl, or -$(C_0-C_3)$-alkylene-het, wherein het is unsubstituted or mono-, di- or trisubstituted independently of one another by R14,

$R^{4'}$and $R^{5'}$ are independent of one another are identical or different and are hydrogen atom or -$(C_1-C_4)$-alkyl,

$R^2$ is a direct bond or-$(C_1-C_4)$-alkylene, or

$R^1$ and R3 together with the atoms to which they are bonded can form a 6- to 8-membered cyclic group, containing up to 1, 2, 3 or 4 heteroatoms chosen from nitrogen, sulfur or oxygen, wherein said cyclic group is unsubstituted or mono-, di- or trisubstituted independently of one another by R14, or

$R^1$-N-$R^2$-V can form a 4- to 8-membered cyclic group, containing 1, 2, 3 or 4 heteroatoms chosen from nitrogen, sulfur or oxygen, wherein said cyclic group is unsubstituted or mono-, di- or trisubstituted independently of one another by R14,

R14 is halogen, -OH, =O, -$(C_1-C_8)$-alkyl, -$(C_1-C_4)$-alkoxy, $-NO_2$, -C(O)-OH, -CN, $-NH_2$, -C(O)-O-$(C_1-C_4)$-alkyl, -$(C_1-C_8)$-alkylsulfonyl, $-SO_2$-N$(R^{18})$-$R^{21}$, -C(O)-NH-$(C_1-C_8)$-alkyl, -C(O)-N-[$(C_1-C_8)$-alkyl$]_2$, -$NR^{18}$-C(O)-NH-$(C_1-C_8)$-alkyl, -C(O)-$NH_2$, -S-$R^{18}$, or -$NR^{18}$-C(O)-NH-[$(C_1-C_8)$-alkyl$]_2$,
wherein $R^{18}$ and $R^{21}$ are independently from each other hydrogen atom, -$(C_1-C_3)$-perfluoroalkyl or-$(C_1-C_6)$-alkyl,

V is
1) a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14,
2) a 6- to 14-membered aryl, wherein aryl is unsubstituted or mono-, di- or trisubstituted independently of one another by R14, or
3) a monocyclic or bicyclic 4- to 15-membered heterocyclyl, wherein said heterocyclyl is unsubstituted or mono-, di- or trisubstituted independently of one another by R14,

G is a direct bond, $-(CH_2)_m-NR^{10}-SO_2-NR^{10}-(CH_2)_n-$, $-(CH_2)_m-CH(OH)-(CH_2)_n-$, $-(CH_2)_m-$ ,$-(CH_2)_m-O-(CH_2)_n-$, $-(CH_2)_m-C(O)-NR^{10}$ $-(CH_2)_n-$, $-(CH_2)-SO_2-(CH_2)_n-$, $-(CH_2)_m-NR^{10}-C(O)-NR^{10}-(CH_2)_n-$, $-(CH_2)_m-NR^{10}-C(O)-(CH_2)_n-$,$(CH_2)_n-C(O)-(CH_2)_n-$, $-(CH_2)-S-(CH_2)_n-$, $-(CH_2)_m-SO_2-NR^{10}-(CH_2)_n-$, $-(CH_2)_n-NR^{10}-SO_2-(CH_2)_n-$, $-(CH_2)_m-NR^{10}-(CH_2)_m-O-C(O)-NR^{10}-(CH_2)_m-NR^{10}-C(O)-O-(CH_2)_n-$,

n and m are independently of one another identical or different and are the integers zero, 1, 2, 3, 4, 5 or 6,

M is
1) a hydrogen atom,
2) $-(C_1-C_8)$-alkyl, wherein alkyl is unsubstituted or mono-, di- or trisubstituted independently of one another by R14,
3) -C(O)-N(R11)-R12,
4) $-(CH_2)_m-NR^{10}$,
5) $-(C_6-C_{14})$-aryl, wherein aryl is unsubstituted or mono-, di- or trisubstituted independently of one another by R14,
6) $-(C_4-C_{15})$-heterocyclyl, wherein heterocyclyl is unsubstituted or mono-, di- or trisubstituted independently of one another by R14,
7) $-(C_3-C_8)$-cycloalkyl, wherein said cycloalkyl is unsubstituted or mono-, di- or trisubstituted independently of one another by R14, or
8) a 3- to 7-membered cyclic residue, containing up to 1, 2, 3 or 4 heteroatoms chosen from nitrogen, sulfur or oxygen, wherein said cyclic residue is unsubstituted or mono-, di- or trisubstituted independently of one another by R14, wherein R14 is defined above,

R3 is
1) hydrogen atom,
2) halogen,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_4)$-alkylene-O-R19, wherein R19 is

a) hydrogen atom,
b) $-(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) $-CF_3$,
e) $-CHF_2$,

7) $-NO_2$,
8) -CN,
9) $-SO_s-R^{11}$, wherein s is 1 or 2,
10) $-SO_t-N(R^{11})-R^{12}$, wherein t is 1 or 2,
11) $-(C_0-C_4)$-alkylene-C(O)-$R^{11}$,
12) $-(C_0-C_4)$-alkylene-C(O)-O-$R^{11}$,
13) $-(C_0-C_4)$-alkylene-C(O)-N($R^{11}$)-$R^{12}$,
14) $-(C_0-C_4)$-alkylene-N($R^{11}$)-$R^{12}$,
15) $-NR^{10}-SO_2-R^{10}$,
16) $-S-R^{10}$,
17) $-(C_0-C_2)$alkylene-C(O)-O-$(C_2-C_4)$-alkylene-O-C(O)-$(C_1-C_4)$-alkyl,
18) -C(O)-O-C(R15, R16)-O-C(O)-R17,
19) $-(C_0-C_2)$alkylene-C(O)-O-$(C_2-C_4)$-alkylene-O-C(O)-O-$(C_1-C_6)$-alkyl,
20) -C(O)-O- C(R15, R16)-O-C(O)-O-R17,
21) $-(C_0-C_4)$-alkylene-$(C_6-C_{14})$-aryl, wherein aryl is mono-, di- or trisubstituted independently of one another by R13,
22) $-(C_0-C_4)$-alkylene-$(C_4-C_{15})$-heterocyclyl, wherein heterocyclyl is unsubstituted or mono-, di- or trisubstituted independently of one another by R13
23) $-(C_0-C_4)$-alkylene-$(C_3-C_8)$-cycloalkyl, wherein cycloalkyl is unsubstituted or mono-, di- or trisubstituted independently of one another by R13,

24) -($C_0$-$C_4$)-alkylene-het, wherein het is unsubstituted or mono-, di- or trisubstituted independently of one another by R13, or
25) a residue from the following list

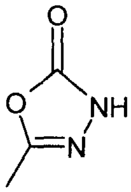 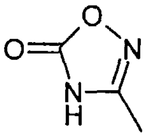 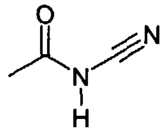 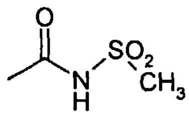 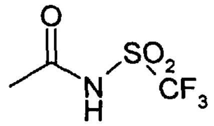 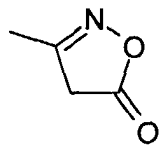

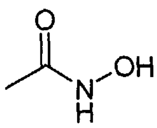 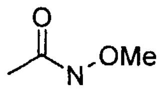 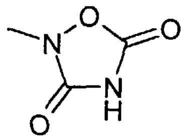 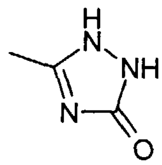 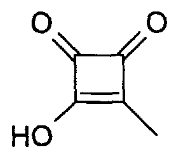

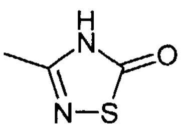 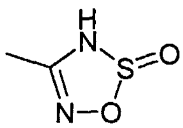 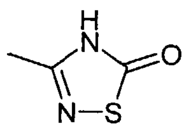 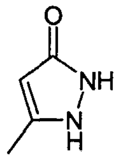 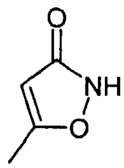 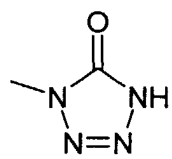

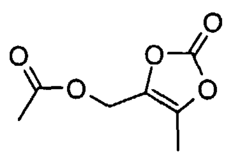 and 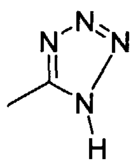

wherein Me is methyl, or
if two -OR19 residues are attached to adjacent atoms they can form together with the atoms which they are attached to a 5- or 6- membered ring, which is unsubstituted or substituted one, two, three or four times by R13,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_3$-$C_8$)-cycloalkyl,
4) -$SO_t$-$R^{10}$, wherein t is 1 or 2,
5) -($C_0$-$C_6$)-alkyl-($C_6$-$C_{14}$)-aryl, wherein alkyl and aryl independently from one another are unsubstituted or mono-, di- or trisubstituted by R13,
6) -($C_1$-$C_3$)-perfluoroalkyl,
7) -O-$R^{17}$, or
8) -($C_0$-$C_6$)-alkyl-($C_4$-$C_{15}$)-heterocyclyl, wherein alkyl and heterocyclyl

independently from one another are unsubstituted or mono-, di- or trisubstituted by R13, or

R11 and R12 together with the nitrogen atom to which they are bonded can form a 4- to 8-membered monocyclic heterocyclic ring which in addition to the nitrogen atom can contain one or two identical or different ring heteroatoms chosen from oxygen, sulfur and nitrogen; wherein said heterocyclic ring is unsubstituted or mono-, di- or trisubstituted independently of one another by R13,

R13 is halogen, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_3$-$C_8$)-cycloalkyl, -($C_0$-$C_3$)-alkylene-O-$R^{10}$, -Si-$(CH_3)_3$, -N($R^{10}$)-S$(O)_u$-$R^{10}$, wherein u is 1 or 2, -S-$R^{10}$, -$SO_r$-$R^{10}$, wherein r is 1 or 2, -S$(O)_v$-N($R^{10}$)-$R^{20}$, wherein v is 1 or 2, -C(O)-$R^{10}$, -($C_1$-$C_8$)-alkyl, -($C_1$-$C_8$)-alkoxy, phenyl, phenyloxy-, -O-$CF_3$, -($C_0$-$C_4$)-alkyl-C(O)-O-C(R15,

R16)-O-C(O)-R17, $-(C_1-C_4)$-alkoxy-phenyl, $-(C_0-C_4)$-alkyl-C(O)-O-C(R15, R16)-O-C(O)-O-R17, $-(C_1-C_3)$-perfluoroalkyl, -O-R15, -NH-C(O)-NH-$R^{10}$, -NH-C(O)-O-$R^{10}$, or a residue from the following list

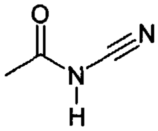
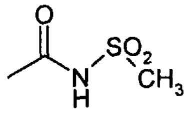
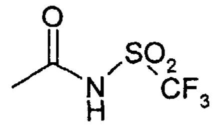
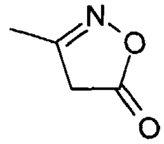
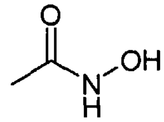
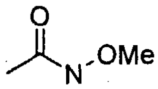
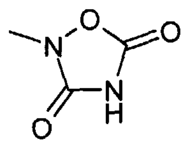
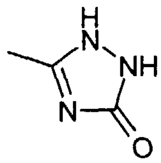
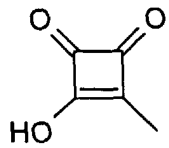
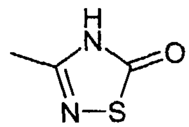
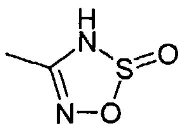
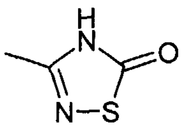
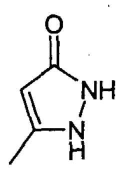
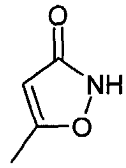
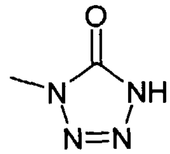
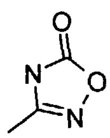
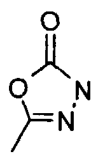
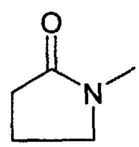
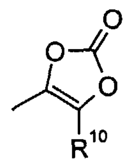
and
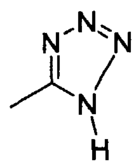
,

| | |
|---|---|
| $R^{10}$ and $R^{20}$ are | independently of one another hydrogen, $-(C_1-C_6)$-alkyl or $-(C_1-C_3)$-perfluoroalkyl, |
| R15 and R16 are | independently of one another hydrogen, $-(C_1-C_6)$-alkyl, or together with the carbon atom to which they are bonded they can form a 3- to 6 membered carbocyclic ring which is unsubstituted or substituted one to three times by $R^{10}$, and |
| R17 is | $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_8)$-alkyl-$(C_3-C_8)$-cycloalkyl, wherein said cycloalkyl ring is unsubstituted or substituted one, two or three times by-OH, -O-$(C_1-C_4)$-alkyl or $R^{10}$, |

in all its stereoisomeric forms and mixtures thereof in any ratio, and its physiologically tolerable salts

**3.** A compound of formula I as claimed in claims 1 or 2, wherein

$R^0$ is

1) a monocyclic or bicyclic 6- to 14-membered aryl out of the group phenyl, naphthyl, biphenylyl, anthryl or fluorenyl, wherein aryl is mono-, di- or trisubstituted independently of one another by R8,
2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, phenylpyridyl, phthalazinyl, pteridinyl, purinyl, pyridinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, wherein said heterocyclyl is unsubstituted or mono-, di- or trisubstituted independently of one another by R8, or
3) a heterocyclyl, wherein heterocyclyl is selected out of the group acridinyl, azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxa-zolinyl, dioxazolyl, dioxazinyl, 1,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl,

isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,2-oxazinyl, 1,3-oxazinyl, 1,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, 1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,4-triazolyl, 1,2,5-triazolyl, 1,3,4-triazolyl and xanthenyl,

wherein said heterocyclyl is unsubstituted or mono-, di- or trisubstituted independently of one another by R8, and
which is additionally substituted by a heterocyclyl selected out of the group acridinyl, azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxa-zolinyl, dioxazolyl, dioxazinyl, 1,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,2-oxazinyl, 1,3-oxazinyl, 1,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl,1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1 ,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,4-triazolyl, 1,2,5-triazolyl, 1,3,4-triazolyl and xanthenyl,
wherein heterocyclyl is unsubstituted or mono-, di- or trisubstituted independently of one another by R8,

R8 is
1) halogen,
2) $-NO_2$,
3) -CN,
4) $-C(O)-NH_2$,
5) -OH,
6) $-NH_2$,
7) $-O-CF_3$
8) a monocyclic or bicyclic 6- to 14-membered aryl, wherein aryl is as defined above and wherein aryl is mono-, di- or trisubstituted independently of one another by halogen or-O-$(C_1-C_8)$-alkyl,
9) -$(C_1-C_8)$-alkyl, wherein alkyl is unsubstituted or mono-, di- or trisubstituted independently of one another by halogen, $NH_2$, -OH or a methoxy residue, or

10) -O-($C_1$-$C_8$)-alkyl, wherein alkyl is unsubstituted or mono-, di- or trisubstituted independently of one another by halogen, $NH_2$, -OH or a methoxy residue,
11) -$SO_2$-$CH_3$ or
12) -$SO_2$-$CF_3$,

provided that R8 is at least one halogen, -C(O)-$NH_2$ or-O-($C_1$-$C_8$)-alkyl residue, if $R^0$ is a monocyclic or bicyclic 6- to 14-membered aryl, wherein aryl is as defined above, substructure D is a residue selected out of the group azetidine, azetine, azocane, azocane-2-one, cyclobutyl, cyclooctane, cyclooctene, cyclopentyl, cyclohexyl, cycloheptyl, cyclooctyl, 1,4-diazepane, 1,2-diazepine, 1,3-diazepine, 1,4-diazepine, [1,4]diazocane, [1,2]diazocan-3-one, [1,3]diazocan-2-one, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxa-thiepane, 1,2-oxathiolan, 1,2-oxazine, 1,3-oxazine, 1,4-oxazine, oxazole, [1,4]oxazocane, [1,3]oxazocan-2-one, oxetan, oxocane, oxocan-2-one, piperazine, piperidine, phenyl, pyran, pyrazine, pyrazole, pyrazoline, pyrazolidine, pyridazine, pyridine, pyrimidine, pyrrole, pyrrolidine, pyrrolidinone, pyrroline, 5,6,7,8-tetrahydro-1H-azocin-2-one, tetrahydrofuran, tetrahydropyran, tetrahydropyridine, tetrazine, thiadiazine, thiadiazole, 1,2-thiazine, 1,3-thiazine, 1,4-thiazine, 1,3-thiazole, thiazole, thiazolidine, thiazoline, thietan, thiocane, thiocane-1,1-dioxide, thiocane-1-oxide, thiocan-2-one, thiomorpholine, thiophene, thiopyran, 1,2,3-triazine, 1,2,4-triazine, 1,3,5-triazine, 1,2,3-triazole or 1,2,4-triazole and is unsubstituted or substituted 1, 2, 3,4, 5 or 6 times by R3,

Q is a direct bond, -($C_0$ -$C_2$)-alkylene-C(O)-$NR^{10}$-, -$NR^{10}$-C(O)-$NR^{10}$-, -$NR^{10}$-C(O)-, -$SO_2$-, -($C_1$-$C_6$)-alkylene, -$(CH_2)_m$-$NR^{10}$-C(O)-$NR^{10}$-$(CH_2)_n$-, -$(CH_2)_m$-$NR^{10}$-C(O)-$(CH_2)_{n-}$, -$(CH_2)_m$-S-$(CH_2)_n$-, -$(CH_2)_m$-C(O)-$(CH_2)_n$-, -$(CH_2)_n$-$SO_2$-$NR^{10}$-$(CH_2)$n-, -$(CH_2)_m$-$NR^{10}$-$SO_2$-$(CH_2)_n$-, -$(CH_2)_m$-$NR^{10}$-$SO_2$-$NR^{10}$-$(CH_2)_n$-, -$(CH_2)_m$-CH(OH)-$(CH_2)_n$-, -$(CH_2)_m$-O-C(O)-$NR^{10}$-$(CH_2)_n$-, ($C_2$-$C_3$)-alkylene-O-($C_2$-$C_3$)-alkylene-, -($C_2$-$C_3$)-alkylene-S(O)-, -($C_2$-$C_3$)-alkylene-$S(O)_2$-, -$(CH_2)_m$-$NR^{10}$-C(O)-O-$(CH_2)_n$-, -($C_2$-$C_3$)-alkylene-$S(O)_2$-NH-($R^{10}$)-, -($C_2$-$C_3$)-alkylene-N($R^{10}$)- or -($C_0$-$C_3$)-alkylene-C(O)-O-,
wherein $R^{10}$ is as defined below, and wherein n and m are independently of one another identical or different and are the integers zero, 1, 2, 3, 4, 5 or 6, wherein the alkylene residues which are formed by -$(CH_2)_m$- or -$(CH_2)_n$- are unsubstituted or mono-, di- or trisubstituted independently of one another by halogen, -$NH_2$ or -OH; or -($C_3$-$C_6$)-cycloalkylen, wherein cycloalkylen is unsubstituted or mono-, di- or trisubstituted independently of one another by halogen, -$NH_2$ or-OH;

$R^1$ is a hydrogen atom, -($C_1$-$C_4$)-alkyl, wherein alkyl is unsubstituted or substituted one to three times by R13; -($C_1$-$C_3$)-alkylene-C(O)-NH-$R^0$, -($C_1$-$C_3$)-alkylene-C(O)-O-R15, an aryl out of the group phenyl, naphthyl, biphenylyl, anthryl or fluorenyl, wherein aryl is mono-, di- or trisubstituted independently of one another by R8, wherein R8 is as defined above;
a monocyclic or bicyclic 4- to 15-membered heterocyclyl,which is as defined above; -($C_1$-$C_3$)-perfluoroalkylene, -($C_1$-$C_3$)-alkylene-S(O)-($C_1$-$C_4$)-alkyl, -($C_1$-$C_3$)-alkylene-$S(O)_2$-($C_1$-$C_3$)-alkyl, -($C_1$-$C_3$)-alkylene-$S(O)_2$-N($R^{4'}$)-$R^{5'}$, -($C_1$-$C_3$)-alkylene-O-($C_1$-$C_4$)-alkyl, -($C_1$-$C_3$)-alkylene-($C_3$-$C_8$)-cycloalkyl, or -($C_0$-$C_3$)-alkylene-het, wherein het is a residue selected out of the group azepine, azetidine, aziridine, azirine, 1,4-diazapane, 1,2-diazepine, 1,3-diazepine, 1,4-diazepine, diaziridine, diazirine, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,4-oxazepane, 1,2-oxa-thiepane, 1,2-oxathiolane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently of one another by R14,

$R^{4'}$ and $R^{5'}$ are independent of one another are identical or different and are hydrogen atom or -($C_1$-$C_4$)-alkyl,

$R^2$ is a direct bond or-($C_1$-$C_4$)-alkylene, or

$R^1$ and R3 together with the atoms to which they are bonded can form a 6- to 8- membered cyclic residue selected out of the group azocane, azocane-2-one, 1,2-diazepine, 1,3-diazepine, 1,4-diazepine, [1,4]diazocane, [1,2]diazocan-3-one, [1,3]diazocan-2-one, dioxazine, [1,4]dioxocane, dioxole, ketopiperazine, morpholine, 1,2-oxazine, 1,3-oxazine, 1,4-oxazine, [oxocane, oxocan-2-one, piperazine, piperidine, pyran, pyrazine, pyridazine, pyrimidine or 5,6,7,8-tetrahydro-1H-azocin-2-one, wherein said cyclic group is unsubstituted or mono-, di- or trisubstituted independently of one another by R14, or

$R^1$-N-$R^2$-V can form a 4- to 8-membered cyclic group selected out of the group azepine, azetidine, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole, wherein said cyclic group is unsubstituted or mono-, di- or trisubstituted independently of one another by R14,

R14 is fluorine, chlorine, bromine, iodine, -OH, =O, -($C_1$-$C_8$)-alkyl, -($C_1$-$C_4$)-alkoxy, -$NO_2$, -C(O)-OH, -CN, -$NH_2$, -C(O)-O-($C_1$-$C_4$)-alkyl, -($C_1$-$C_8$)-alkylsulfonyl, -$SO_2$-($R^{18}$)-$R^{21}$, -C(O)-NH-($C_1$-$C_8$)-alkyl,-C(O)-N-[($C_1$-$C_8$)-alkyl]$_2$, -$NR^{18}$-C(O)-NH-($C_1$-$C_8$)-alkyl, -C(O)-$NH_2$, -S-$R^{18}$, or-$NR^{18}$-C(O)-NH-[($C_1$-$C_8$)-alkyl]$_2$,
wherein $R^{18}$ and $R^{21}$ are independently from each other hydrogen atom, -($C_1$-$C_3$)-perfluoroalkyl or-($C_1$-$C_6$)-alkyl,

V is 1) a monocyclic or bicyclic 6- to 14-membered aryl out of the group phenyl, naphthyl, biphenylyl, anthryl or fluorenyl, wherein aryl is mono-, di- or trisubstituted independently of one another by R14,

2) a heterocyclyl out of the group acridinyl, azaindole (1H-pyrrolopyridine), azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 1,4-diazepane, 4,5-dihydrooxa-zolinyl, dioxazolyl, dioxazinyl, 1,3-dioxolanyl, 1,3-dioxolenyl, 6H-1,5,2-dithiaziny), dihydrofuro [2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1 ,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,2-oxazinyl, 1 ,3-oxazinyl, 1 ,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisochinolinyl, tetrahydrochinolinyl, 1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenyl, thiopyranyl, 1,2,3-triazinyl, 1,2,3-triazolyl, 1,2,3-triazolyl, 1,2,4-triazolyl, 1,2,5-triazolyl, 1,3,4-triazolyl and xanthenyl,
wherein said heterocyclyl is unsubstituted or mono-, di- or trisubstituted independently of one another by R14,

G is a direct bond, $-(CH_2)_m-NR^{10}-SO_2-NR^{10}-(CH_2)_n-$, $-(CH_2)_m-CH(OH)-(CH_2)_n-$, $-(CH_2)_m-$, $-(CH_2)_m-O-(CH_2)_n-$, $-(CH_2)_m-C(O)-NR^{10}-(CH_2)_n-$, $-(CH_2)-SO_2-(CH_2)_n-$, $-(CH_2)_m-NR^{10}-C(O)-NR^{10}-(CH_2)_{n-}$, $-(CH_2)_m-NR^{10}-C(O)-(CH_2)_n-$ $(CH_2)_m-C(O)-(CH_2)_n-$, $-(CH_2)-S-(CH_2)_n-$, $-(CH_2)_m-SO_2-NR^{10}-(CH_2)_n-$ $-(CH_2)_m-NR^{10}-SO_2-(CH_2)_n-$, $-(CH_2)_m-NR^{10}-$, $-(CH_2)_m-O-C(O)-NR^{10}-(CH_2)_n-$ or-$(CH_2)_m-NR^{10}-C(O)-(CH_2)_{n-}$,

n and m are independently of one another identical or different and are the integers zero, 1, 2, 3, 4, 5 or 6,

M is
1) a hydrogen atom,
2) $-(C_1-C_8)$-alkyl, wherein alkyl is unsubstituted or mono-, di- or trisubstituted independently of one another by R14,
3) -C(O)-N(R11)-R12,
4) $-(CH_2)_m-NR^{10}$,
5) $-(C_6-C_{14})$-aryl, wherein aryl is as defined above and wherein aryl is unsubstituted or mono-, di- or trisubstituted independently of one another by R14,
6) $-(C_4-C_{15})$-heterocyclyl, wherein heterocyclyl is as defined above and is unsubstituted or mono-, di- or trisubstituted independently of one another by R14, or
7) $-(C_3-C_8)$-cycloalkyl, wherein said cycloalkyl is unsubstituted or mono-, di- or trisubstituted independently of one another by R14,

R3 is
1) hydrogen atom,
2) halogen,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_4)$-alkylene-O-$R^{19}$, wherein R19 is

   a) hydrogen atom,
   b) $-(C_1-C_4)$-alkyl, wherein alkyl is unsubstituted or mono-, di- or trisubstituted independently of one another by R13, or
   c) phenyl, wherein phenyl is unsubstituted or mono-, di- or trisubstituted independently of one another by R13,
   d) $-CF_3$,
   e) $-CHF_2$,

7) $-NO_2$,
8) -CN,
9) $-SO_s-R^{11}$, wherein s is 1 or 2,
10) $-SO_t-N(R^{11})-R^{12}$, wherein t is 1 or 2,
11) $-(C_0-C_4)$-alkylene-C(O)-$R^{11}$,
12) $-(C_0-C_4)$-alkylene-C(O)-O-$R^{11}$,
13) $-(C_0-C_4)$-alkylene-C(O)-N($R^{11}$)-$R^{12}$,
14) $-(C_0-C_4)$-alkylene-N($R^{11}$)-$R^{12}$,
15) $-NR^{10}-SO_2-R^{10}$,
16) $-S-R^{10}$,
17) $-(C_0-C_2)$alkylene-C(O)-O-$(C_2-C_4)$-alkylene-O-C(O)-$(C_1-C_4)$-alkyl,
18) -C(O)-O-C(R15, R16)-O-C(O)-R17,
19) $-(C_0-C_2)$alkylene-C(O)-O-$(C_2-C_4)$-alkylene-O-C(O)-O-$(C_2-C_6)$-alkyl,
20) -C(O)-O- C(R15, R16)-O-C(O)-O-R17,
21) $-(C_0-C_4)$-alkylene-$(C_6-C_{14})$-aryl, wherein aryl is mono-, di- or trisubstituted independently of one another by R13,
22) $-(C_0-C_4)$-alkylene-$(C_4-C_{15})$-heterocyclyl, wherein heterocyclyl is unsubstituted or mono-, di- or trisubstituted independently of one another by R13
23) $-(C_0-C_4)$-alkylene-$(C_3-C_8)$-cycloalkyl, wherein cycloalkyl is unsubstituted or mono-, di- or trisubstituted independently of one another by R13,
24) $-(C_0-C_4)$-alkylene-het, wherein het is unsubstituted or mono-, di- or trisubstituted independently of one another by R13, or
25) a residue from the following list

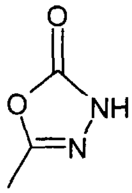
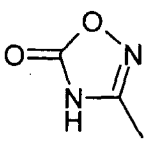
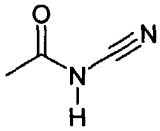
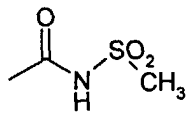
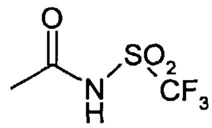
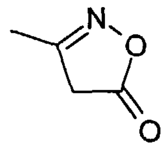
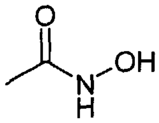
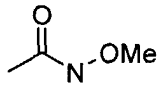
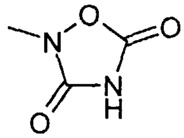
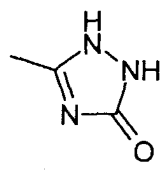
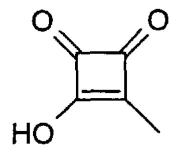
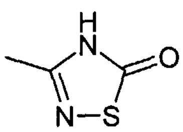
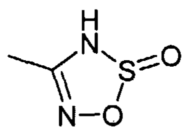
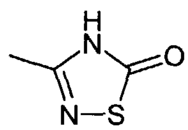
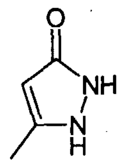
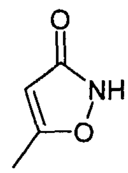
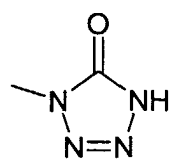
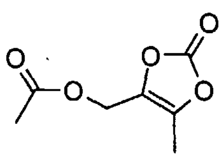
and
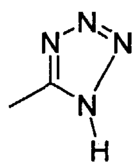

wherein Me is methyl, or

if two -OR19 residues are attached to adjacent atoms they can form together with the atoms which they are attached to a 1,3-dioxole ring or a 2,3-dihydro-[1,4]dioxine ring, which is substituted one, two, three or four times by R13,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_3$-$C_8$)-cycloalkyl,
4) -$SO_t$-$R^{10}$, wherein t is 1 or 2,
5) -($C_0$-$C_6$)-alkyl-($C_6$-$C_{14}$)-aryl, wherein alkyl and aryl independently from one another are unsubstituted or mono-, di- or trisubstituted by R13,
6) -($C_1$-$C_3$)-perfluoroalkyl,
7) -O-$R^{17}$, or
8) -($C_0$-$C_6$)-alkyl-($C_4$-$C_{15}$)-heterocyclyl, wherein alkyl and heterocyclyl are as

defined above and are independently from one another unsubstituted or mono-, di- or trisubstituted by R13, or

R11 and R12 together with the nitrogen atom to which they are bonded form a heterocyclic ring out of the group azepine, azetidine, dioxazole, dioxazine, 1,4-diazepane, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said heterocyclic ring is unsubstituted or mono-, di- or trisubstituted independently of one another by R13,

R13 is halogen, $-NO_2$, -CN, =O, -OH, $-CF_3$, $-C(O)-O-R^{10}$, $-C(O)-N(R^{10})-R^{20}$, $-N(R^{10})-R^{20}$, $-(C_3-C_8)$-cycloalkyl, $-(C_0-C_3)$-alkylene-O-$R^{10}$, $-Si-(CH_3)_3$, $-N(R^{10})-S(O)_u-R^{10}$, wherein u is 1 or 2, $-S-R^{10}$, $-SO_r-R^{10}$, wherein r is 1 or 2, $-S(O)_v-N(R^{10})-R^{20}$, wherein v is 1 or 2, $-C(O)-R^{10}$, $-(C_1-C_8)$-alkyl, $-(C_1-C_8)$-alkoxy, phenyl, phenyloxy-, $-O-CF_3$, $-(C_0-C_4)$-alkyl-C(O)-O-C(R15, R16)-O-C(O)-R17, $-(C_1-C_4)$-alkoxy-phenyl, $-(C_0-C_4)$-alky)-C(O)-O-C(R15, R16)-0-C(0) -O-R17, $-(C_1-C_3)$-perfluoroalkyl, -O-R15, $-NH-C(O)-NH-R^{10}$, $-NH-C(O)-O-R^{10}$, or a residue from the following list

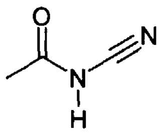 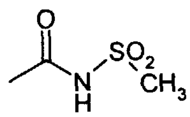 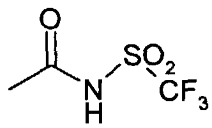 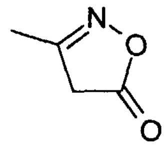 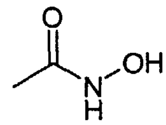

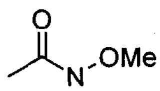 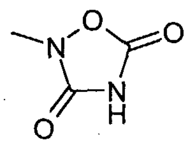 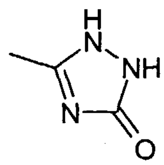 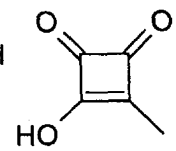 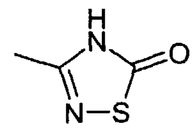

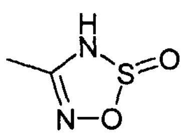 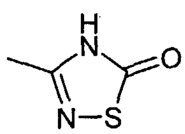 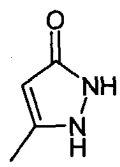 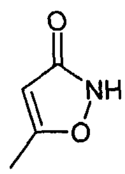 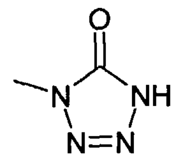

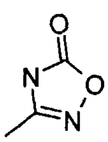 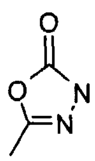 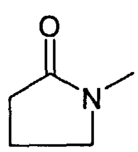 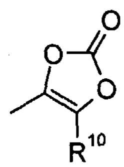 and 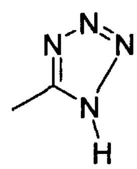 ,

$R^{10}$ and $R^{20}$ are independently of one another hydrogen, $-(C_1-C_6)$-alkyl, $-(C_0-C_4)$-alkyl-OH, $-(C_0-C_4)$-alkyl-O-$(C_1-C_4)$-akyl or $-(C_1-C_3)$-perfluoroalkyl,

R15 and R16 are independently of one another hydrogen, $-(C_1-C_6)$-alkyl, cyclopropyl, cyclobutyl, cyclopentyl or cyclohexyl, wherein each ring is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by-OH, -O-$(C_1-C_4)$-alkyl or $R^{10}$,

in all its stereoisomeric forms and mixtures thereof in any ratio, and its physiologically tolerable salts.

**4.** A compound of formula I as claimed in claims 1 to 3, wherein

$R^0$ is 1) a monocyclic or bicyclic 6- to 14-membered aryl out of the group phenyl, naphthyl, biphenyl, anthryl or fluorenyl, wherein aryl is mono-, di- or trisubstituted independently of one another by R8,

2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, phenylpyridyl, phthalazinyl, pteridinyl, purinyl, pyridinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, wherein said heterocyclyl is unsubstituted or mono-, di- or trisubstituted independently of one another by R8, or

3) a heterocyclyl out of the group azabenzimidazolyl, benzimidazolyl, 1,3-benzodioxolyl, benzofuranyl, benzothiazolyl, benzothiophenyl, benzoxazolyl, chromanyl, cinnolinyl,

2-furyl, 3-furyl; imidazolyl, indolyl, indazolyl, isochromanyl, isoindolyl, isoquinolinyl, isothiazolyl, isoxazolyl, oxazolyl, phthalazinyl, pteridinyl, purinyl, pyrazinyl, pyrazolyl, pyridazinyl, pyridoimidazolyl, pyridopyridinyl, pyridopyrimidinyl, 2-pyridyl, 3-pyridyl, 4-pyridyl, pyrimidinyl, pyrrolyl; 2-pyrrolyl, 3-pyrrolyl, quinolinyl, quinazolinyl, quinoxalinyl, tetrazolyl, thiazolyl, 2-thienyl or 3-thienyl,

which is additionally substituted by a heterocyclyl selected out of the group acridinyl, azabenzimidazolyl, azaspirodecanyl, azepinyl, azetidinyl, aziridinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxa-zolinyl, dioxazolyl, dioxazinyl, 1,3-dioxolanyl, 1,3-dioxolenyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,2-oxazinyl, 1,3-oxazinyl, 1,4-oxazinyl, oxazolidinyl, oxazolinyl, oxazolyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisochinolinyl, tetrahydrochinolinyl, 1,4,5,6-tetrahydro-pyridazinyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenyl, thiopyranyl, 1,2,3-triazinyl, 1,2,3-triazolyl, 1,2,3-triazolyl, 1,2,4-triazolyl, 1 ,2,5-triazolyl, 1,3,4-triazolyl and xanthenyl,

wherein heterocyclyl is unsubstituted or mono-, di- or trisubstituted independently of one another by R8,

R8 is

1. fluor, chlor or brom,
2. $-NO_2$,
3. -CN,
4. $-C(O)-NH_2$,
5. -OH,
6. $-NH_2$,
7. $-OCF_3$
8. a monocyclic or bicyclic 6- to 14-membered aryl, wherein aryl is as defined above and is mono-, di- or trisubstituted independently of one another by halogen or $-O-(C_1-C_8)$-alkyl,
9. $-(C_1-C_8)$-alkyl, wherein alkyl is unsubstituted or mono-, di- or trisubstituted independently of one another by halogen, $NH_2$, -OH or a methoxy residue, or
10. $-O-(C_1-C_8)$-alkyl, wherein alkyl is unsubstituted or mono-, di- or trisubstituted independently of one another by halogen, $NH_2$, -OH or a methoxy residue,
11. $-SO_2CH_3$ or
12. $-SO_2CF_3$,

provided that R8 is at least one halogen, $-C(O)-NH_2$ or-$O-(C_1-C_8)$-alkyl residue, if R0 is a aryl or a heterocyclyl, which are as defined above,

substructure D is a residue selected out of the group phenyl, pyridyl, pyridyl-N-oxide pyridyl, pyrrolyl, furyl, thienyl, imidazolyl, pyrazolyl, oxazolyl, isoxazolyl, thiazolyl, triazolyl, isothiazolyl, thiadiazolyl, pyrimidinyl, pyridazinyl, pyrazinyl and is unsubstituted or substituted 1, 2, 3 or 4 times by R3,

Q is a direct bond, $-(C_0 -C_2)$-alkylene-$C(O)-NR^{10}$-, $-NR^{10}-C(O)-NR^{10}$-, $-NR^{10}-C(O)$-, $-SO_2$- or-$(C_1-C_6)$-alkylene,

$R^1$ is a hydrogen atom, $-(C_1-C_4)$-alkyl, wherein alkyl is unsubstituted or substituted one to three times by R13; $-(C_1-C_3)$-alkylene-C(O)-NH-$R^0$, $-(C_1-C_3)$-alkylene-C(O)-O-R15, $-(C_1-C_3)$-perfluoroalkylene, $-(C_1-C_3)$-alkylene-S(O)-$(C_1-C_4)$-alkyl, $-(C_1-C_3)$-alkylene-$S(O)_2$-$(C_1-C_3)$-alkyl, $-(C_1-C_3)$-alkylene-$S(O)_2$-N($R^{4'}$)-$R^{5'}$, $-(C_1-C_3)$-alkylene-O-$(C_1-C_4)$-alkyl, $-(C_0-C_3)$-alkylene-$(C_3-C_8)$-cycloalkyl, or $-(C_0-C_3)$-alkylene-het, wherein het is a residue selected out of the group azepine, azetidine, aziridine, azirine, 1,4-diazepane, 1,2-diazepine, 1,3-diazepine, 1,4-diazepine, diaziridine, diazirine, dioxazole, dioxazine, dioxole, 1,3-dioxolene, 1 , 3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxa-thiepane, 1,2-oxathiolane, 1,4-oxazepane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, wherein het is unsubstituted or mono-, di- or trisubstituted independently of one another by R14,

$R^{4'}$ and $R^{5'}$ are independent of one another are identical or different and are hydrogen atom or $-(C_1-C_4)$-alkyl,

$R^2$ is a direct bond or $-(C_1-C_4)$-alkylene, or

$R^1$-N-$R^2$-V form a 4- to 8-membered cyclic group selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, 1,4-oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole, wherein said cyclic group is unsubstituted or mono-, di- or trisubstituted independently of one another by R14,

R14 is fluorine, chlorine, bromine, iodine, -OH, =O, $-(C_1-C_8)$-alkyl, $-(C_1-C_4)$-alkoxy, $-NO_2$, -C(O)-OH, -CN, $-NH_2$, -C(O)-O-$(C_1-C_4)$-alkyl, $-(C_1-C_8)$-alkylsulfonyl, $-SO_2$-($R^{18}$)-$R^{21}$, -C(O)-NH-$(C_1-C_8)$-alkyl, -C(O)-N-$[(C_1-C_8)\text{-alkyl}]_2$, -$NR^{18}$-C(O)-NH-$(C_1-C_8)$-alkyl, -C(O)-$NH_2$, -S-$R^{18}$, or -$NR^{18}$-C(O)-NH-$[(C_1-C_8)\text{-alkyl}]_2$,

wherein $R^{18}$ and $R^{21}$ are independently from each other hydrogen atom, $-(C_1-C_3)$-perfluoroalkyl or $-(C_1-C_6)$-alkyl,

V is

1) a het residue out of the group azaindole ( 1H-pyrrolopyridine), azepine, azetidine, aziridine, azirine, 1,4-diazepane, 1,2-diazepine, 1,3-diazepine, 1,4-diazepine, diaziridine, diazirine, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketopiperazine, morpholine, 1,2-oxa-thiepane, 1,2-oxathiolane, 1,4-oxazepane, 1,2-oxazine, 1,3-oxazine, 1,4-oxazine, oxazole, oxaziridine, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiadiazine, thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, which is as defined above and wherein het is unsubstituted or mono-, di- or trisubstituted independently of one another by R14, or

2) phenyl, wherein phenyl is unsubstituted or mono-, di- or trisubstituted independently of one another by R14,

G is a direct bond, $-(CH_2)_m$-$NR^{10}$-$SO_2$-$NR^{10}$-$(CH_2)_n$-, $-(CH_2)_m$-CH(OH)-$(CH_2)_n$-, $-(CH_2)_m$-, $-(CH_2)_m$-O-$(CH_2)_n$-, $-(CH_2)_m$-C(O)-$NR^{10}$ -$(CH_2)_n$-, $-(CH_2)$-$SO_2$-$(CH_2)_n$-, $-(CH_2)_m$-$NR^{10}$-C(O)-$NR^{10}$-$(CH_2)_n$-, $-(CH_2)_m$-$NR^{10}$-C(O)-$(CH_2)_n$-, $-(CH_2)_m$-C(O)-$(CH_2)_n$-, $-(CH_2)$-S-$(CH_2)_n$-, $-(CH_2)_m$-$SO_2$-$NR^{10}$-$(CH_2)_n$-, $-(CH_2)_m$-$NR^{10}$-$SO_2$-$(CH_2)_n$-, $-(CH_2)_m$-$NR^{10}$-, $-(CH_2)_m$-O-C(O)-$NR^{10}$-$(CH_2)_n$- or $-(CH_2)_m$-$NR^{10}$-C(O)-O-$(CH_2)_n$-,

n and m are independently of one another identical or different and are the integers zero, 1, 2, 3, 4, 5 or 6,

M is

1) a hydrogen atom,

2) $-(C_1-C_8)$-alkyl, wherein alkyl is unsubstituted or mono-, di- or trisubstituted independently of one another by R14,
3) -C(O)-N(R11)-R12,
4) $-(CH_2)_m-NR^{10}$,
5) phenyl or naphthyl, wherein phenyl or naphthyl are unsubstituted or mono-, di- or trisubstituted independently of one another by R14,
6) heterocyclyl, wherein heterocyclyl is a residue out of the group which can be derived from azepane, azepine, 1,4-diazepane, 1,2-diazepine, 1,3-diazepine, 1,4-diazepine, imidazole, isothiazole, isoxazole, isoxazolidine, 2-isoxazoline, ketomorpholine, ketopiperazine, morpholine, oxazole, [1,4]-oxazepane, piperazine, piperazinone, piperidine, piperidinone, pyrazine, pyridazine, pyridazinone, pyridine, pyridone, pyrimidine, pyrrolidine, pyrrolidinone, tetrahydropyran, 1,4,5,6-tetrahydro-pyridazinyl, tetrazine, tetrazole, thiadiazole, thiazole, thiophene, thiomorpholine, 1,2,3-triazine, 1,2,4-triazine, 1,3,5-triazine, 1,2,3-triazole or 1,2,4-triazole, wherein said heterocyclyl is unsubstituted or mono-, di- or trisubstituted independently of one another by R14, or
7) $-(C_3-C_8)$-cycloalkyl, wherein said cycloalkyl is unsubstituted or mono-, di- or trisubstituted independently of one another by R14,

R3 is

1) hydrogen atom,
2) halogen,
3) $-(C_1-C_4)$-alkyl, wherein alkyl is unsubstituted or mono-, di- or trisubstituted independently of one another by R13,
4) $-(C_1-C_3)$-perfluoroalkyl,
5) phenyl, wherein phenyl is unsubstituted or mono-, di- or trisubstituted independently of one another by R13,
6) $-(C_0-C_4)$-alkylene-O-R19, wherein R19 is

a) hydrogen atom,
b) $-(C_1-C_4)$-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) $-CF_3$, or
e) $CHF_2$,

7) -CN,
8) $-(C_0-C_4)$-alkylene-$(C_4-C_{15})$-heterocyclyl, wherein heterocyclyl is as defined above and is unsubstituted or mono-, di- or trisubstituted independently of one another by R13,
9) $-SO_s-R^{11}$, wherein s is 1 or 2,
10) $-SO_t-N(R^{11})-R^{12}$, wherein t is 1 or 2,
11) $-(C_0-C_4)$-alkylene-$C(O)-R^{11}$,
12) $-(C_0-C_4)$-alkylene-$C(O)-O-R^{11}$,
13) $-(C_0-C_4)$-alkylene-$C(O)-N(R^{11})-R^{12}$,
14) $-(C_0-C_4)$-alkylene-$N(R^{11})-R^{12}$,
15) $-NR^{10}-SO_2-R^{10}$,
16) $-(C_0-C_4)$-alkylene-het, wherein het is as defined above and is unsubstituted or mono-, di- or trisubstituted independently of one another by R13,
17) $-(C_0-C_2)$alkylene-C(O)-O-$(C_2-C_4)$-alkylene-O-C(O)-$(C_1-C_4)$-alkyl,
18) -C(O)-O-C(R15, R16)-0-C(0)-R17,
19) $-(C_0-C_2)$alkylene-C(O)-O-$(C_2-C_4)$-alkylene-O-C(O)-O-$(C_1-C_6)$-alkyl,
20) -C(O)-O- C(R15, R16)-O-C(O)-O-R17,
21) $-(C_0-C_4)$-alkylene-$(C_6-C_{14})$-aryl, wherein aryl is as defined above and is mono-, di- or trisubstituted independently of one another by R13,
22) $-(C_0-C_4)$-alkylene-$(C_3-C_8)$-cycloalkyl, wherein cycloalkyl is unsubstituted or mono-, di- or trisubstituted independently of one another by R13, or
23) a residue from the following list

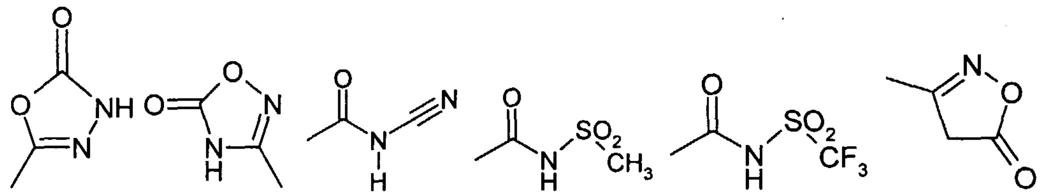

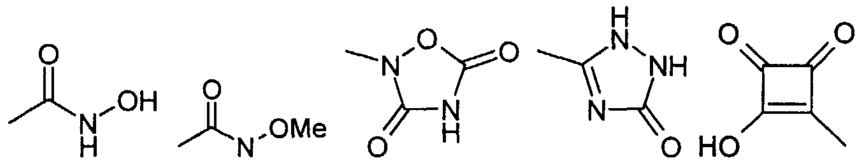

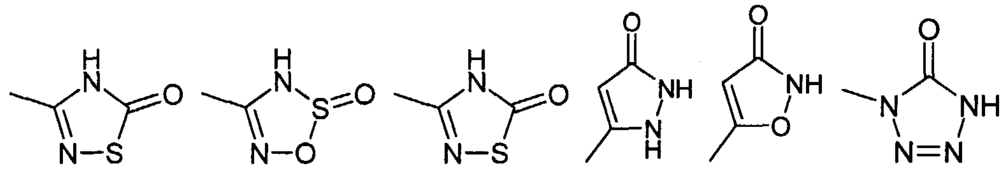

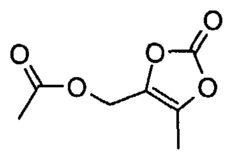

and

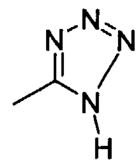

wherein Me is methyl,

if two -OR19 residues are attached to adjacent atoms they can form together with the atoms which they are attached to a 1,3-dioxole ring or a 2,3-dihydro-[1,4]dioxine ring, which is substituted one, two, three or four times by R13,

R11 and R12 are independently of one another identical or different and are

1) hydrogen atom,
2) -($C_1$-$C_6$)-alkyl, wherein alkyl is unsubstituted or mono-, di- or trisubstituted independently of one another by R13,
3) -($C_0$-$C_6$)-alkyl-($C_6$-$C_{14}$)-aryl, wherein aryl is as defined above and wherein alkyl and aryl are independently from one another unsubstituted or mono-, di- or trisubstituted by R13,
4) -O-$R^{17}$, or
5) -($C_0$-$C_6$)-alkyl-($C_4$-$C_{15}$)-heterocyclyl, wherein alkyl and heterocyclyl is as defined above and independently from one another are unsubstituted or mono-, di- or trisubstituted by R13, or

R11 and R12 together with the nitrogen atom to which they are bonded can form a ring selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline, isoxazole, isoxazoline, isoxazolidine, 2-isoxazoline, ketopiperazine, morpholine, [1,4]oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, 1,2,3-triazine, 1,2,4-triazine, 1,3,5-triazine, 1,2,3-triazole or 1,2,4-triazole, which is unsubstituted or mono-, di- or trisubstituted independently of one another by R13,

R13 is fluorine, chlorine, bromine, iodine, -$NO_2$, -CN, =O, -OH, -$CF_3$, -C(O)-O-$R^{10}$, -C(O)-N($R^{10}$)-$R^{20}$, -N($R^{10}$)-$R^{20}$, -($C_0$-$C_3$)-alkylene-O-$R^{10}$, -Si-$(CH_3)_3$, -N($R^{10}$)-$S(O)_2$-$R^{10}$, -S-$R^{10}$, -$SO_2$-$R^{10}$, -$S(O)_2$-N($R^{10}$)-$R^{20}$, -C(O)-$R^{10}$, -($C_1$-$C_8$)-alkyl, -($C_1$-$C_8$)-alkoxy, phenyl, phenyloxy-, -O-$CF_3$, -($C_1$-$C_3$)-perfluoroalkyl, -($C_0$-$C_4$)-alkyl-C(O)-O-C(R15, R16)-O-C(O)-R17, -($C_1$-$C_4$)-alkoxy-phenyl, -($C_0$-$C_4$)-alkyl-C(O)-O-C(R15, R16)-O-C(O)-O-R17, -0-R15, -NH-C(O)-NH-$R^{10}$, -NH-C(O)-O-$R^{10}$, or a residue from the following list

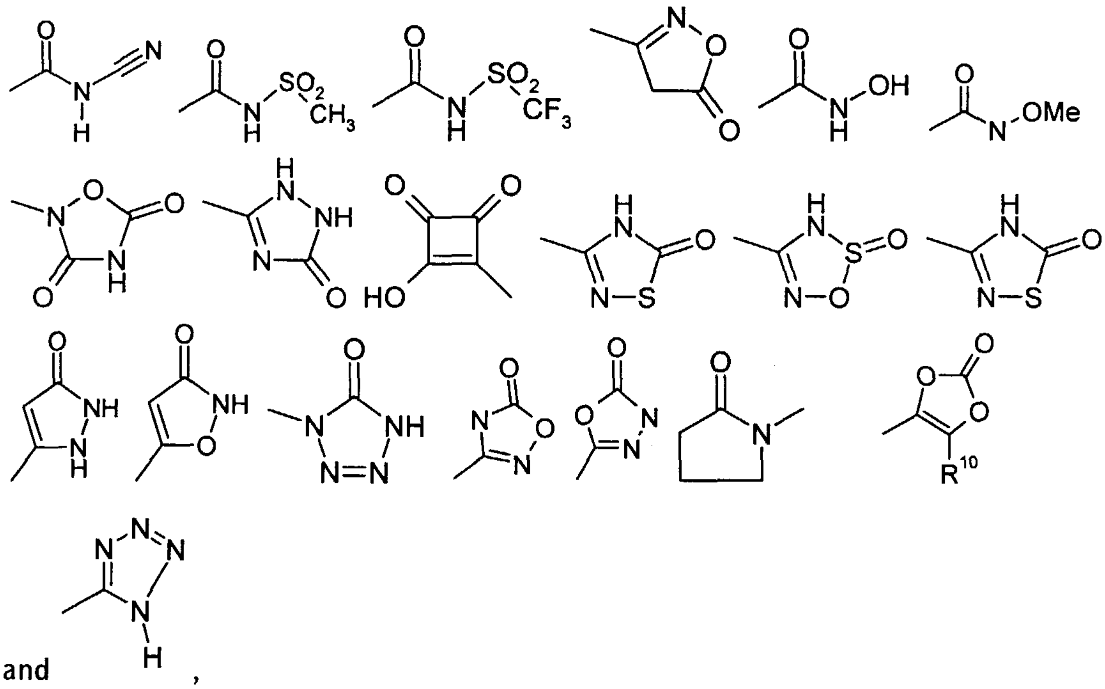

and ,

| | |
|---|---|
| $R^{10}$ and $R^{20}$ are | independently of one another hydrogen, -($C_1$-$C_6$)-alkyl, -($C_0$-$C_4$)-alkyl-OH, -($C_0$-$C_4$)-alkyl-O-($C_1$-$C_4$)-akyl or -($C_1$-$C_3$)-perfluoroalkyl, |
| R15 and R16 are | independently of one another hydrogen, -($C_1$-$C_6$)-alkyl, or together form a ring out of the droup cyclopropyl, cyclobutyl, cyclopentyl or cyclohexyl, wherein each ring is unsubstituted or substituted one to three times by $R^{10}$, and |
| R17 is | -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by-OH, -O-($C_1$-$C_4$)-alkyl or $R^{10}$, |

in all its stereoisomeric forms and mixtures thereof in any ratio, and its physiologically tolerable salts.

**5.** A compound of formula I as claimed in claims 1 to 4, wherein

| | |
|---|---|
| R0 is | 1) phenyl, wherein phenyl is unsubstituted or mono-, di- or trisubstituted independently of one another by R8,<br>2) a heterocyclyl out of the group benzimidazolyl, 1,3-benzodioxolyl, benzofuranyl, benzoxazolyl, benzothiazolyl, benzothiophenyl, cinnolinyl, chromanyl, indazolyl, indolyl, isochromanyl, isoindolyl, isoquinolinyl, phenylpyridyl, phthalazinyl, pteridinyl, purinyl, pyridinyl, pyridoimidazolyl, pyridopyridinyl, pyridopyrimidinyl, pyrimidinyl, quinazolinyl, quinolyl, quinoxalinyl or 1,4,5,6-tetrahydro-pyridazinyl, wherein said heterocyclyl is unsubstituted or mono-, di- or trisubstituted independently of one another by R8, or<br>3) a heterocyclyl out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said heterocyclyl is unsubstituted or mono-, di- or trisubstituted independently of one another by R8, and in addition is substituted by a residue selected out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said residue is unsubstituted or mono-, di- or trisubstituted independently of one another by R8 |
| R8 is | 1. F, Cl, Br or J,<br>2. -C(O)-$NH_2$, |

3. $-(C_1-C_4)$-alkyl, wherein alkyl is unsubstituted or mono-, di- or trisubstituted independently of one another by halogen, -OH or a methoxy residue, or

4. $-O-(C_1-C_4)$-alkyl, wherein alkyl is unsubstituted or mono-, di- or trisubstituted independently of one another by halogen or a methoxy residue,

provided that R8 is at least one halogen, $-C(O)-NH_2$ or $-O-(C_1-C_8)$-alkyl residue, if R0 is a aryl or a heterocyclyl, which are as defined above,

substructure D is a residue selected out of the group phenyl, pyridyl, pyridyl-N-oxide, pyrrolyl, furyl, thienyl, imidazolyl, pyrazolyl, oxazolyl, isoxazolyl, thiazolyl, triazolyl, isothiazolyl, thiadiazolyl, pyrimidinyl, pyridazinyl, pyrazinyl and is unsubstituted or substituted 1, 2, 3 or 4 times by R3,

Q is a direct bond, -C(O)-; $-SO_2-$ or $-(C_1-C_6)$-alkylene, $-(C_0-C_2)$-alkylene-C(O)-$NR^{10}$-,

$R^1$ is hydrogen atom, $-(C_1-C_2)$-alkyl, $-(C_1-C_3)$-alkylene-C(O)-NH-R0, $-(C_1-C_3)$-perfluoroalkylene, $-(C_1-C_3)$-alkylene-C(O)-O-$R^{15}$, $-(C_1-C_3)$-alkylene-$S(O)_2$-$(C_1-C_3)$-alkyl or $-(C_1-C_3)$-alkylene-$S(O)_2$-$N(R^{4'})$-$R^{5'}$,

wherein $R^{4'}$ and $R^{5'}$ are independent of one another are identical or different and are hydrogen atom or $-(C_1-C_4)$-alkyl,

$R^2$ is a direct bond or $-(C_1-C_2)$-alkylene,

$R^1$-N-$R^2$-V can form a 4- to 7- membered cyclic group out of the group azetidine, azetidinone, piperidine, piperazine, pyridine, pyrimidine, pyrrolidine, pyrrolidinone, 1,2,3-triazine, 1,2,4-triazine, 1,3,5-triazine, 1,2,3-triazole, 1,2,4-triazole, tetrazine, tetrazole, 1,4-diazepane, 1,2-diazepine, 1,3-diazepine, 1,4-diazepine, azepine, ketopiperazine, 1,4-oxazepane, oxazole, isoxazole, isoxazolidine, 2-isoxazoline, morpholine, thiazole, isothiazole, thiadiazole or thiomorpholine, wherein said cyclic group is unsubstituted or mono-, di- or trisubstituted independently of one another by R14,

R14 is fluorine, chlorine, -OH, =O, $-(C_1-C_8)$-alkyl, -C(O)-OH, -CN, $-NH_2$, -C(O)-O-$(C_1-C_4)$-alkyl, -C(O)-NH-$(C_1-C_8)$-alkyl, -C(O)-N-$[(C_1-C_8)\text{-alkyl}]_2$, -C(O)-$NH_2$ or -$N(R^{18})$-$R^{21}$,

wherein $R^{18}$ and $R^{21}$ are independently from each other hydrogen atom, $-(C_1-C_3)$-perfluoroalkyl or-$(C_1-C_4)$-alkyl,

V is 1. a cyclic residue out of the group containing compounds which are

derived from azaindole ( 1H-pyrrolopyridine), aziridine, azirine, azetidine, azetidinone, 1,4-diazepane, pyrrole, pyrrolidine, pyridonyl, imidazole, pyrazole, 1,2,3-triazole, 1,2,4-triazole, tetrazole, pyridine, pyrimidine, pyrazine, 1,2,3-triazine, 1,2,4-triazine, 1,3,5-triazine, tetrazine, tetrazole, azepine, diazirine, 1,2-diazepine, 1,3-diazepine, 1,4-diazepine, pyridazine, piperidine, piperazine, pyrrolidinone, ketopiperazine, furan, pyran, dioxole, 1,4-oxazepane, oxazole, isoxazole, 2-isoxazoline, isoxazolidine, morpholine, oxirane, oxaziridine, 1,3-dioxolene, 1,3-dioxolane, 1,2-oxazine, 1,3-oxazine, 1,4-oxazine, oxaziridine, thiophene, thiopyran, thietan, thiazole, isothiazole, isothiazoline, isothiazolidine, 1,2-oxathiolan, thiodiazole, thiopyran, 1,2-thiazine, 1,3-thiazole, 1,3-thiazine, 1,4-thiazine, thiadiazine or thiomorpholine,

wherein said cyclic residue is unsubstituted or mono-, di- or trisubstituted independently of one another by R14, or

2. phenyl, wherein phenyl is unsubstituted or mono-, di- or trisubstituted independently of one another by R14, or

G is a direct bond, $-(CH_2)_m-$, or $-(CH_2)_m-NR^{10}-$,

m is the integers zero, 1, 2, 3 or 4,

M is 1. a hydrogen atom,

2. heterocyclyl, wherein heterocyclyl is a residue out of the group which can be derived from azepane, azepine, 1,4-diazepane, 1,2-diazepine, 1,3-diazepine, 1,4-diazepine, imidazole, isothiazole, isoxazole, isoxazolidine, 2-isoxazoline, ketomorpholine, ketopiperazine, morpholine, oxazole, [1,4]-oxazepane, piperazine, piperazinone, piperidine, piperidinone, pyrazine, pyridazine, pyridazinone, pyridine, pyridone, pyrimidine, pyrrolidine, pyrrolidinone, tetrahydropyran, 1,4,5,6-tetrahydro-pyridazinyl, tetrazine, tetrazole, thiadiazole, thiazole, thiomorpholine, thiophene, 1,2,3-triazine, 1,2,4-triazine, 1,3,5-triazine, 1,2,3-triazole or 1,2,4-triazole, wherein said heterocyclyl is unsubstituted or mono-, di- or trisubstituted independently of one another by R14,

3. -($C_1$-$C_6$)-alkyl, wherein alkyl is unsubstituted or mono-, di- or trisubstituted independently of one another by R14,
4. ($C_3$-$C_6$)-cycloalkyl, or
5. -C(O)-N($R^{11}$)-$R^{12}$,

R3 is

1) hydrogen atom,
2) halogen,
3) -($C_1$-$C_4$)-alkyl, wherein alkyl is unsubstituted or mono-, di- or trisubstituted independently of one another by R13,
4) -($C_1$-$C_3$)-perfluoroalkyl,
5) phenyl, wherein phenyl is unsubstituted or mono-, di- or trisubstituted independently of one another by R13,
6) -($C_0$-$C_4$)-alkylene-O-R19, wherein R19 is

a) hydrogen atom,
b) -($C_1$-$C_4$)-alkyl, wherein alkyl is unsubstituted or mono-, di- or trisubstituted independently of one another by R13, or
c) phenyl, wherein phenyl is unsubstituted or mono-, di- or trisubstituted independently of one another by R13,
d) -$CF_3$, or
e) $CHF_2$,

7) -CN,
8) -$NR^{10}$-$SO_2$-$R^{10}$,
9) -$SO_s$-$R^{11}$, wherein s is 1 or 2,
10) -$SO_t$-N($R^{11}$)-$R^{12}$, wherein t is 1 or 2,
11) -($C_0$-$C_4$)-alkylene-C(O)-$R^{11}$,
12) -($C_0$-$C_4$)-alkylene-C(O)-O-$R^{11}$,
13) -($C_0$-$C_4$)-alkylene-C(O)-N($R^{11}$)-$R^{12}$,
14) -($C_0$-$C_4$)-alkylene-N($R^{11}$)-$R^{12}$,
15) -($C_0$-$C_2$)alkylene-C(O)-O-($C_2$-$C_4$)-alkylene-O-C(O)-($C_1$-$C_4$)-alkyl,
16) -C(O)-O-C(R15, R16)-O-C(O)-R17,
17) -($C_0$-$C_2$)alkylene-C(O)-O-($C_2$-$C_4$)-alkylene-O-C(O)-O-($C_1$-$C_6$)-alkyl,
18) -C(O)-O- C(R15, R16)-O-C(O)-O-R17, or
19) a residue from the following list

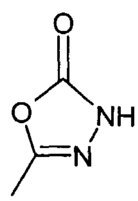 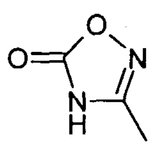 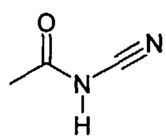 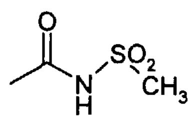 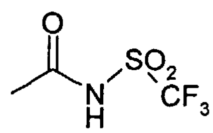 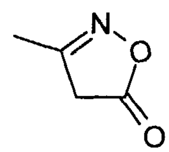

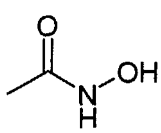 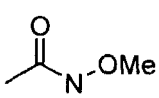 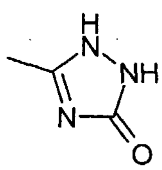 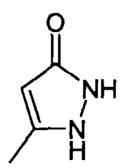 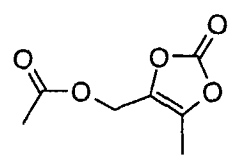 and 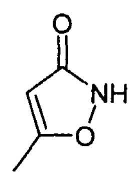

wherein Me is methyl,

if two -OR19 residues are attached to adjacent atoms they can form together with the atoms which they are attached to a 1,3-dioxole ring or a 2,3-dihydro-[1,4]dioxine ring, which is substituted one, two, three or four times by R13,

$R^{11}$ and $R^{12}$ together with the nitrogen atom to which they are bonded can form a ring selected out of the group azepine, azetidine, 1,4-diazepane, dioxazole, dioxazine, 1,2-diazepine, 1,3-diazepine, 1,4-diazepine, imidazole, imidazoline, imidazolidine, isothiazole, isothiazolidine, isothiazoline,

isoxazole, isoxazoline, isoxazolidine, 2-isoxazoline, ketopiperazine, morpholine, [1,4]-oxazepane, oxazole, piperazine, piperidine, pyrazine, pyrazole, pyrazoline, pyrazolidine, pyridazine, pyridine, pyrimidine, pyrrole, pyrrolidine, pyrrolidinone, pyrroline, tetrahydropyridine, tetrazine, tetrazole, thiazole, thiadiazole, thiazolidine, thiazoline, thiomorpholine, thiophene, 1,2,3-triazine, 1,2,4-triazine, 1,3,5-triazine, 1,2,3-triazole or 1,2,4-triazole, wherein said ring is unsubstituted or mono-, di- or trisubstituted independently of one another by R13,

R13 is fluorine, chlorine, $-NO_2$, -CN, =O, -OH, $-CF_3$, $-C(O)-O-R^{10}$, $-C(O)-N(R^{10})-R^{20}$, $-N(R^{10})-R^{20}$, $-(C_0-C_3)$-alkylene-O-$R^{10}$, $-Si-(CH_3)_3$, $-N(R^{10})-S(O)_2-R^{10}$, $-S-R^{10}$, $-SO_2-R^{10}$, $-S(O)_2-N(R^{10})-R^{20}$, $-C(O)-R^{10}$, $-(C_1-C_8)$-alkyl, $-(C_1-C_8)$-alkoxy, phenyl, phenyloxy-, $-O-CF_3$, $-(C_1-C_3)$-perfluoroalkyl, -NH-C(O)-NH-$R^{10}$, $-(C_0-C_4)$-alkyl-C(O)-O-C(R15, R16)-O-C(O)-R17, $-(C_1-C_4)$-alkoxy-phenyl, $-(C_0-C_4)$-alkyl-C(O)-O-C(R15, R16)-O-C(O)-O-R17, -O-R15, -NH-C(O)-O-$R^{10}$, or a residue from the following list

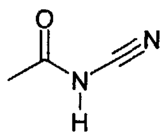 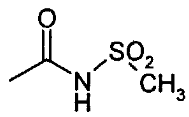 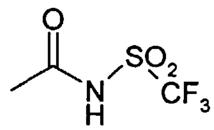 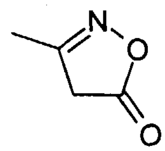 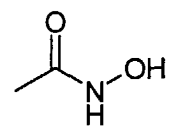

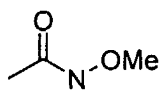 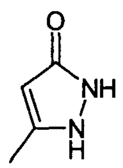 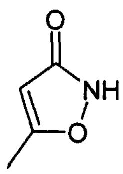 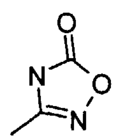 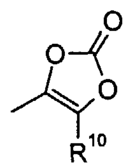 and, 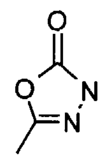

wherein Me is methyl,

$R^{10}$ and $R^{20}$ are independently of one another hydrogen, $-(C_1-C_6)$-alkyl, $-(C_0-C_4)$-alkyl-OH, $-(C_0-C_4)$-alkyl-O-$(C_1-C_4)$-akyl or $-(C_1-C_3)$-perfluoroalkyl, R15 and R16 are independently of one another hydrogen, $-(C_1-C_6)$-alkyl, or together form a ring out of the droup cyclopropyl, cyclobutyl, cyclopentyl or cyclohexyl, wherein each ring is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_8)$-alkyl-$(C_3-C_8)$-cycloalkyl, wherein said cycloalkyl ring is unsubstituted or substituted one; two or three times by -OH, -O-$(C_1-C_4)$-alkyl or $R^{10}$,

in all its stereoisomeric forms and mixtures thereof in any ratio, and its physiologically tolerable salts.

**6.** A compound of formula I as claimed in claims 1 to 5, wherein

R0 is 1. phenyl, wherein phenyl is unsubstituted or mono-, di- or trisubstituted independently of one another by R8,

2. a heterocyclyl selected out of the group indolyl, isoindolyl, benzofuranyl, benzothiophenyl, 1,3-benzodioxolyl, indazolyl, benzimidazolyl, benzoxazolyl, benzothiazolyl, quinolinyl, isoquinolinyl, chromanyl, isochromanyl, cinnolinyl, quinazolinyl, quinoxalinyl, phthalazinyl, pyridoimidazolyl, pyridopyridinyl, pyridopyrimidinyl, pyridinyl, purinyl and pteridinyl,
wherein said heterocyclyl is unsubstituted or mono-, di- or trisubstituted independently of one another by R8,

3. a heterocyclyl out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said heterocyclyl is unsubstituted or mono-, di- or trisubstituted independently of one another by R8, and in addition is substituted by a residue selected out of the group pyridyl, 2-pyridyl, 3-pyridyl, 4-pyridyl, pyrrolyl, 2-pyrrolyl, 3-pyrrolyl, furyl, 2-furyl, 3-furyl; thienyl, 2-thienyl, 3-thienyl, imidazolyl, pyrazolyl, oxazolyl, isoxazolyl, thiazolyl, thiadiazolyl, isothiazolyl, triazolyl, tetrazolyl, pyridazinyl and pyrazinyl, wherein said residue is unsubstituted or mono-, di- or trisubstituted independently of one another by R8

R8 is 1. is F, Cl, Br, J,
2. -C(O)-$NH_2$,
3. -($C_1$-$C_4$)-alkyl, wherein alkyl is unsubstituted or mono-, di- or trisubstituted independently of one another by halogen, -OH or a methoxy residue, or
4. -O-($C_1$-$C_4$)-alkyl, wherein alkyl is unsubstituted or mono-, di- or trisubstituted independently of one another by halogen or a methoxy residue,

provided that R8 is at least one halogen, -C(O)-$NH_2$ or-O-($C_1$-$C_8$)-alkyl residue, if R0 is a aryl or a heterocyclyl, which are as defined above,
substructure D is a residue selected out of the group phenyl, pyridyl, pyridyl-N-oxide, pyrrolyl, furyl, thienyl, imidazolyl, pyrazolyl, oxazolyl, isoxazolyl, thiazolyl, triazolyl, isothiazolyl, thiadiazolyl, pyrimidinyl, pyridazinyl, pyrazinyl and is unsubstituted or substituted 1, 2, 3 or 4 times by $R^3$,

Q is a direct bond, -C(O)-; -$SO_2$- or -($C_1$-$C_6$)-alkylen, -($C_0$-$C_2$)-alkylen-C(O)-$NR^{10}$-,
$R^1$ is hydrogen atom or -($C_1$-$C_2$)-alkyl,
$R^2$ is a direct bond or -($C_1$-$C_2$)-alkylen, or

$R^1$-N-$R^2$-V can form a 4- to 7- membered cyclic group out of the group piperidine, piperazine, pyridine, pyrimidine, pyrrolidine, pyrrolidinone, 1,2,3-triazine, 1,2,4-triazine, 1,3,5-triazine, 1,2,3-triazole, 1,2,4-triazole, tetrazine, tetrazole, 1,2-diazepine, 1,3-diazepine, 1,4-diazepine, azepine, ketopiperazine, oxazole, isoxazole, isoxazolidine, 2-isoxazoline, morpholine, thiazole, isothiazole, thiadiazole or thiomorpholine, wherein said cyclic group is unsubstituted or mono-, di- or trisubstituted independently of one another by R14,

R14 is fluoro, chlorine, -($C_1$-$C_4$)-alkyl or -$NH_2$,
V is 1. a cyclic residue out of the group containing compounds, which are derived from azaindolyl (1H-pyrrolopyridyl), azetidine, azepine, aziridine, azirine, 1,4-diazepane, 1,2-diazepine, 1,3-diazepine, 1,4-diazepine, diazirine, 1,3-dioxolane, dioxazole, furan, imidazole, isoquinoline, isothiazole, isothiazolidine, isothiazoline, isoxazole, 2-isoxazoline, isoxazolidine, ketopiperazine, morpholine, 1,2-oxazine, 1,3-oxazine, 1,4-oxazine, oxazole, 1,2-oxathiolan, piperidine, pyran, pyrazine, pyrazole, pyridazine, piperazine, pyridine, pyridone, pyrimidine, pyrrole, pyrrolidine, pyrrolidinone, quinazoline, quinoline, tetrazine, tetrazole, thiadiazine, 1,2-thiazine, 1,3-thiazine, 1,4-thiazine, 1 ,3-thiazole, thietan, thiomorpholine, thiophene, thiopyran, 1,2,3-triazine, 1,2,4-triazine, 1,3,5-triazine, 1,2,3-triazole or 1,2,4-triazole,
wherein said cyclic residue is unsubstituted or mono-, di- or trisubstituted independently of one another by R14, or
2. phenyl, wherein phenyl is unsubstituted or mono-, di- or trisubstituted independently of one another by R14,

G is a direct bond, -$(CH_2)_m$-, or -$(CH_2)_m$-$NR^{10}$-,
m is the integers zero, 1, 2, 3 or 4,
M is 1. a hydrogen atom,
2. heterocyclyl, wherein heterocyclyl is a residue out of the group which can be derived from 1,4-diazepane, ketomorpholine, thiophene, pyridazone, piperidine, piperazine, pyridine, pyrimidine, pyrrolidine, pyrrolidinone, pyridonyl, imidazole, pyridazine, pyrazine, 1,2,3-triazine, 1,2,4-triazine, 1,3,5-triazine, 1,2,3-triazole, 1,2,4-triazole, tetrazine, tetrazole, 1,2-diazepine, 1,3-diazepine, 1,4-diazepine, azepine, ketopiperazine, oxazole, isoxazole, isoxazolidine, 2-isoxazoline, morpholine, thiazole, isothiazole, tetrahydropyran, 1,4,5,6-tetrahydro-pyridazinyl, thiadiazole or thiomorpholine, wherein said heterocyclyl is unsubstituted or mono-, di- or trisubstituted independently of one another by R14,
3. -($C_1$-$C_6$)-alkyl, wherein alkyl is unsubstituted or mono-, di- or trisubstituted independently of one another by R14, or
4. ($C_3$-$C_6$)-cycloalkyl,

R3 is 1) hydrogen atom,

2) halogen,
3) -($C_1$-$C_4$)-alkyl, wherein alkyl is unsubstituted or mono-, di- or trisubstituted independently of one another by R13,

4) -($C_1$-$C_3$)-perfluoroalkyl,

5) phenyl, wherein phenyl is unsubstituted or mono-, di- or trisubstituted independently of one another by R13,

6) -($C_0$-$C_4$)-alkylene-O-R19, wherein R19 is

a) hydrogen atom,

b) -($C_1$-$C_4$)-alkyl, wherein alkyl is unsubstituted or mono-, di- or trisubstituted independently of one another by R13, or

c) phenyl, wherein phenyl is unsubstituted or mono-, di- or trisubstituted independently of one another by R13,

d) -$CF_3$, or

e) -$CHF_2$,

7) -CN,

8) -$NR^{10}$-$SO_2$-$R^{10}$,

9) -$SO_s$-$R^{11}$, wherein s is 1 or 2,

10) -$SO_t$-N($R^{11}$)-$R^{12}$, wherein t is 1 or 2,

11) -($C_0$-$C_4$)-alkylene-C(O)-$R^{11}$,

12) -($C_0$-$C_4$)-alkylene-C(O)-O-$R^{11}$,

13) -($C_0$-$C_4$)-alkylene-C(O)-N($R^{11}$)-$R^{12}$,

14) -($C_0$-$C_4$)-alkylene-N($R^{11}$)-$R^{12}$,

15) -($C_0$-$C_2$)alkylene-C(O)-O-($C_2$-$C_4$)-alkylene-O-C(O)-($C_1$-$C_4$)-alkyl,

16) -C(O)-O-C(R15, R16)-O-C(O)-R17,

17) -($C_0$-$C_2$)alkylene-C(O)-O-($C_2$-$C_4$)-alkylene-O-C(O)-O-($C_1$-$C_6$)-alkyl,

18) -C(O)-O- C(R15, R16)-O-C(O)-O-R17, or

19) a residue from the following list

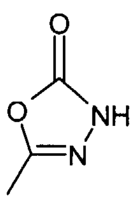
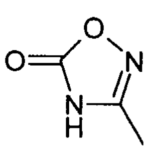
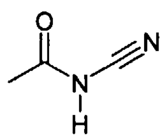
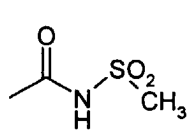
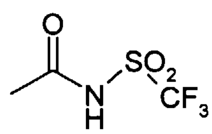
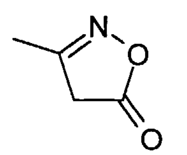
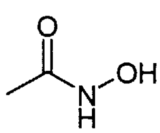
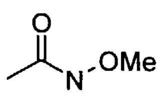
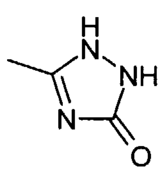
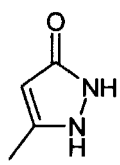
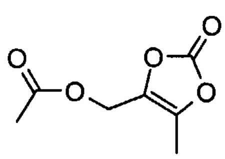
and
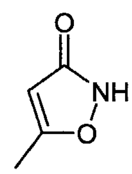

wherein Me is methyl,

R11 and R12 are independently of one another identical or different and are

1) hydrogen atom,

2) -($C_1$-$C_4$)-alkyl, wherein alkyl is unsubstituted or mono-, di- or trisubstituted independently of one another by R13,

3) -($C_0$-$C_6$)-alkyl-($C_3$-$C_6$)-cycloalkyl,

4) -O-$R^{17}$, or

5) -($C_0$-$C_6$)-alkyl-($C_4$-$C_{15}$)-heterocyclyl, wherein alkyl and heterocyclyl independently from one another are unsubstituted or mono-, di- or trisubstituted by R13 and wherein heterocyclyl is selected out of the group azetidine, cyclopropyl, cyclobutyl, 4,5-dihydro-oxazole, imidazolidine, morpholine, (1,4)-oxazepane, oxazolidine, piperidine, piperazine, pyrrolidine, tetrahydrothiophene, thiazolidine or thiomorpholine, or

R11 and R12 together with the nitrogen atom to which they are bonded form a heterocyclic ring, which is selected out of the group azetidine, cyclopropyl, cyclobutyl, 4,5-dihydro-oxazole, imidazolidine, morpholine, (1,4)-oxazepane, oxazolidine, piperidine, piperazine, pyrrolidine, tetrahydrothiophene, thiazolidine or thiomorpholine, wherein said ring is unsubstituted or mono-, di- or trisubstituted by R13,

R13 is fluorine, -CN, =O, -OH, $-CF_3$, $-C(O)-O-R^{10}$, $-C(O)-N(R^{10})-R^{20}$, $-N(R^{10})-R^{20}$, $-(C_3-C_6)$-cycloalkyl, $-(C_0-C_3)$-alkylene-O-$R^{10}$, $-Si-(CH_3)_3$, $-S-R^{10}$, $-SO_2-R^{10}$, $-(C_1-C_3)$-perfluoroalkyl, or a residue from the following list

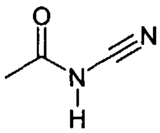
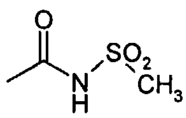
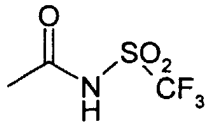
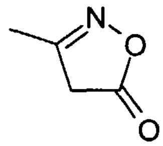
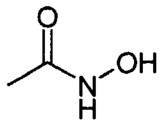
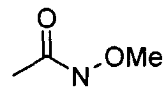
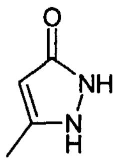
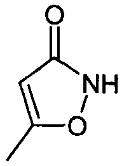
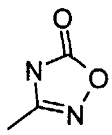
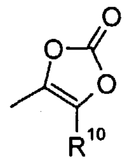
and,
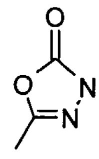

wherein Me is methyl,

$R^{10}$ and $R^{20}$ are independently of one another hydrogen, $-(C_1-C_4)$-alkyl or $-(C_1-C_3)$-perfluoroalkyl,

$R^{15}$ and $R^{16}$ are independently of one another hydrogen, $-(C_1-C_4)$-alkyl, or together form a ring out of the droup cyclopropyl, cyclobutyl, cyclopentyl or cyclohexyl, wherein each ring is unsubstituted or substituted one to three times by $R^{10}$, and

R17 is $-(C_1-C_6)$-alkyl, $-(C_1-C_6)$-alkyl-OH, $-(C_1-C_6)$-alkyl-O-$(C_1-C_6)$-alkyl, $-(C_3-C_8)$-cycloalkyl, $-(C_1-C_6)$-alkyl-O-$(C_1-C_8)$-alkyl-$(C_3-C_8)$-cycloalkyl, $-(C_1-C_6)$-alkyl-$(C_3-C_8)$-cycloalkyl, wherein said cycloalkyl ring is unsubstituted or substituted one, two or three times by -OH, -O-$(C_1-C_4)$-alkyl or $R^{10}$,

in all its stereoisomeric forms and mixtures thereof in any ratio, and its physiologically tolerable salts.

**7.** A compound of formula I as claimed in claims 1 to 6, wherein

R0 is 1. phenyl, wherein phenyl is unsubstituted or mono- or disubstituted

independently of one another by R8,
2. pyridyl, wherein pyridyl is unsubstituted or mono- or disubstituted independently of one another by R8, or
3. a heterocyclyl out of the group thienyl, thiadiazolyl, isoxazolyl and thiazolyl, wherein said heterocyclyl is substituted by a residue selected out of the group thienyl, 2-thienyl and 3-thienyl, wherein said residue is unsubstituted or mono- or disubstituted independently of one another by R8,

R8 is F, Cl, Br, $-OCH_3$, $-C(O)-NH_2$ or-O-$CF_3$,
substructure D is a residue selected out of the group phenyl, pyridyl, pyridyl-N-oxide, pyrrolyl, thienyl, imidazolyl, pyrazolyl, oxazolyl, isoxazolyl, thiazolyl, isothiazolyl, thiadiazolyl, pyrimidinyl, pyridazinyl or pyrazinyl and is unsubstituted or substituted 1, 2, 3 or 4 times by R3,

Q is a direct bond, -C(O)-; $-SO_2$-, $-CH_2-C(O)-NH$-, methylene or ethylene,

$R^1$ is hydrogen atom,

$R^2$ is a direct bond or methylene,

$R^1$-N-$R^2$-V can form a 4- to 8-membered cyclic group out of the group azetidine, pyrrolidine, piperidine and piperazine,

R14 is fluorine, chlorine, methyl, ethyl or $-NH_2$,

V is 1. a residue out of the group containing compounds which is derived from azaindolyl (1H-pyrrolopyridyl), azetidine, 1,4-diazepane, isoxazole, isoquinoline, piperazine, piperidine, pyrazine, pyridazine, pyrimidine, pyrrolidine, quinazoline, quinoline or tetrahydropyrane, wherein said cyclic residue is unsubstituted or mono- or disubstituted independently of

one another by R14, or

2. phenyl, wherein phenyl is unsubstituted or mono- or disubstituted independently of one another by R14,

G is a direct bond, $-(CH_2)_m-$, or $-(CH_2)_m-NR^{10}-$,

m is the integers zero, 1 or 2,

M is a hydrogen atom, $(C_2-C_4)$-alkyl, azepanyl, cyclopropyl, cyclobutyl, cyclopentyl, cyclohexyl, imidazolyl, ketomorpholinyl, morpholinyl, [1,4]Oxazepanyl, piperidinyl, piperidonyl, pyrazinyl, pyrazolyl, pyridazinyl, pyridinyl, pyrimidyl, pyrrolidinyl, 1,4,5,6-tetrahydro-pyridazinyl, or tetrahydropyranyl, wherein the residues are unsubstituted or mono- or disubstituted independently of one another by R14

R3 is

1) hydrogen atom,

2) fluorine, chlorine,

3) $-(C_1-C_4)$-alkyl, wherein alkyl is unsubstituted or mono-, di- or trisubstituted independently of one another by R13,

4) $-(C_1-C_3)$-perfluoroalkyl,

5) phenyl, wherein phenyl is unsubstituted or mono-, di- or trisubstituted independently of one another by R13,

6) $-(C_0-C_2)$-alkylene-O-R19, wherein R19 is

a) hydrogen atom,

b) $-(C_1-C_4)$-alkyl, wherein alkyl is unsubstituted or mono-, di- or trisubstituted independently of one another by R13, or

c) phenyl, wherein phenyl is unsubstituted or mono-, di- or trisubstituted independently of one another by R13,

d) $-CF_3$, or

e) $-CHF_2$,

7) -CN,

8) $-NR^{10}-SO_2-R^{10}$,

9) $-SO_s-R^{11}$, wherein s is 1 or 2,

10) $-SO_t-N(R^{11})-R^{12}$, wherein t is 1 or 2,

11) $-(C_0-C_4)$-alkylene-C(O)-$R^{11}$,

12) $-(C_0-C_4)$-alkylene-C(O)-O-$R^{11}$,

13) $-(C_0-C_4)$-alkylene-C(O)-N($R^{11}$)-$R^{12}$,

14) $-(C_0-C_4)$-alkylene-N($R^{11}$)-$R^{12}$,

15) $-(C_0-C_2)$alkylene-C(O)-O-$(C_2-C_4)$-alkylene-O-C(O)-$(C_1-C_4)$-alkyl,

16) -C(O)-O-C(R15, R16)-O-C(O)-R17,

17) $-(C_0-C_2)$alkylene-C(O)-O-$(C_2-C_4)$-alkylene-O-C(O)-O-$(C_1-C_6)$-alkyl,

18) -C(O)-O- C(R15, R16)-O-C(O)-O-R17, or

19) a residue from the following list

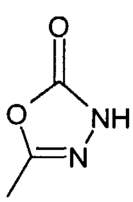
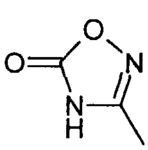
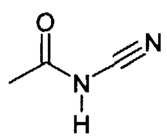
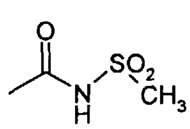
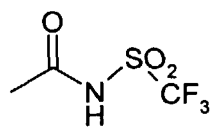
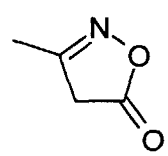
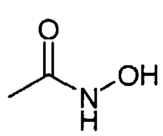
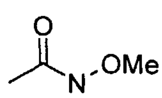
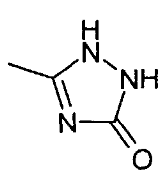
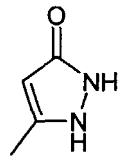
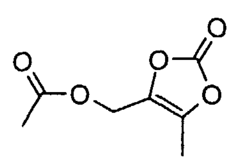
and
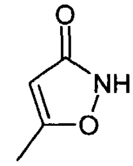

wherein Me is methyl,

R11 and R12 are independently of one another identical or different and are

1) hydrogen atom,
2) -($C_1$-$C_4$)-alkyl, wherein alkyl is unsubstituted or mono-, di- or trisubstituted independently of one another by R13,
3) -($C_0$-$C_6$)-alkyl-($C_3$-$C_6$)-cycloalkyl,
4) -O-$R^{17}$, or
5) -($C_0$-$C_6$)-alkyl-heterocyclyl, wherein alkyl and heterocyclyl independently from one another are unsubstituted or mono-, di- or trisubstituted by R13 and wherein heterocyclyl is selected out of the group azetidine, imidazolidine, morpholine, (1,4)-oxazepane or pyrrolidine or

R11 and R12 together with the nitrogen atom to which they are bonded can form a ring, which is selected out of the group azetidine, imidazolidine, morpholine, (1,4)-oxazepane piperazine, piperidine, pyrrolidine or thiomorpholine, wherein said ring is unsubstituted or mono-, di- or trisubstituted by R13,

R13 is fluorine, -CN, =O, -OH, -$CF_3$, -C(O)-O-$R^{10}$, -C(O)-N($R^{10}$)-$R^{20}$, -N($R^{10}$)-$R^{20}$, -($C_3$-$C_6$ )-cycloalkyl, -($C_1$-$C_3$)-alkylene-O-$R^{10}$, -Si-$(CH_3)_3$, -S-$R^{10}$, -$SO_2$-$R^{10}$, -($C_1$-$C_3$)-perfluoroalkyl, or a residue from the following list

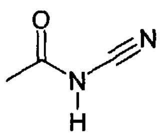 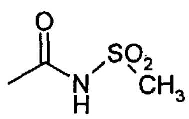 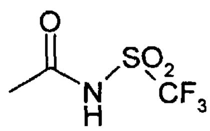 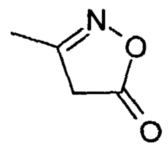 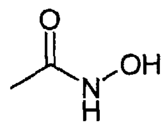 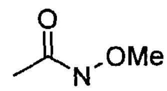

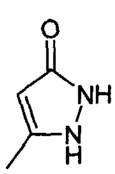 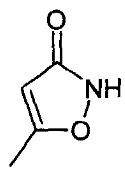 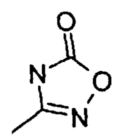 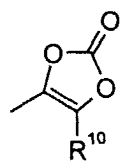 and, 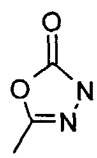

wherein Me is methyl,

$R^{10}$ and $R^{20}$ are independently of one another hydrogen, -($C_1$-$C_4$)-alkyl or -($C_1$-$C_3$)-perfluoroalkyl,

$R^{15}$ and $R^{16}$ are independently of one another hydrogen, -($C_1$-$C_4$)-alkyl, or together form a ring out of the droup cyclopropyl, cyclobutyl, cyclopentyl or cyclohexyl, wherein each ring is unsubstituted or substituted one to three times by $R^{10}$, and R17 is -($C_1$-$C_6$)-alkyl, -($C_1$-$C_6$)-alkyl-OH, -($C_1$-$C_6$)-alkyl-O-($C_1$-$C_6$)-alkyl, -($C_3$-$C_8$)-cycloalkyl, -($C_1$-$C_6$)-alkyl-O-($C_1$-$C_8$)-alkyl-($C_3$-$C_8$)-cycloalkyl, -($C_1$-$C_6$)-alkyl-($C_3$-$C_8$)-cycloalkyl, wherein said cycloalkyl ring is unsubstituted or substituted one, two or three times by -OH, -O-($C_1$-$C_4$)-alkyl or $R^{10}$,

in all its stereoisomeric forms and mixtures thereof in any ratio, and its physiologically tolerable salts.

**8.** A compound of formula I as claimed in one or more of claims 1 to 7, wherein the compound of formula I is
1-[5-(5-Chloro-thiophen-2-yl)-isoxazol-3-ylmethyl]-1H-benzoimidazole-2-carboxylic acid (1-isopropyl-piperidin-4-yl)-amide
1-[(4-Chloro-phenylcarbamoyl)-methyl]-1H-benzoimidazole-2-carboxylic acid (1-isopropyl-piperidin-4-yl)-amide
1-[(5-Chloro-pyridin-2-ylcarbamoyl)-methyl]-1H-benzoimidazole-2-carboxylic acid (1-isopropyl-piperidin-4-yl)-amide,
1-[5-(5-Chloro-thiophen-2-yl)-isoxazol-3-ylmethyl]-1H-benzoimidazole-2-carboxylic acid (1-pyrimidin-4-yl-piperidin-4-yl)-amide,
1-[5-(5-Chloro-thiophen-2-yl-isoxazol-3-ylmethyl]-2-(1-isopropyl-piperidin-4-ylcarbamoyl)-1H-benzoimidazole-5-carboxylic acid methyl ester,
3-[5-(5-Chloro-thiophen-2-yl-isoxazol-3-ylmethyl]-2-(1-isopropyl-piperidin-4-ylcarbamoyl)-3H-benzoimidazole-5-carboxylic acid methyl ester,
1-[5-(5-Chloro-thiophen-2-yl)-isoxazol-3-ylmethyl]-2-(1-isopropyl-piperidin-4-ylcarbamoyl)-1H-benzoimidazole-5-carboxylic acid,
3-[5-(5-Chloro-thiophen-2-yl-isoxazol-3-ylmethy]-2-(1-isopropyl-piperidin-4-ylcarbamoyl)-3H-benzoimidazole-5-carboxylic acid,

1-[2-(5-Chloro-thiophen-2-yl)-thiazol-5-ylmethyl]-2-(1-isopropyl-piperidin-4-ylcarbamoyl)-1H-benzoimidazole-5-carboxylic acid methyl ester,
3-[2-(5-Chloro-thiophen-2-yl)-thiazol-5-ylmethyl]-2-(1-isopropyl-piperidin-4-ylcarbamoyl)-3H-benzoimidazole-5-carboxylic acid methyl ester,
1-[2-(5-Chloro-thiophen-2-yl)-thiazol-5-ylmethyl]-2-(1-isopropyl-piperidin-4-ylcarbamoyl)-1H-benzoimidazole-5-carboxylic acid,
3-[2-(5-Chloro-thiophen-2-yl)-thiazol-5-ylmethyl]-2-(1-isopropyl-piperidin-4-ylcarbamoyl-3H-benzoimidazole-5-carboxylic acid,
1-[5-(5-Chloro-thiophen-2-yl)-isoxazol-3-ylmethyl]-2-(1-isopropyl-piperidin-4-ylcarbamoyl)-1H-benzoimidazole-4-carboxylic acid methyl ester,
3-[5-(5-Chloro-thiophen-2-yl)-isoxazol-3-ylmethyl]-2-(1-isopropyl-piperidin-4-ylcarbamoyl)-3H-benzoimidazole-4-carboxylic acid methyl ester,
1-[5-(5-Chloro-thiophen-2-yl)-isoxazol-3-ylmethyl]-2-(1-isopropyl-piperidin-4-ylcarbamoyl)-1H-benzoimidazole-4-carboxylic acid,
3-[5-(5-Chloro-thiophen-2-yl)-isoxazol-3-ylmethyl]-2-(1-isopropyl-piperidin-4-ylcarbamoyl)-3H-benzoimidazole-4-carboxylic acid,
3-[5-(5-Chloro-thiophen-2-yl)-isoxazol-3-ylmethyl]-3H-imidazo[4,5-b]pyridine-2- carboxylic acid (1-isopropyl-piperidin-4-yl)-amide,
1-[(5-Chloro-pyridin-2-ylcarbamoyl)-methyl]-2-(1-isopropyl-piperidin-4-ylcarbamoyl)-1H-benzoimidazole-4-carboxylic acid methyl ester,
1-[(5-Chloro-pyridin-2-ylcarbamoyl)-methyl]-2-(1-isopropyl-piperidin-4-ylcarbamoyl)-1H-benzoimidazole-4-carboxylic acid,
1-[(5-Chloro-pyridin-2-ylcarbamoyl)-methyl]-2-(1-isopropyl-piperidin-4-ylcarbamoyl)-1H-benzoimidazole-5-carboxylic acid methyl ester,
3-[(5-Chloro-pyridin-2-ylcarbamoyl)-methyl]-2-(1-isopropyl-piperidin-4-ylcarbamoyl)-3H-benzoimidazole-5-carboxylic acid methyl ester,
1-[(5-Chloro-pyridin-2-ylcarbamoyl)-methyl]-2-(1-isopropyl-piperidin-4-ylcarbamoyl)-1H-benzoimidazole-5-carboxylic acid,
3-[(5-Chloro-pyridin-2-ylcarbamoyl)-methyl]-2-(1-isopropyl-piperidin-4-ylcarbamoyl)-3H-benzoimidazole-5-carboxylic acid,
1-[5-(5-Chloro-thiophen-2-yl)-isoxazol-3-ylmethyl]-2-(1-isopropyl-piperidin-4-ylcarbamoyl)-1H-benzoimidazole-4-carboxylic acid 1-ethoxycarbonyloxy-ethyl ester ,
1-[5-(5-Chloro-thiophen-2-yl)-isoxazol-3-ylmethyl]-2-(1-isopropyl-piperidin-4-ylcarbamoyl)-1H-benzoimidazole-4-carboxylic acid 1-cyclohexyloxycarbonyloxy-ethyl ester ,
1-[5-(5-Chloro-thiophen-2-yl)-isoxazol-3-ylmethyl]-1H-benzoimidazole-2,4-dicarboxylic acid 4-[(2-hydroxy-ethyl)-amide] 2-[(1-isopropyl-piperidin-4-yl)-amide],
1-[5-(5-Chloro-thiophen-2-yl)-isoxazol-3-ylmethyl]-4-(3-hydroxy-azetidine-1-carbonyl)-1H-benzoimidazole-2-carboxylic acid (1-isopropyl-piperidin-4-yl)-amide,
1-[5-(5-Chloro-thiophen-2-yl)-isoxazol-3-ylmethyl]-1H-benzoimidazole-2,4-dicarboxylic acid 4-[(2-hydroxy-ethyl)-methyl-amide] 2-[(1-isopropyl-piperidin-4-yl)-amide],
1-[5-(5-Chloro-thiophen-2-yl)-isoxazol-3-ylmethyl]-1H-benzoimidazole-2,5-dicarboxylic acid 5-[(2-hydroxy-ethyl)-methyl-amide] 2-[(1-isopropyl-piperidin-4-yl)-amide],
1-[5-(5-Chloro-thiophen-2-yl)-isoxazol-3-ylmethyl]-5-(3-hydroxy-azetidine-1-carbonyl)-1H-benzoimidazole-2-carboxylic acid (1-isopropyl-piperidin-4-yl)-amide,
1-[5-(5-Chloro-thiophen-2-yl)-isoxazol-3-ylmethyl]-2-(1-isopropyl-piperidin-4-ylcarbamoyl)-1H-benzoimidazole-5-carboxylic acid 1-ethoxycarbonyloxy-ethyl ester,
1-[5-(5-Chloro-thiophen-2-yl)-isoxazol-3-ylmethyl]-2-(1-isopropyl-piperidin-4-ylcarbamoyl)-1H-benzoimidazole-5-carboxylic acid 1-cyclohexyloxycarbonyloxy-ethyl ester,
1-[5-(5-Chloro-thiophen-2-yl)-isoxazol-3-ylmethyl]-5-(2-hydroxy-ethanesulfonyl)-1H-benzoimidazole-2-carboxylic acid (1-isopropyl-piperidin-4-yl)-amide,
1-[5-(5-Chloro-thiophen-2-yl)-isoxazol-3-ylmethyl]-6-(2-hydroxy-ethanesulfonyl)-1H-benzoimidazole-2-carboxylic acid (1-isopropyl-piperidin-4-yl)-amide,
1-[(5-Chloro-pyridin-2-ylcarbamoyl)-methyl]-1H-benzoimidazole-2,4-dicarboxylic acid 4-[(2-hydroxy-ethyl)-amide] 2-[(1-isopropyl-piperidin-4-yl)-amide],
1-[(5-Chloro-pyridin-2-ylcarbamoyl)-methyl]-1H-benzoimidazole-2,4-dicarboxylic acid 4-[(2-hydroxy-ethyl)-methyl-amide] 2-[(1-isopropyl-piperidin-4-yl)-amide],
1-[(5-Chloro-pyridin-2-ylcarbamoyl)-methyl]-4-(3-hydroxy-azetidine-1-carbonyl)-1H-benzoimidazole-2-carboxylic acid (1-isopropyl-piperidin-4-yl)-amide,

1-[(5-Chloro-pyridin-2-ylcarbamoyl)-methyl]-2-(1-isopropyl-piperidin-4-ylcarbamoyl)-1H-benzoimidazole-4-carboxylic acid 1-ethoxycarbonyloxy-ethyl ester,
1-[(5-Chloro-pyridin-2-ylcarbamoyl)-methyl]-2-(1-isopropyl-piperidin-4-ylcarbamoyl)-1H-benzoimidazole-4-carboxylic acid 1-cyclohexyloxycarbonyloxy-ethyl ester,
1-[(5-Chloro-pyridin-2-ylcarbamoyl)-methyl]-2-[4-(3-oxo-morpholin-4-yl)-phenylcarbamoyl]-1H-benzoimidazole-4-carboxylic acid methyl ester or
1-[(5-Chloro-pyridin-2-ylcarbamoyl)-methyl]-1H-benzoimidazole-2-carboxylic acid [4-(3-oxo-morpholin-4-yl)-phenyl]-amide.

**9.** A process for the preparation of a compound of the formula I as claimed in one or more of claims 1 to 8, which comprises condensing a compound of the formula 2 with a compound of the formula $HR^{8'}$ to give a compound of the formula 3 and optionally converting the compound of the formula 3 into a compound of the formula I,

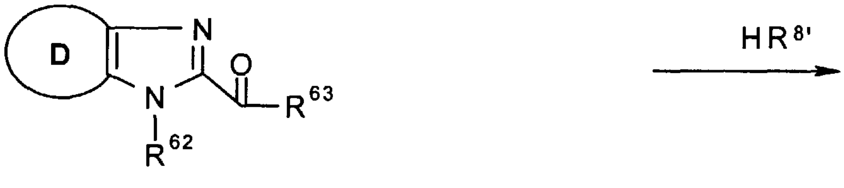


2

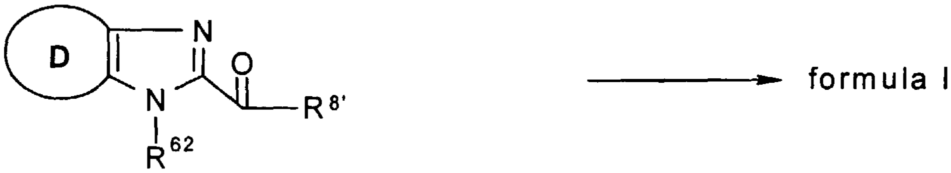


3

wherein the residue $R^{8'}$ has the donation of-$N(R^1)$-$R^2$-V-G-M as indicated in claims 1 to 8, but where in $R^{8'}$ functional groups can also be present in the form of groups that are subsequently transformed into the final functional groups present in -$N(R^1)$-$R^2$-V-G-M, and where the residue $R^{62}$ denotes the group -Q-$R^0$ or can denote a group which is subsequently transformed into the group -Q-$R^0$, and where the group -C(O)-$R^{49}$ can be a carboxylic acid group or derivatives thereof, as defined in claims 1 to 8, wherein said functional groups can also be present in protected form or in the form of precursor groups.

**10.** A pharmaceutical preparation, comprising at least one compound of the formula I as claimed in one or more of claims 1 to 8 in all its stereoisomeric forms and mixtures thereof in any ratio and/or its physiologically tolerable salts and a pharmaceutically acceptable carrier.

**11.** The use of a compound of the formula I as claimed in one or more of claims 1 to 8 in all its stereoisomeric forms and mixtures thereof in any ratio and/or their physiologically tolerable salts for the production of pharmaceuticals for inhibition of factor Xa and/or factor VIIa or for influencing blood coagulation or fibrinolysis.

**12.** The use as claimed in claim11 for abnormal thrombus formation, acute myocardial infarction, cardiovascular disorders, unstable angina, thromboembolism, acute vessel closure associated with thrombolytic therapy or percutaneous transluminal coronary angioplasty (PTCA), transient ischemic attacks, stroke, intermittent claudication, bypass grafting of the coronary or peripheral arteries, vessel luminal narrowing, restenosis post coronary or venous angioplasty, maintenance of vascular access patency in long-term hemodialysis patients, pathologic thrombus formation occurring in the veins of the lower extremities following abdominal, knee or hip surgery, pathologic thrombus formation occurring in the veins of the lower extremities following abdominal, knee and hip surgery, a risk of pulmonary thromboembolism, or disseminated systemic intravascular coagulatopathy occurring in vascular systems during septic shock, viral infections or cancer, or reducing an inflammatory response, fibrinolysis, or treatment of coronary heart disease, myocardial infarction, angina pectoris, vascular restenosis, for example restenosis following angioplasty like PTCA, adult respiratory distress syndrome, multi-organ failure and disseminated intravascular clotting disorder, deep vein or proximal vein thrombosis, which can occur following surgery.

European Patent Office

# EUROPEAN SEARCH REPORT

Application Number
EP 03 01 1305

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| A | US 6 562 828 B1 (HAYASHI MIKIO ET AL) 13 May 2003 (2003-05-13)<br>* page 4 - column 6 *<br>* example 114; table 8 * | 1-12 | C07D413/14<br>C07D401/04<br>C07D401/14<br>C07D417/14<br>C07D471/04<br>A61K31/4184<br>A61P7/02 |
| A | MATTER, HANS ET AL: "Design and Quantitative Structure-Activity Relationship of 3-Amidinobenzyl-1H-indole-2-carboxamides as Potent, Nonchiral, and Selective Inhibitors of Blood Coagulation Factor Xa"<br>JOURNAL OF MEDICINAL CHEMISTRY (2002), 45(13), 2749-2769 CODEN: JMCMAR; ISSN: 0022-2623, 2002, XP002256632<br>* the whole document * | 1-12 | |
| X | PATENT ABSTRACTS OF JAPAN vol. 1998, no. 12, 31 October 1998 (1998-10-31)<br>& JP 10 182459 A (OTSUKA PHARMACEUT CO LTD), 7 July 1998 (1998-07-07)<br>* abstract *<br>-& JP 10 182459 A (OTSUKA PHARMACEUT CO LTD) 7 July 1998 (1998-07-07)<br>* page 57 - page 58; examples 24,25 * | 1-7,10 | TECHNICAL FIELDS SEARCHED (Int.Cl.7)<br>C07D<br>A61K<br>A61P |
| X | PATENT ABSTRACTS OF JAPAN vol. 1997, no. 09, 30 September 1997 (1997-09-30)<br>& JP 9 124609 A (NISSAN CHEM IND LTD), 13 May 1997 (1997-05-13)<br>* abstract *<br>-& JP 09 124609 A (NISSAN CHEM IND LTD) 13 May 1997 (1997-05-13)<br>* tables 20,21 *<br>* tables 51-54 * | 1-7,10 | |
| | -/-- | | |

The present search report has been drawn up for all claims

| Place of search | Date of completion of the search | Examiner |
|---|---|---|
| Munich | 16 January 2004 | Kollmannsberger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 01 1305

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN<br>vol. 0154, no. 39 (P-1273),<br>8 November 1991 (1991-11-08)<br>& JP 3 184043 A (FUJI PHOTO FILM CO LTD),<br>12 August 1991 (1991-08-12)<br>* abstract *<br>-& JP 03 184043 A (FUJI PHOTO FILM CO LTD)<br>12 August 1991 (1991-08-12)<br>* page 10; example 3 *<br>----- | 1-7,10 | |
| X | GB 1 377 642 A (KONINKLIJKE GIST SPIRITUS)<br>18 December 1974 (1974-12-18)<br>* claims *<br>* example 10 *<br>----- | 1-7,10 | |
| X | EP 0 254 322 A (NISSHIN FLOUR MILLING CO)<br>27 January 1988 (1988-01-27)<br>* claim 1 *<br>* example 28 *<br>----- | 1-7,10 | |
| X | DE 38 28 537 A (BASF AG)<br>1 March 1990 (1990-03-01)<br>* example 17 *<br>----- | 1-7,10 | TECHNICAL FIELDS SEARCHED (Int.Cl.7) |
| X | DATABASE CHEMABS [Online]<br>CHEMICAL ABSTRACTS SERVICE, COLUMBUS, OHIO, US; 1997,<br>NATSUKARI, HIDEAKI ET AL: "Preparation of heterocyclic compounds as cholesterol acyltransferase inhibitors"<br>XP002256741<br>retrieved from STN<br>Database accession no. 1997:51439<br>* abstract *<br>* compounds with RN 185332-08-5, 185332-09-6, 185332-12-1 *<br>-/-- | 1-7,10 | |

The present search report has been drawn up for all claims

| Place of search | Date of completion of the search | Examiner |
|---|---|---|
| Munich | 16 January 2004 | Kollmannsberger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 03 01 1305

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| | -& JP 08 295667 A (TAKEDA CHEMICAL INDUSTRIES LTD, JAPAN) 12 November 1996 (1996-11-12) * claims * ----- | | |
| X | DATABASE CHEMCATS CHEMICAL ABSTRACTS SERVICE, COLUMBUS, OHIO, US; 2003, XP002256742 order numbers: 7215720016, 7215720017 & "Otava chemical collection" 21 April 2003 (2003-04-21), OTAVA , KYIV, UKRAINE ----- | 1-7 | |
| X | DATABASE CHEMCATS CHEMICAL ABSTRACTS SERVICES, COLUMBUS, OHIO, US; 2003, XP002256743 order numbers STOCK2S-06321, t0311-0063, t0311-0061, t011-0059, t0311-0057 & "Ambinter: Exploratory library" 30 April 2003 (2003-04-30), AMBINTER , PARIS, FRANCE ----- | 1-7 | TECHNICAL FIELDS SEARCHED (Int.Cl.7) |

The present search report has been drawn up for all claims

| Place of search | Date of completion of the search | Examiner |
|---|---|---|
| Munich | 16 January 2004 | Kollmannsberger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 01 1305

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2004

| Patent document cited in search report | Publication date | Patent family member(s) | Publication date |
|---|---|---|---|
| US 6562828 B1 | 13-05-2003 | AU 752588 B2 | 26-09-2002 |
| | | AU 3167799 A | 01-11-1999 |
| | | BR 9910122 A | 16-10-2001 |
| | | CA 2327488 A1 | 21-10-1999 |
| | | CN 1305470 T | 25-07-2001 |
| | | EP 1070714 A1 | 24-01-2001 |
| | | HU 0101137 A2 | 28-10-2001 |
| | | WO 9952895 A1 | 21-10-1999 |
| | | JP 3283485 B2 | 20-05-2002 |
| | | JP 2000136190 A | 16-05-2000 |
| | | NO 20005083 A | 08-12-2000 |
| | | NZ 508101 A | 20-12-2002 |
| | | PL 343424 A1 | 13-08-2001 |
| | | RU 2201927 C2 | 10-04-2003 |
| | | SG 74717 A1 | 22-08-2000 |
| | | SK 15072000 A3 | 06-08-2001 |
| | | TR 200002904 T2 | 21-02-2001 |
| | | ZA 200006430 A | 25-07-2001 |
| JP 10182459 A | 07-07-1998 | NONE | |
| JP 9124609 A | 13-05-1997 | NONE | |
| JP 3184043 A | 12-08-1991 | NONE | |
| GB 1377642 A | 18-12-1974 | NL 7200486 A | 18-07-1972 |
| EP 0254322 A | 27-01-1988 | JP 2054428 C | 23-05-1996 |
| | | JP 7084462 B | 13-09-1995 |
| | | JP 63146871 A | 18-06-1988 |
| | | AU 597696 B2 | 07-06-1990 |
| | | AU 7596587 A | 28-01-1988 |
| | | CA 1305481 C | 21-07-1992 |
| | | DE 3781845 D1 | 29-10-1992 |
| | | DE 3781845 T2 | 18-02-1993 |
| | | EP 0254322 A1 | 27-01-1988 |
| | | ES 2044878 T3 | 16-01-1994 |
| | | FI 873205 A ,B, | 26-01-1988 |
| | | KR 9611388 B1 | 22-08-1996 |
| | | NO 873091 A ,B, | 26-01-1988 |
| | | US 4886803 A | 12-12-1989 |
| | | BR 8703857 A | 29-03-1988 |
| DE 3828537 A | 01-03-1990 | DE 3828537 A1 | 01-03-1990 |
| | | CA 1334420 C | 14-02-1995 |
| | | DE 58908064 D1 | 25-08-1994 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 03 01 1305

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2004

| Patent document cited in search report | Publication date | Patent family member(s) | Publication date |
|---|---|---|---|
| DE 3828537 A | | EP 0358025 A1 | 14-03-1990 |
| | | JP 3002171 A | 08-01-1991 |
| | | US 5008397 A | 16-04-1991 |
| JP 8295667 A | 12-11-1996 | NONE | |

EPO FORM P0459

For more details about this annex : see Official Journal of the European Patent Office, No. 12/82